# EUROPEAN PATENT APPLICATION

(11) **EP 4 417 617 A1**
(43) Date of publication of application: **21.08.2024**
(21) Application number: 22881386.1
(22) Date of filing: 13.10.2022
(51) Int. Cl.: C07K 7/06, C07K 7/08, A61K 38/00, A61K 47/16

(54) **MITOCHONDRIA-SPECIFIC PEPTIDE THAT CAN BE INTRACELLULARLY DELIVERED AT NANOMOLAR CONCENTRATION, AND USE THEREOF**

(30) Priority: 13.10.2021 KR 20210135826; 09.12.2021 KR 20210176133; 03.01.2022 KR 20220000562
(71) Applicant: Camp Therapeutics Inc., Seoul 08826 (KR)
(72) Inventor: YU, Jea Hoon, Seongnam-si, Gyeonggi-do 13531 (KR); CHOI, Yoon Hwa, Seoul 08826 (KR)
(74) Representative: Dehns
(86) International application number: PCT/KR2022/015539
(87) International publication number: WO 2023/063759

(57) **Abstract**

The present invention relates to a peptide having a cell penetration ability and a strong mitochondrial targeting property at a low concentration, and a medical use thereof. A peptide or a dimer thereof, of the present invention, is very useful in the prevention or treatment of diseases accompanied by mitochondrial dysfunction, and liver diseases.

## Description

### Technical Field

The present disclosure relates to a peptide that has both cell-penetrating ability and strong mitochondria-targeting properties, and medicinal uses thereof.

This application claims priorities based on Korean Patent Application No. 10-2021-0135826, filed on October 13, 2021, Korean Patent Application No. 10-2021-0176133, filed on December 9, 2021, Korean Patent Application No. 10-2022-0000562, filed on January 3, 2022, and Korean Patent Application No. 10-2022-0131798, filed on October 13, 2022, the entire disclosures of which are incorporated herein by reference.

### Background Art

Mitochondrion is an important organelle that produces most of ATP which is essential for cell survival. Mitochondrial dysfunction, which is caused by damage to mitochondrial DNA, environmental or external influence, or the like, is responsible for a variety of intrinsic mitochondrial disorders as well as almost all degenerative diseases. One of the main causes of mitochondrial dysfunction is depletion of cristae structure which is promoted by deficiency or oxidation of cardiolipin (CL), a phospholipid that contains polyunsaturated fatty acids and is distributed in high density (~20%) in the inner membrane of mitochondria. CL, which contains two phosphate groups, plays an essential role in ATP production by providing multiple charge-charge interactions with functional membrane proteins. A substance, which specifically binds to CL and other phospholipids in the inner mitochondrial membrane (IMM), can prevent oxidation of CL. Thus, such a substance can protect the mitochondria from destruction of cristae structure and allows normal mitochondrial function to be maintained.

On the other hand, for a molecule, its specificity for mitochondria may be attributed to a functional group that is hydrophobic and has a positive charge. Triphenylphosphonium (TPP; Ph3P⁺-R) cation and the like are typical mitochondria-specific molecules, which target the negative charge of phospholipids present in the inner membrane. However, molecules with these properties have problems with cell penetration at low concentrations and can only be delivered into cells at high concentrations. In a case where these molecules are delivered into cells at high concentrations, there is a high possibility that they will easily bind to other molecules within the cell and cause toxicity. In addition, these mitochondria-specific molecules have weak binding capacity for CL, which is a typical phospholipid in the inner mitochondrial membrane (IMM), so that they are unable to remain in the inner membrane that is rich in CL and continue to penetrate the interior of the membrane. Therefore, most mitochondria-targeting molecules (peptides) developed to date have been used only to kill mitochondria by targeting DNA present inside the mitochondrial membrane. On the contrary, very few molecules have an effect of increasing or restoring mitochondrial activity.

A representative example of the substance, which specifically binds to CL, is a mitochondria-specific SS (Szeto-Schiller) peptide that consists of two hydrophilic amino acids and two hydrophobic amino acids arranged in an alternating way. This peptide has two positively charged amines and two aromatic functional groups which have hydrophilic and hydrophobic interactions with phosphate and fatty acid components of CL, respectively. However, affinity of the SS peptide for CL is relatively weak (which requires micromolar concentrations). Moreover, in a case where this SS peptide is extended to have a sequence of six amino acids, although it has slightly improved mitochondrial penetration and specificity for mitochondria, there is a problem of causing serious toxicity.

### Disclosure of Invention

### Technical Problem

The object of the present disclosure is to solve all of the above-mentioned problems.

An object of the present disclosure is to provide a peptide or a dimer thereof with excellent cell-penetrating ability and mitochondria-targeting ability by arrangement of hydrophobic amino acids and hydrophilic amino acids at specific positions of the peptide.

Another object of the present disclosure is to provide a pharmaceutical use of the peptide or the dimer thereof.

Yet another object of the present disclosure is to provide a pharmaceutical composition comprising, as an active ingredient, the peptide or the dimer thereof, or a method for preventing or treating a disease using the same.

Still yet another object of the present disclosure is to provide a pharmaceutical composition for prevention or treatment of a mitochondrial dysfunction-related disease, and a method for preventing or treating a mitochondrial dysfunction-related disease using the same.

Still yet another object of the present disclosure is to provide a pharmaceutical composition for prevention or treatment of a liver disease, and a method for preventing or treating a liver disease using the same.

The object of the present disclosure is not limited to the above-mentioned objects. The objects of the present disclosure will become clearer from the following description and may be realized by means and combinations thereof as set forth in the claims.

### Solution to Problem

Representative configurations of the present disclosure to achieve the above-mentioned objects are as follows.

According to an aspect of the present disclosure, there is provided a pharmaceutical composition for prevention or treatment of a disease accompanied by mitochondrial dysfunction, comprising, as an active ingredient, a peptide comprising the monomer represented by Formula 1, or a dimer thereof:

<Formula 1> X₁X₂X₃X₄X₅X₆X₇X₈X₉X₁₀

In Formula 1, X₁, X₄, X₅, and X₈ are each independently a hydrophobic amino acid; X₃, X₆, X₇, and X₁₀ are each independently a hydrophilic amino acid; X₂ and X₉ are each independently an amino acid that forms a bond so that two monomers can be linked to each other at one or more positions selected from X₂ and X₉ to form a dimeric peptide; and X₁ is N-terminus and X₁₀ is C-terminus.

According to another aspect of the present disclosure, there is provided a use of a composition for prevention or treatment of a disease accompanied by mitochondrial dysfunction, the composition comprising, as an active ingredient, a peptide comprising the monomer represented by Formula 1, or a dimer thereof.

According to yet another aspect of the present disclosure, there is provided a use of a peptide or a dimer thereof for the manufacture of a medicament used in a disease accompanied by mitochondrial dysfunction, the peptide comprising the monomer represented by Formula 1.

In an embodiment, the disease accompanied by mitochondrial dysfunction may be selected from the group consisting of mitochondrial myopathy, MELAS (mitochondrial encephalopathy, lactic acidosis, and stroke-like episodes) syndrome, Charcot Marie Tooth disease (CMT), Leber hereditary optic neuropathy, Pearson syndrome, Leigh syndrome, Friedreich's ataxia, and Barth syndrome.

According to still yet another aspect of the present disclosure, there is provided a pharmaceutical composition for prevention or treatment of a liver disease, the composition comprising, as an active ingredient, a peptide comprising the monomer represented by Formula 1, or a dimer thereof.

According to still yet another aspect of the present disclosure, there is provided a use of a composition for prevention or treatment of a liver disease, the composition comprising, as an active ingredient, a peptide comprising the monomer represented by Formula 1, or a dimer thereof.

According to still yet another aspect of the present disclosure, there is provided a use of a peptide or a dimer thereof for manufacture of a medicament used in a liver disease, the peptide comprising the monomer represented by Formula 1.

In an embodiment, the liver disease may be selected from the group consisting of acute liver failure, acute liver injury, liver cirrhosis, and hepatitis.

In another embodiment, X₁, X₄, X₅, and X₈ in Formula 1 may be each independently leucine (L), isoleucine (I), phenylalanine (F), tyrosine (Y), valine (V), norvaline (norV), tryptophan (W), pentylglycine (pg), neopentylglycine (Npg), alanine (A), or cyclohexylalanine (Cha).

In yet another embodiment, X₁, X₄, X₅, and X₈ in Formula 1 may be each independently leucine (L), phenylalanine (F), tyrosine (Y), or cyclohexylalanine (Cha).

In an embodiment, X₃, X₆, X₇, and X₁₀ in Formula 1 may be each independently arginine (R), lysine (K), homoarginine (hR), norarginine (norR), histidine (H), omithine (O), diaminobutanoic acid (Dab), or diaminopropanoic acid (Dap).

In another embodiment, X₃, X₆, X₇, and X₁₀ in Formula 1 may be each independently arginine (R), lysine (K), homoarginine (hR), norarginine (norR), or histidine (H).

In an embodiment, X₂ and X₉ in Formula 1 may be each independently cysteine (C), homocysteine (Hcy), penicillamine (Pen), selenocysteine (U), or leucine (L), provided that X₂ and X₉ are not leucine (L) at the same time.

In another embodiment, X₂ and X₉ may be each independently cysteine (C), homocysteine (Hcy), or penicillamine (Pen).

In an embodiment, the dimer may be such that the two peptides, each of which comprises the monomer represented by Formula 1, are linked to each other in an antiparallel direction.

In another embodiment, X₂ and X₉ may be each independently cysteine (C), homocysteine (Hcy), or penicillamine (Pen), and the linkage may be by a disulfide bond.

In yet another embodiment, the monomer represented by Formula 1 may be a peptide consisting of an amino acid sequence of any one of SEQ ID NOS: 1 to 19.

In still yet another embodiment, the composition may comprise the monomer or the dimer thereof at a nanomolar concentration.

According to still yet another aspect of the present disclosure, there is provided a peptide comprising the monomer represented by Formula 1, or a dimer thereof:

<Formula 1> X₁X₂X₃X₄X₅X₆X₇X₈X₉X₁₀

In Formula 1, X₁, X₄, X₅, and X₈ are each independently leucine (L), phenylalanine (F), tyrosine (Y), or cyclohexylalanine (Cha); X₃, X₆, X₇, and X₁₀ are each independently a hydrophilic amino acid; X₂ and X₉ are each independently an amino acid that forms a bond so that two monomers can be linked to each other at one or more positions selected from X₂ and X₉ to form a dimeric peptide; and X₁ is N-terminus and X₁₀ is C-terminus.

In an embodiment, X₁, X₄, X₅, and X₈ in Formula 1 may be each independently leucine (L), phenylalanine (F), or cyclohexylalanine (Cha).

In another embodiment, at least one of X₁, X₄, X₅, and X₈ in Formula 1 may be each independently cyclohexylalanine (Cha).

In an embodiment, X₃, X₆, X₇, and X₁₀ in Formula 1 may be each independently arginine (R), lysine (K), homoarginine (hR), norarginine (norR), histidine (H), omithine (O), diaminobutanoic acid (Dab), or diaminopropanoic acid (Dap).

In another embodiment, X₃, X₆, X₇, and X₁₀ in Formula 1 may be each independently arginine (R), lysine (K), homoarginine (hR), norarginine (norR), or histidine (H).

In an embodiment, X₂ and X₉ in Formula 1 may be each independently cysteine (C), homocysteine (Hcy), penicillamine (Pen), selenocysteine (U), or leucine (L), provided that X₂ and X₉ are not leucine (L) at the same time.

In another embodiment, the dimer may be such that the two peptides, each of which comprises the monomer represented by Formula 1, are linked to each other in an antiparallel direction.

In yet another embodiment, the peptide or the dimer thereof may interact with cardiolipin in a mitochondrial membrane.

According to still yet another aspect of the present disclosure, there is provided a peptide or a dimer thereof, the peptide comprising a monomer consisting of an amino acid sequence of any one of SEQ ID NOS: 1 to 19.

According to still yet another aspect of the present disclosure, there is provided a method for restoring mitochondrial function comprising a step of administering to a subject in need of restoration of mitochondrial function, a peptide comprising the monomer represented by Formula 1, or a dimer thereof.

### Advantageous Effects of Invention

The amphipathic, α-helical peptide or the dimeric peptide thereof according to the present disclosure exhibits very high cell-penetrating ability and damaged mitochondria-targeting ability even at low concentrations. In addition, it can specifically and strongly interact with cardiolipin contained in the inner mitochondrial membrane (IMM), thereby exhibiting an effect of enhancing mitochondrial function, preventing mitochondrial dysfunction, or restoring mitochondria to normal function. Such an effect enables the peptide or the dimer thereof according to the present disclosure to prevent or treat diseases accompanied by mitochondrial dysfunction as well as various liver diseases.

### Brief Description of Drawings

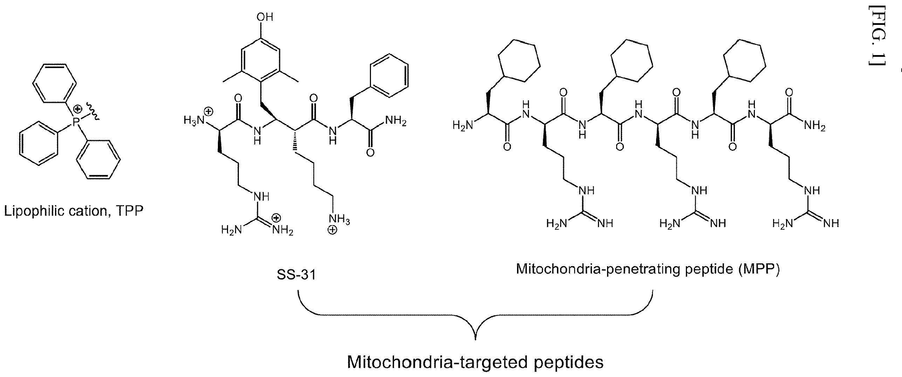

FIG. 1 illustrates examples of representative existing mitochondria-targeting molecules, showing the chemical structures of triphenylphosphonium (TPP) ion, which is a lipophilic cation, SS-31, and mitochondria penetrating peptide (MPP) of Kelly's group, respectively.

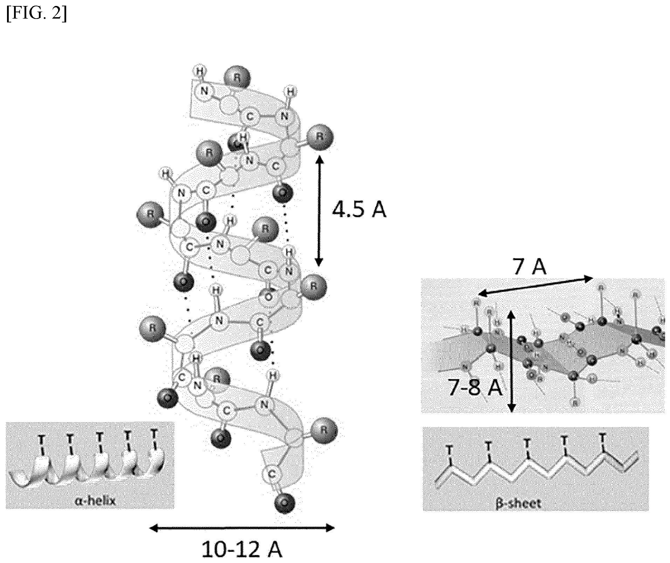

FIG. 2 illustrates an α-helical structure and a β-sheet structure. In the α-helical structure, a length (approximately 4.5 Å) between one side chain and a side chain adjacent thereto and a diameter (10-12 Å) of a helix including side chains are indicated. In the β-sheet structure, a length (about 7 Å) between one side chain and a side chain adjacent thereto and a distance (about 7-8 Å) between a side chain on one side of the sheet and a side chain on the opposite side.

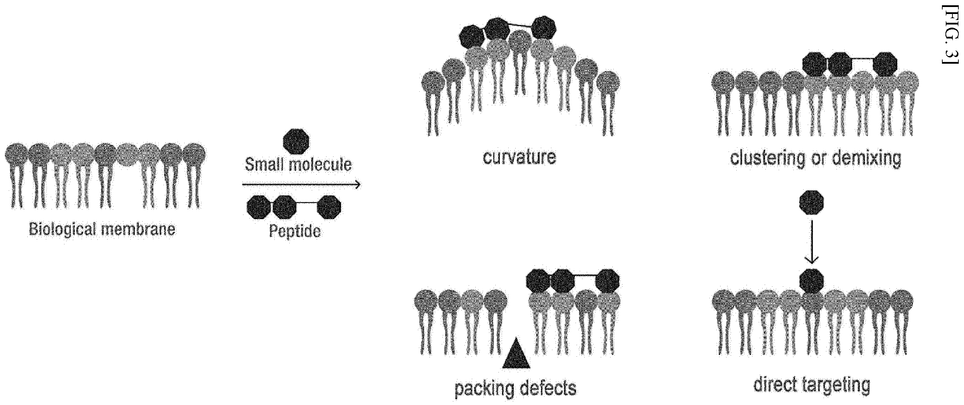

FIG. 3 illustrates a schematic diagram showing a role of a peptide or small molecule for activating biological membranes.

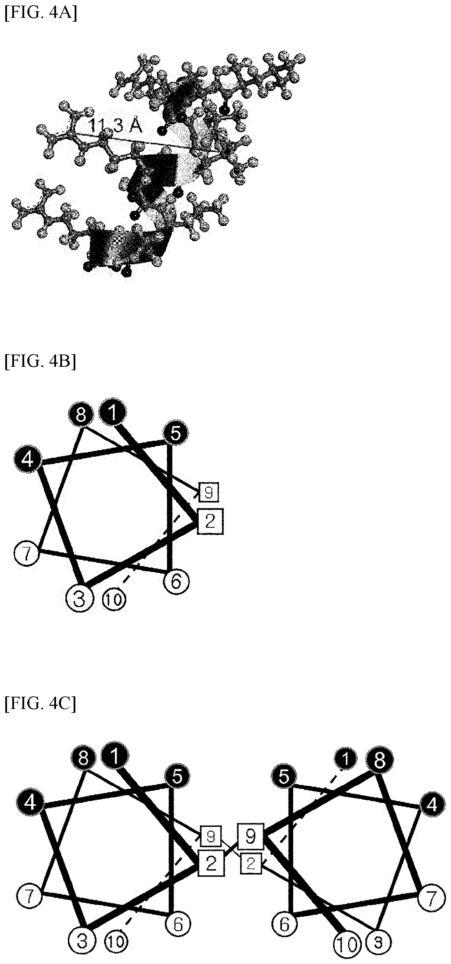

FIGS. 4A to 4C illustrate schematic structures of monomeric and dimeric α-helical peptides, according to an embodiment. FIG. 4A shows a model of an α-helical peptide illustrated using Discovery Studio 2020 (Dassault Systemes BIOVIA, USA). The peptide library monomer was based on the sequence AcNH-LCRLLRRLCR-C(O)NH2 in which calculation was done by using, as a distance monitor, the distance between the two carbon atoms present in the side chains of leucine (L) at position 5 and arginine (R) at position 7. FIG. 4B shows a wheel diagram of the monomeric α-helical peptide. The black circles (amino acids at positions 1, 4, 5, and 8) represent hydrophobic residues, the white circles (amino acids at positions 3, 6, 7, and 10) represent hydrophilic residues, and the white square represents an amino acid residue that forms a bond. FIG. 4C shows a wheel diagram of a dimeric bundle peptide. The structure shown is such that the two peptides are linked to each other in an antiparallel direction.

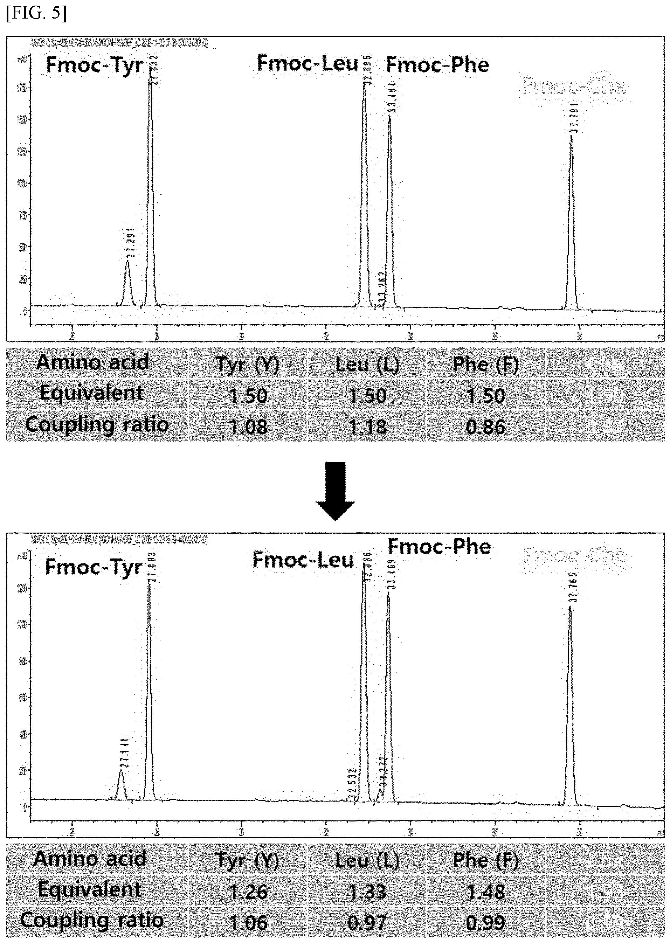

FIG. 5 illustrates HPLC peaks before and after adjusting equivalents of four Fmoc-amino acids so that they produce peptides with the same coupling ratio.

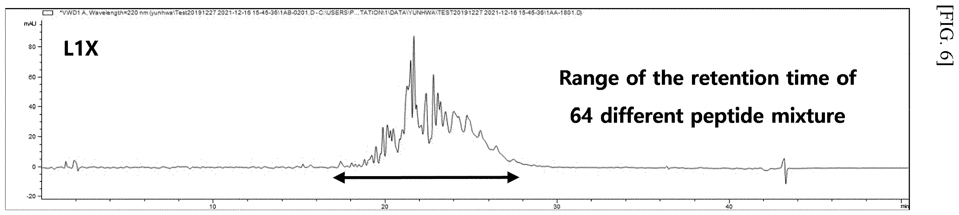

FIG. 6 illustrates an HPLC chromatogram for L1X sub-library that is one of 16 monomeric peptide sub-libraries. The red arrow indicates a range of retention times (RTs) from the first peak to the last peak of the HPLC chromatogram.

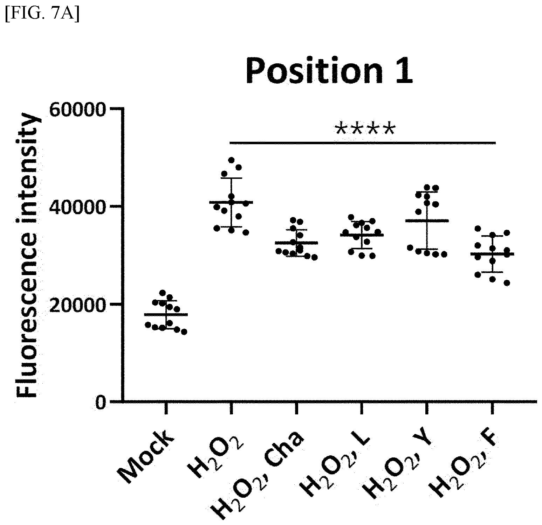

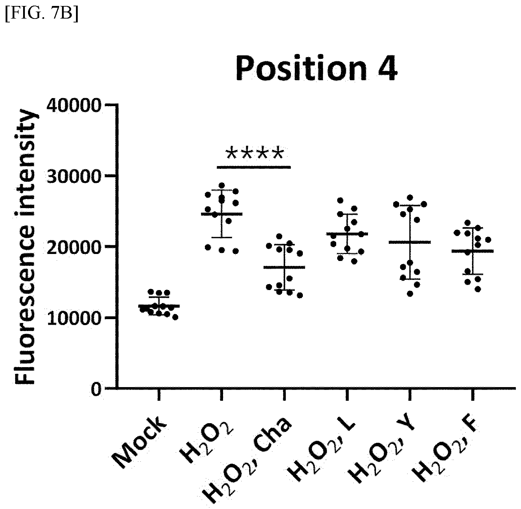

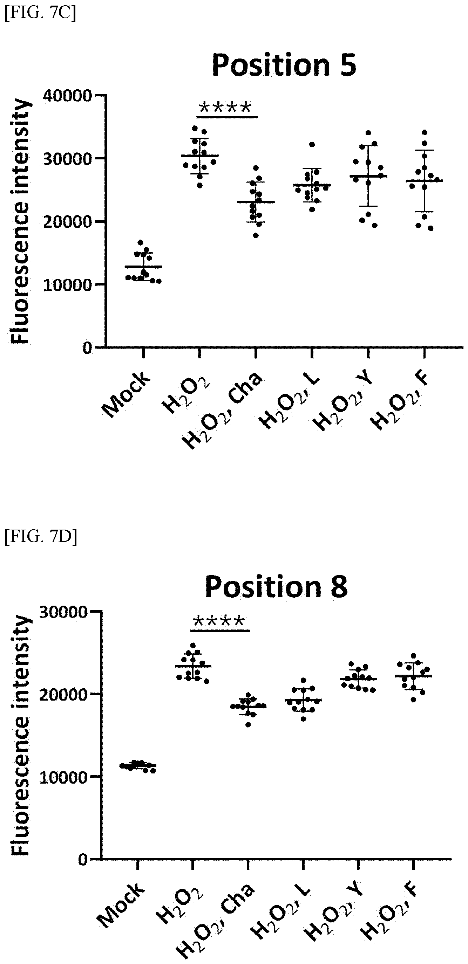

FIGS. 7A to 7D illustrate graphs showing comparison of fluorescence intensity measured to evaluate ROS production inhibiting ability for 16 monomeric peptide sub-libraries: FIG. 7A shows results obtained by measuring ROS levels in HeLa cells upon treatment with four monomeric peptide sub-libraries in which position 1 of the hydrophobic moiety is cyclohexylalanine (Cha), leucine (L), tyrosine (Y), or phenylalanine (F), respectively; FIG. 7B shows results obtained by measuring ROS levels in HeLa cells upon treatment with four monomeric peptide sub-libraries in which position 4 of the hydrophobic moiety is cyclohexylalanine (Cha), leucine (L), tyrosine (Y), or phenylalanine (F), respectively; FIG. 7C shows results obtained by measuring ROS levels in HeLa cells upon treatment with four monomeric peptide sub-libraries in which position 5 of the hydrophobic moiety is cyclohexylalanine (Cha), leucine (L), tyrosine (Y), or phenylalanine (F), respectively; and FIG. 7D shows results obtained by measuring ROS levels in HeLa cells upon treatment with four monomeric peptide sub-libraries in which position 8 of the hydrophobic moiety is cyclohexylalanine (Cha), leucine (L), tyrosine (Y), or phenylalanine (F), respectively.

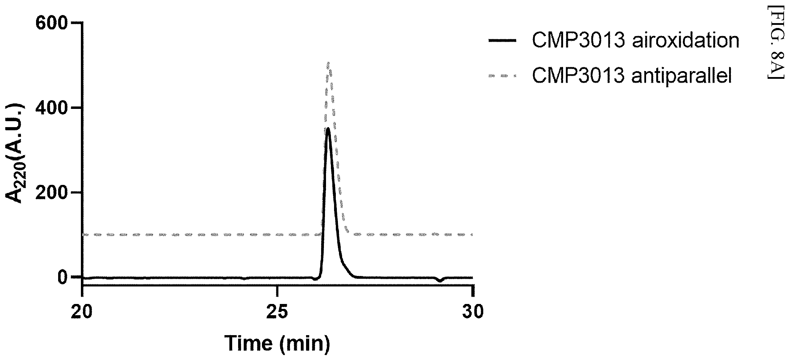

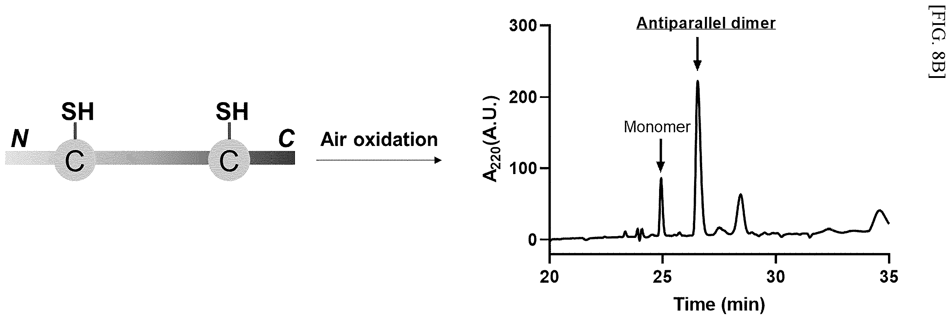

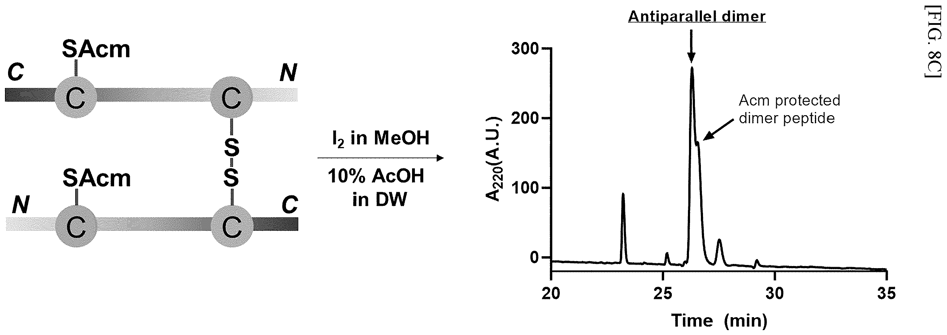

FIGS. 8A to 8C illustrate results obtained by determining a linkage direction (parallel or antiparallel) of dimeric peptides synthesized by air-oxidation or by an air-oxidation method with a protecting group: FIG. 8A shows chromatograms of a dimeric peptide obtained by air-oxidation (labeled CMP3013 airoxidation) and a dimeric peptide obtained by an air-oxidation method with a protecting group (labeled CMP3013 antiparallel); reaction products are shown in blue and major products are underlined and bolded; FIG. 8B shows a schematic diagram of the structure of a monomeric peptide dimerized by air oxidation (left side of arrow) and a chromatogram of the reaction products obtained by the method (right side of arrow). Reaction products are shown in blue and major products are underlined and bolded. FIG. 8C shows a schematic diagram of the structure of an ACM-protected peptide dimerized by an air oxidation method with a protecting group (left side of arrow) and a chromatogram of the reaction products obtained by the method (right side of arrow).

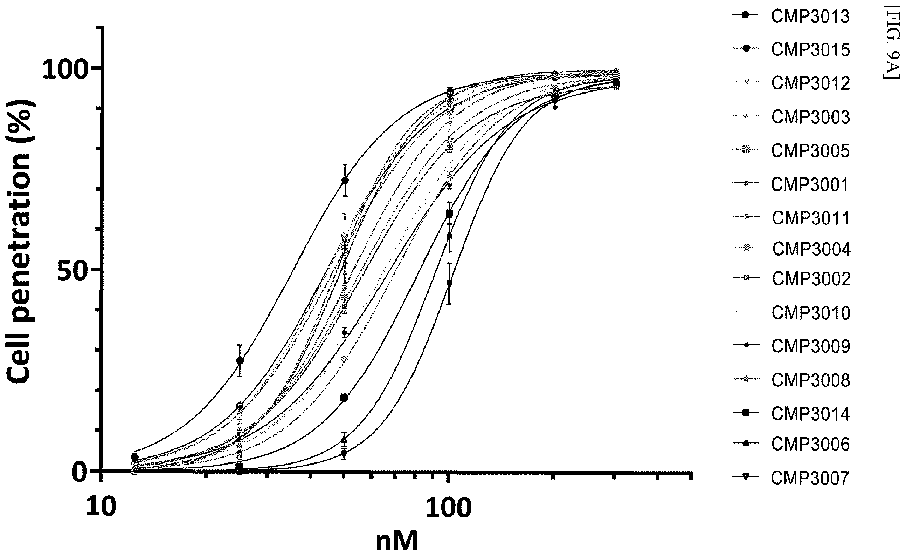

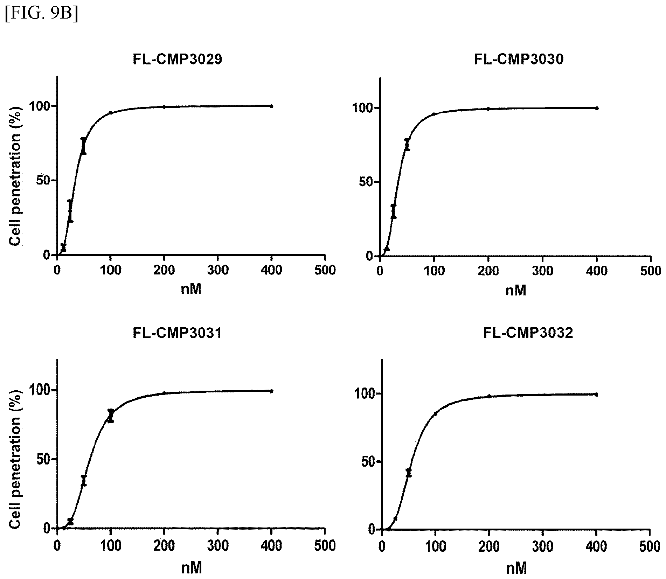

FIGS. 9A and 9B illustrate cell-penetrating ability of each of the fluorescently-labeled dimeric bundle peptides FL-CMP3001 to FL-CMP3015 and FL-CMP3029 to FL-CMP3032: FIG. 9A graphically shows a cell penetration rate of each of FL-CMP3001 to FL-CMP3015 depending on concentrations (legend indicates EC₅₀ values of the peptides in ascending order); and FIG. 9B graphically shows a cell penetration rate of each of FL-CMP3029 to FL-CMP3032 depending on concentrations.

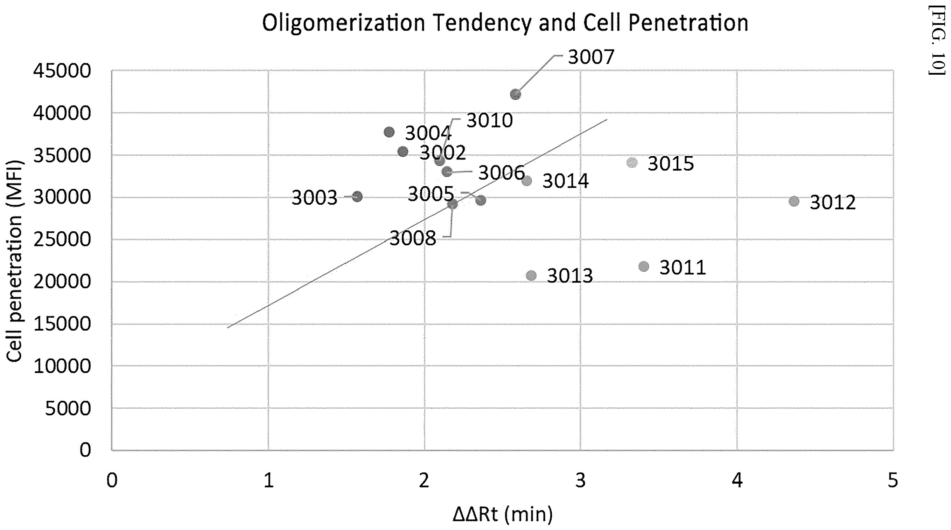

FIG. 10 graphically illustrates correlation between cell-penetrating ability of each of FL-CMP3001 to FL-CMP3015 and oligomerization tendency of each dimeric peptide.

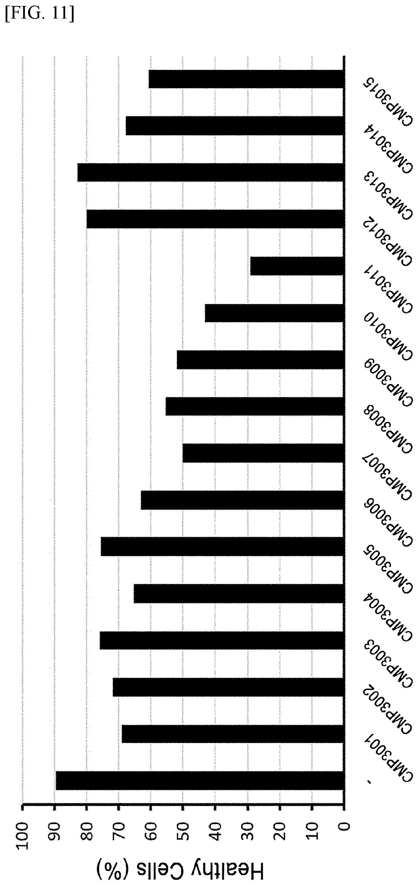

FIG. 11 illustrates a graph showing comparison of cytotoxicity of each of FL-CMP3015 to FL-CMP3031.

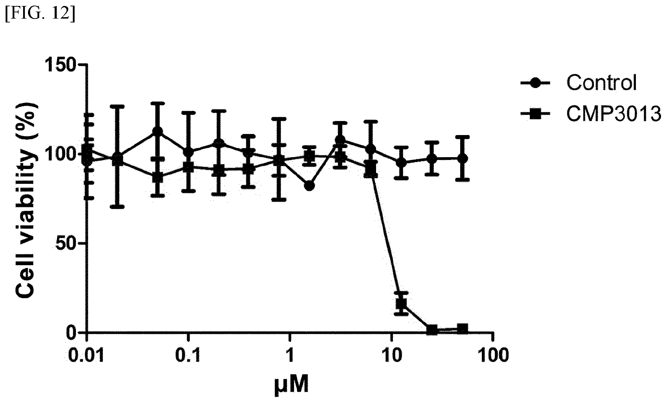

FIG. 12 illustrates a graph showing results obtained by performing WST-1 analysis 24 hours after treating HeLa cells with CMP3013 at different concentrations.

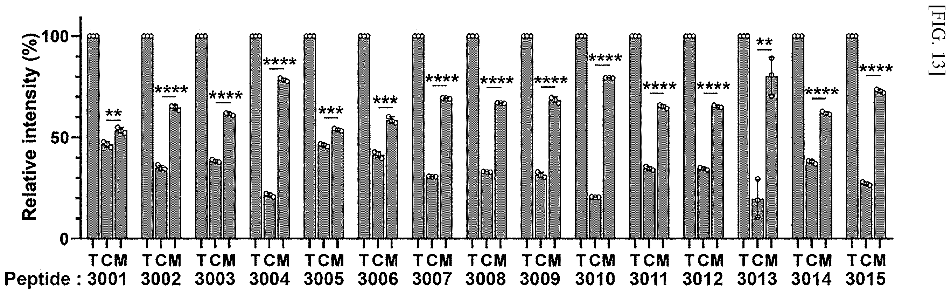

FIG. 13 illustrates results obtained by fractionating HeLa cells that have been treated with each of FL-CMP3001 to FL-CMP3015 at its EC₅₀ concentration for 3 hours. The results are expressed as fluorescence intensity of cytosolic (C) and mitochondrial (M) fractions which is a value relative to the value for the entire cell (Total, T).

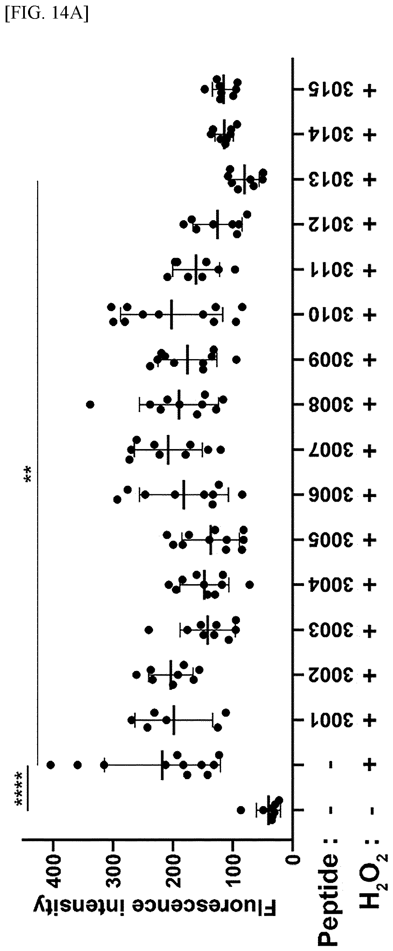

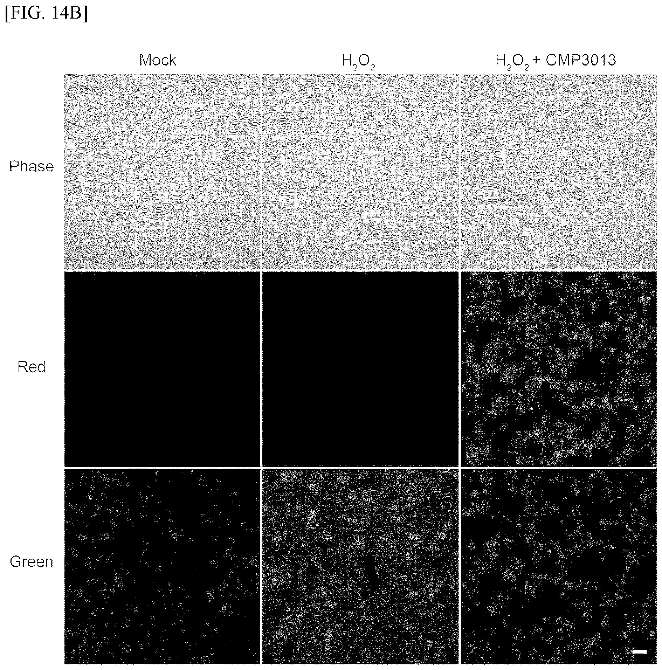

FIGS. 14A and 14B illustrate results obtained by measuring ROS production inhibiting ability of dimeric bundle peptides according to an embodiment: FIG. 14A shows ROS production inhibiting ability of each of FL-CMP301 to FL-CMP3015 peptides in a case where ROS is produced by treatment with 0.5 mM H₂O₂ in HeLa cells. Each peptide was applied at its EC₅₀ concentration for 3 hours. After treatment with 0.5 mM H₂O₂, the cells were imaged by confocal microscopy, and fluorescence intensity thereof was calculated with an image analysis program. Each dot corresponds to the fluorescence intensity obtained from one image. FIG. 14B shows a confocal microscopic image obtained after HeLa cells were treated with 35 nM of FL-CMP3013 for 3 hours, stained with H₂DCFDA, and then treated with 500 µM of H₂O₂ for 30 minutes to induce oxidative stress (Scale bar = 50 µm).

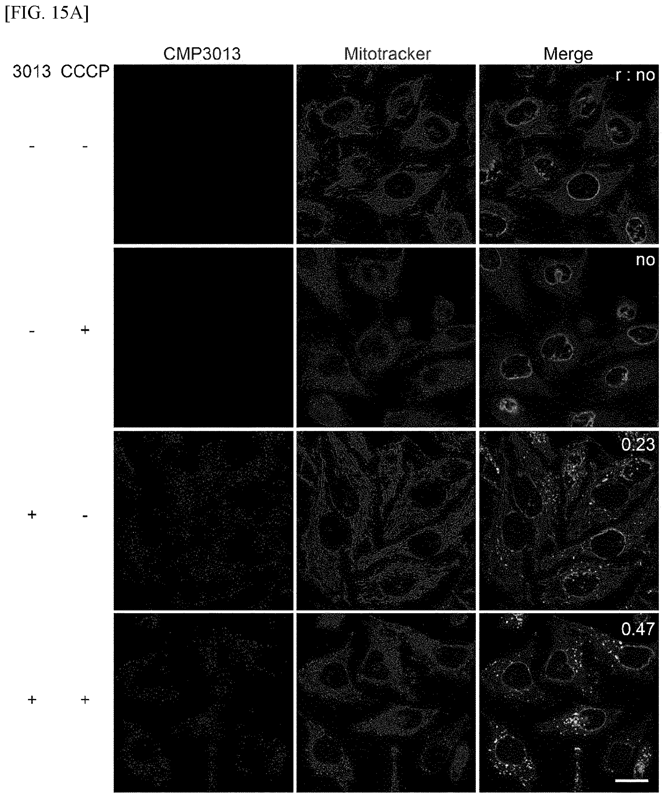

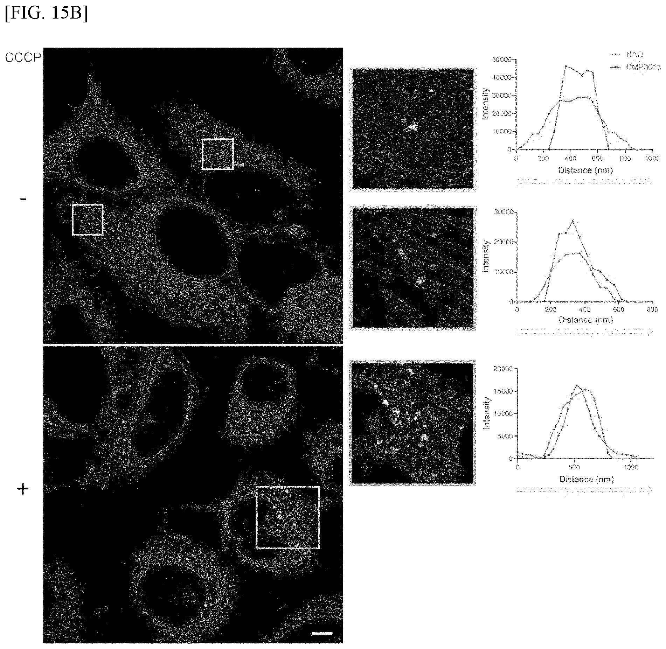

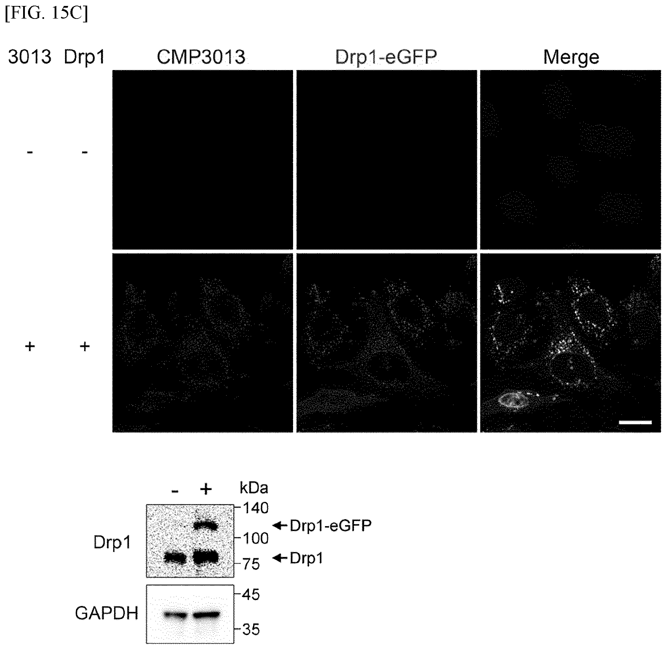

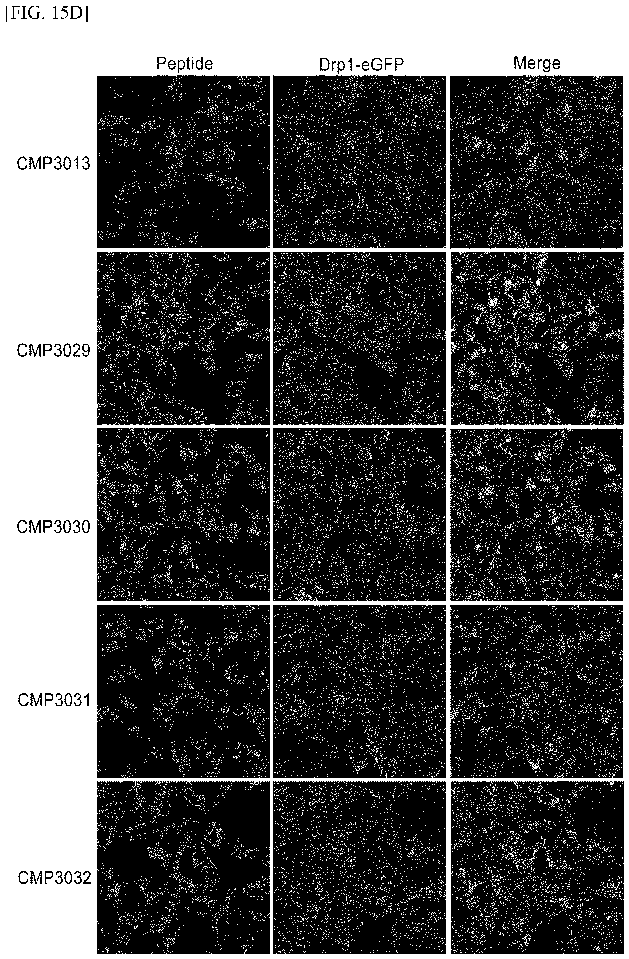

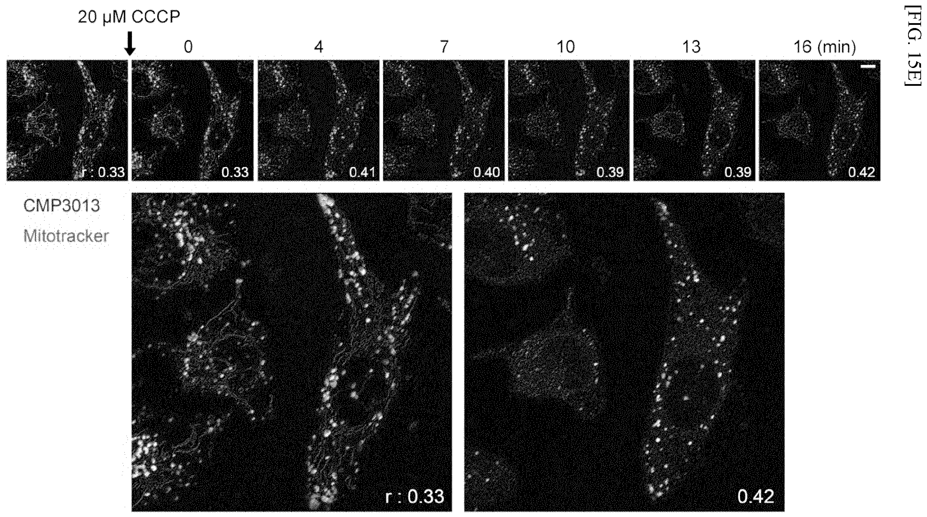

FIGS. 15A to 15E illustrate microscopic images that identify an effect of FL-CMP3013, FL-CMP3029, FL-CMP3030, FL-CMP3031, and/or FL-CMP3032 preferentially moving to or binding to damaged mitochondria. FIG. 15A shows a confocal microscopic image obtained by treating HeLa cells with 100 nM of FL-CMP3013 for 3 hours, performing staining with mitotracker, and then treating the HeLa cells with 20 µM of CCCP for 30 minutes to damage mitochondria. Pearson r value means correlation of red (FL-CMP3013) and green (MitoTracker) fluorescence signals (Scale bar = 20 µm). FIG. 15B shows a ultra-high resolution confocal microscopic image obtained by treating HeLa cells with 100 nM of FL-CMP3013 for 3 hours, performing staining with NAO to label cardiolipin, and then treating the HeLa cells with 20 µM of CCCP for 30 minutes to damage mitochondria (Scale bar = 5 µm). The graphs shown in the right panel illustrate correlation of green (NAO) and red (CMP3 013) fluorescence signals in the portions that are marked with the arrow in the images; and the top panel in FIG. 15C shows confocal microscopic images taken after transfecting HeLa cells with Drp1-eGFP plasmid to induce mitochondrial fission, followed by treatment with 100 nM of FL-CMP3013 for 3 hours the next day (Scale bar = 20 µm). The bottom panel illustrates results obtained by identifying the level of ectopic expression of Drp1 (+, Drp1-eGFP transfected) with Western blotting under the same conditions. FIG. 15D shows confocal microscopic images taken after transfectingHeLa cells with Drp1-eGFP plasmid to induce mitochondrial fission, followed by treatment with FL-CMP3013, FL-CMP3029, FL-CMP3030, FL-CMP3031, or FL-CMP3032, respectively, the next day. FIG. 15E shows time-lapse images obtained by treating HeLa cells with FL-CMP3013 (100 nM, 3 hours) and a mitotracker, and inducing mitochondrial damage by treatment with 20 µM of CCCP while imaging the cells by confocal microscopy (Scale bar = 10 µm).

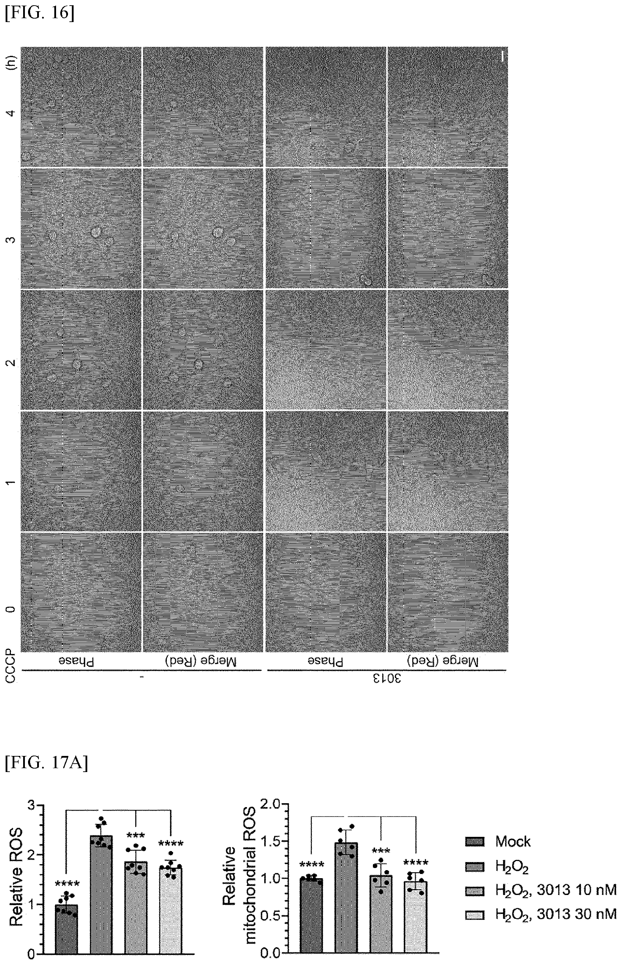

FIG. 16 illustrates microscopic images showing comparison of cell survival rates for cases where HeLa cells whose death was induced by 20 µM CCCP were treated or not treated with FL-CMP3013 (red) (scale bar = 20 µm).

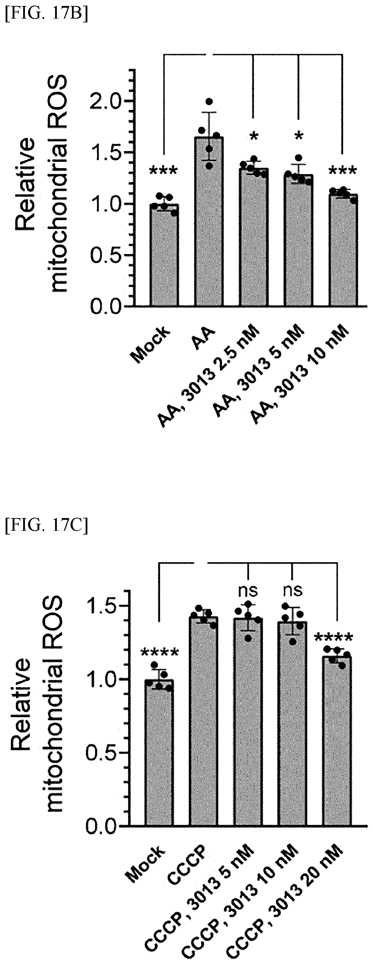

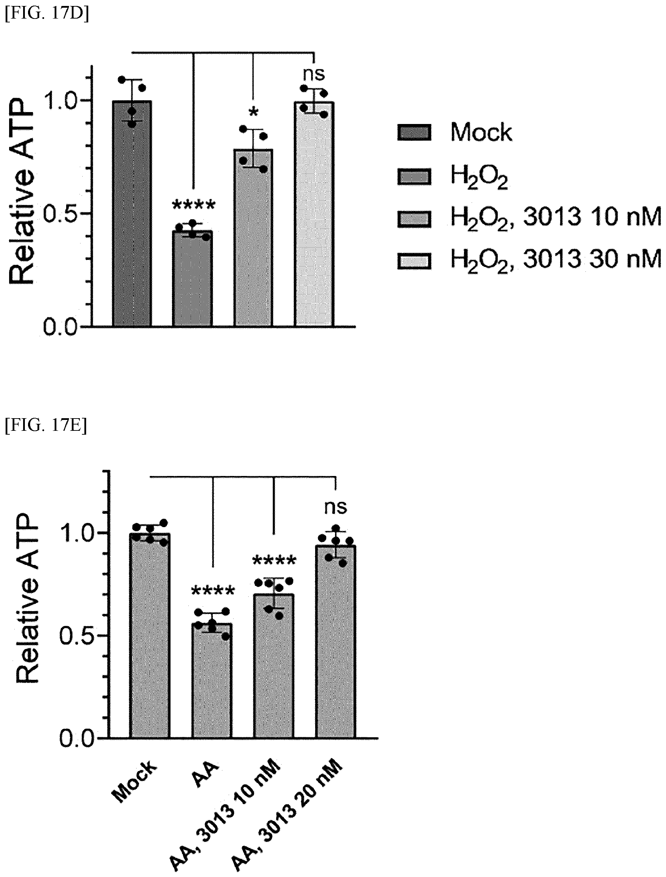

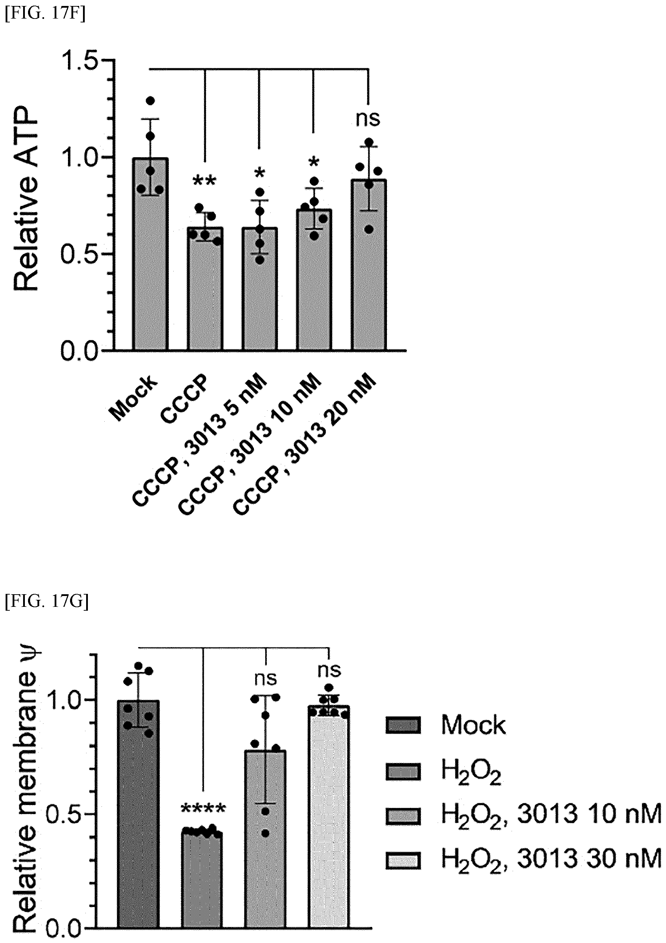

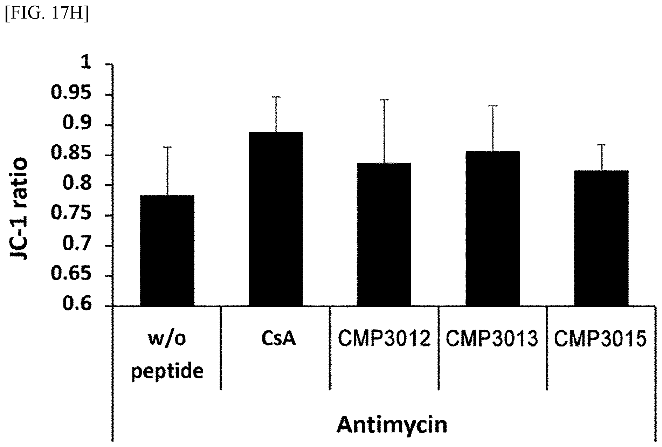

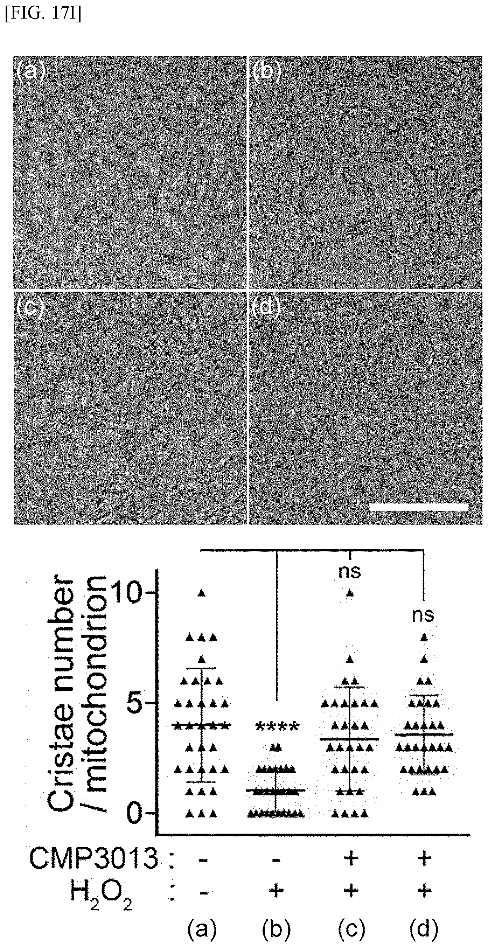

FIGS. 17A to 17I illustrate experimental results that identify an effect of FL-CMP3013 in oxidatively damaged cells on maintenance/restoration of mitochondrial function and protection of cristae within the inner mitochondrial membrane: FIG. 17A illustrates results obtained by measuring levels of total ROS (left graph) and mitochondrial ROS (right graph) after HeLa cells, which had been pretreated with FL-CMP3013 (10 or 30 nM) for 3 hours, were treated with 0.5 mM of H₂O₂ for 30 minutes; FIG. 17B illustrates results obtained by measuring levels of mitochondrial ROS after HeLa cells, which had been pretreated with FL-CMP3013 (2.5, 5, or 10 nM) for 3 hours, were treated with 5 µM antimycin A (AA) as a mitochondrial damaging agent for 90 minutes; FIG. 17C illustrates results obtained by measuring levels of mitochondrial ROS after HeLa cells, which had been pretreated with FL-CMP3013 (5, 10, or 20 nM) for 3 hours, were treated with 10 µM of CCCP as a mitochondrial damaging agent for 90 minutes; FIG. 17D illustrates results obtained by measuring levels of ATP after HeLa cells, which had been pretreated with FL-CMP3013 (10 or 30 nM) for 3 hours, were treated with 1 mM of H₂O₂ for an hour; FIG. 17E illustrates results obtained by measuring levels of ATP after HeLa cells, which had been pretreated with FL-CMP3 013 (10 or 20 nM) for 3 hours, were treated with 1 µM of antimycin A as a mitochondrial damaging agent for 3 hours; FIG. 17F illustrates results obtained by measuring levels of ATP after HeLa cells, which had been pretreated with FL-CMP3 013 (5, 10, or 20 nM) for 3 hours, were treated with 10 µM of CCCP as a mitochondrial damaging agent for an hour; FIG. 17G illustrates results obtained by measuring mitochondrial membrane potential (ψ) after HeLa cells, which had been pretreated with FL-CMP3013 (10 or 30 nM) for 3 hours, were treated with 1 mM of H₂O₂ for an hour; FIG. 17H illustrates results obtained by subjecting SH-SY5Y cells, in which a decrease in membrane potential was induced by antimycin A, to treatment with cyclosporine A (CsA), FL-CMP3012, FL-CMP3013, or FL-CMP3015, and then measuring the relative change in mitochondrial membrane potential as a JC-1 fluorescence ratio; FIG. 17I illustrates transmission electron microscopy (TEM) images of HeLa cells obtained depending on treatment conditions of FL-CMP3013 and H₂O₂. Negative control (mock) (a), treatment with 0.5 mM H₂O₂ (b), treatment with 50 nM of CMP3013 (c) or with 100 nM of CMP3013 (d) for 2 hours followed by treatment with 0.5 mM H₂O₂ for additional 2 hours (Scale bar = 1 µm). The graphs show results obtained by counting and quantifying cristae, which is larger than 0.5 µm, from at least 50 independent mitochondrial images.

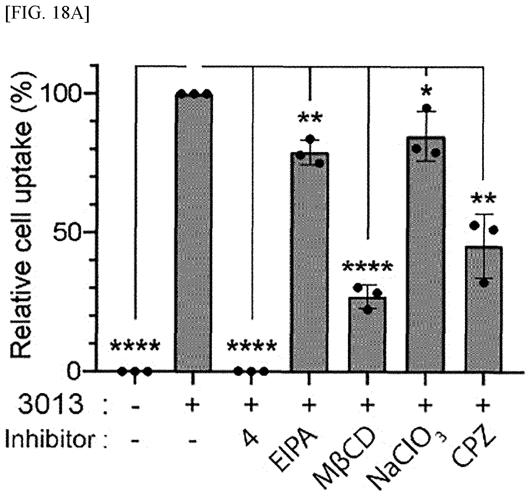

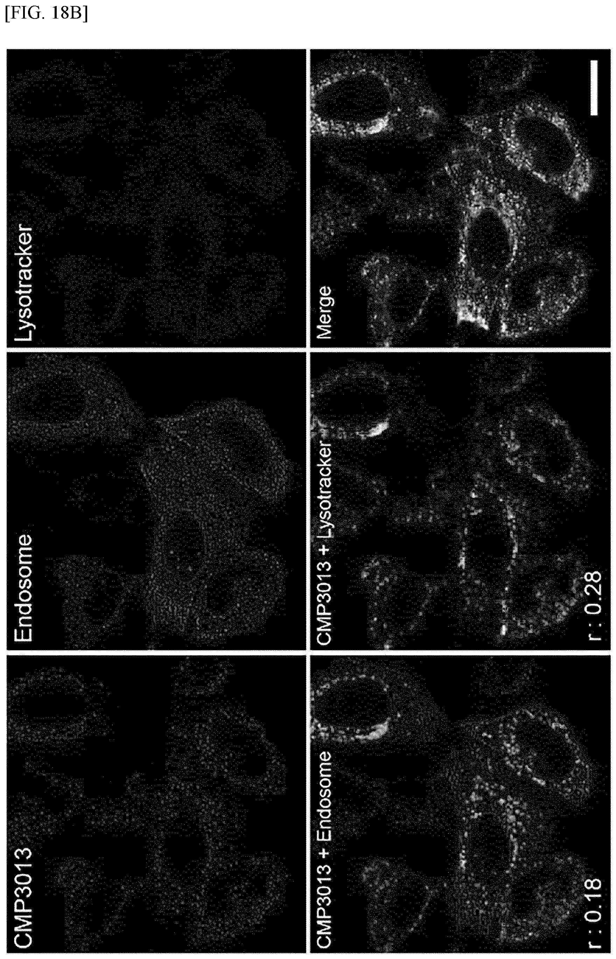

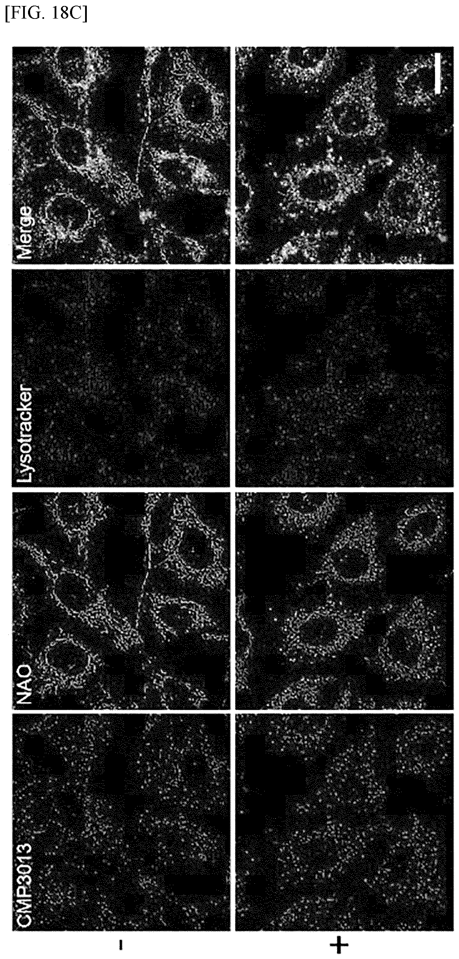

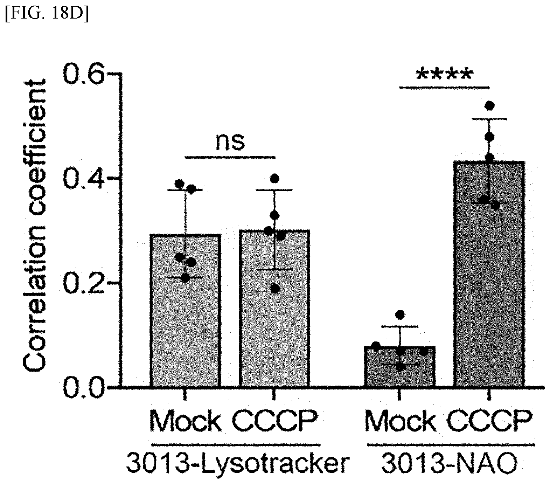

FIGS. 18A to 18D illustrate experimental results that identify the mechanism of intracellular entry of FL-CMP3013 and its final fate in cells: FIG. 18A illustrates results obtained by subjecting HeLa cells to pre-treatment under various inhibitory conditions for 30 minutes (pre-cooling to 4°C, treatment with EIPA [5-(N-ethyl-N-isopropyl)amiloride], treatment with MβCD (methyl-β-cyclodextrin), treatment with sodium chlorate (NaClO3), or treatment with chlorpromazine (CPZ)), performing incubation with 100 nM of FL-CMP3013 for 3 hours, and then performing analysis with FACS; FIG. 18B illustrates confocal microscopic images (r, Pearson correlation coefficient; Scale bar = 20 µm) taken after treating HeLa cells, which express the CellLight^{™} early endosomes-GFP marker, with FL-CMP3013 (100 nM, 3 hours) and LysoTracker^{™} (50 nM, 1 hour); FIG. 18C illustrates confocal microscopic images that identify cardiolipin-specific targeting (with 100 nM NAO, 1 hour) of FL-CMP3013 (100 nM, 3 hours) in the absence (-) or presence (+, 20 µM, 30 min) of CCCP (Scale bar = 20 µm); FIG. 18D illustrates a graph showing comparison of Pearson r values in the microscopic images of FIG. 18C, where the blue bars on the left represent the correlation coefficient for CMP3013-Lysotracer and the green bars on the right represent the correlation coefficient for CMP3013-NAO. Five independent fluorescence images (magnification 63X) were analyzed for each condition.

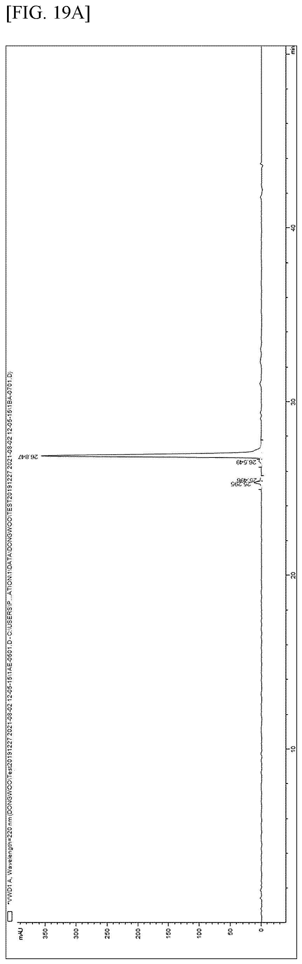

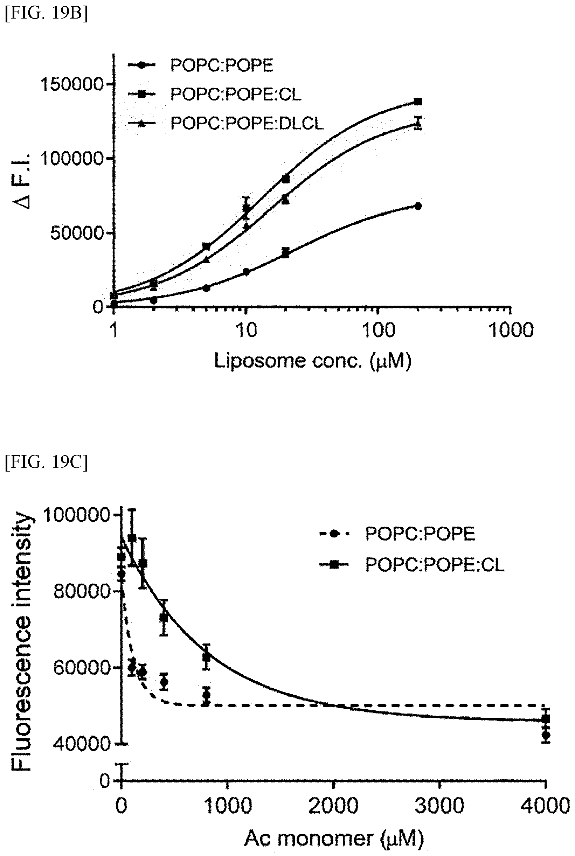

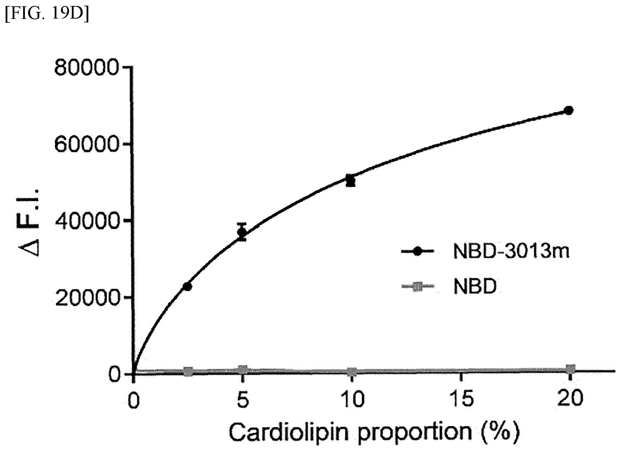

FIGS. 19A to 19D illustrate that CMP3013m (monomeric peptide) strongly interacts with cardiolipin (CL)-containing membranes produced by liposomes: FIG. 19A illustrates an HPLC chromatogram of the monomeric peptide NBD-CMP3013 labeled with NBD (> 95% purity); FIG. 19B illustrates a graph showing changes in fluorescence intensity (ΔF.I.) depending on liposome concentrations in a case where three types of liposome (POPC:POPE = 2:1, black graph; POPC:POPE = 2:1, 10% CL, blue graph; POPC:POPE = 2:1, 10% DLCL, red graph) are added to 1 µM of NBD-labeled CMP3013 monomeric peptide (NBD-CMP3013m); FIG. 19C illustrates a graph obtained by adding liposome to 1 µM of NBD-3013m, performing treatment with an excess of CMP3013 monomer (CMP3013m), and then measuring fluorescence intensity depending on concentrations of CMP3013m; and FIG. 19D illustrates a graph showing changes in fluorescence intensity (ΔF.I.) depending on proportions of cardiolipin in a case where liposomes containing various proportions of cardiolipin (0 to 20%) are incubated with NBD-CMP3013m or NBD dye (6-NBD, 6-(7-nitrobenzofurazan-4-ylamino)hexanoic acid; n = 3).

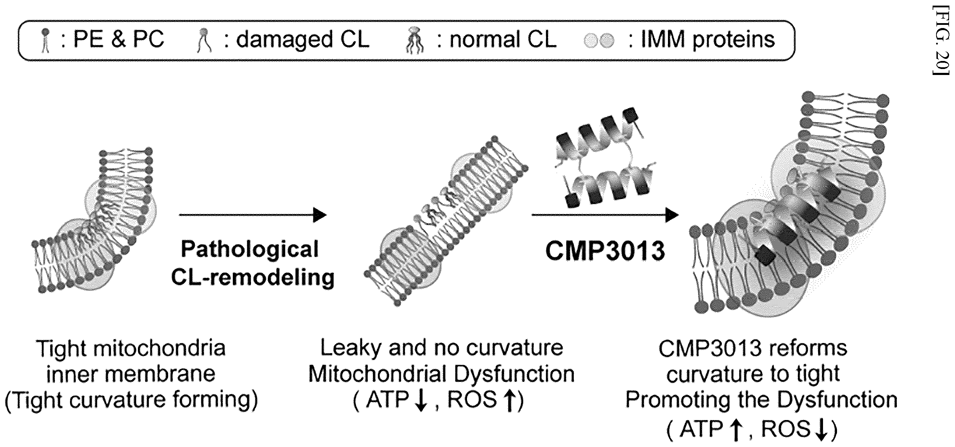

FIG. 20 illustrates a schematic diagram that explains the mechanism of action of CMP3013 according to an embodiment of the present disclosure.

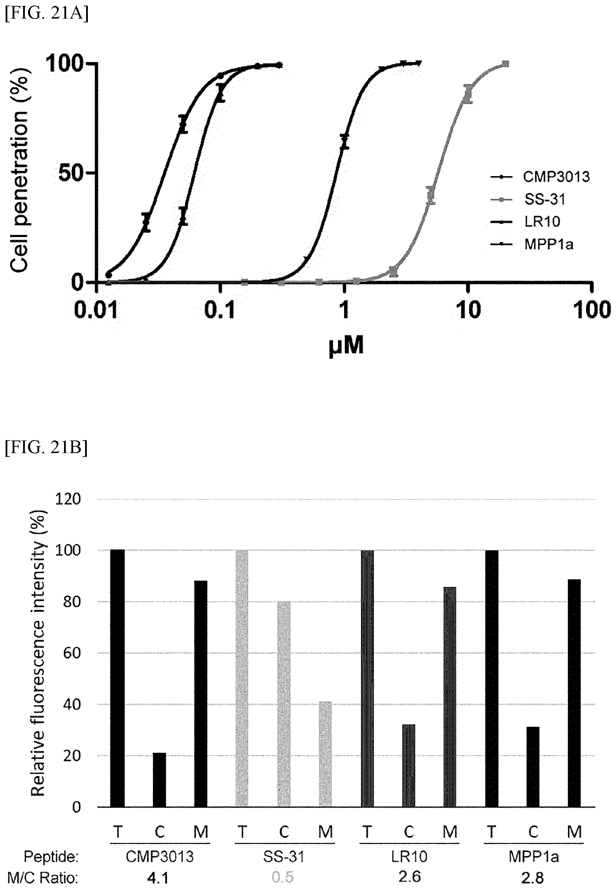

FIGS. 21A and 21B illustrate cell-penetrating ability and mitochondria-targeting ability of CMP3013, SS-31, LR10, and MMP1a: FIG. 21A graphically illustrates the cell penetration rate depending on concentrations of CMP3013, SS-31, LR10, and MMP1a; FIG. 21B illustrates results obtained by fractionating HeLa cells that have been treated with each of CMP3013, SS-31, LR10, and MMPla at its EC₅₀ concentration. The results are expressed as fluorescence intensity of cytosolic (C) and mitochondrial (M) fractions which is a relative value to a value for the entire cell (Total, T) (M/C ratio, relative fluorescence intensity of cytosolic fraction/relative fluorescence intensity of mitochondrial fraction).

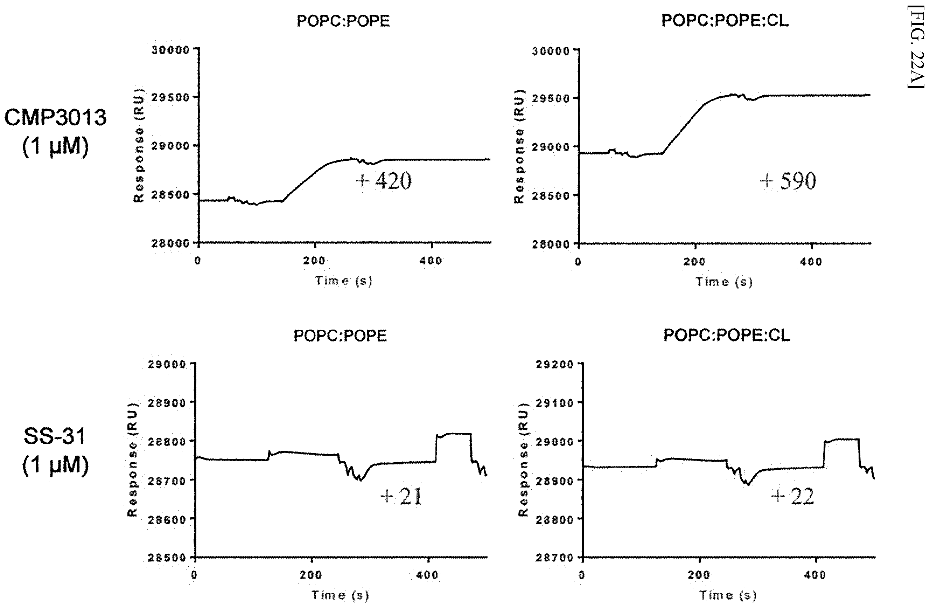

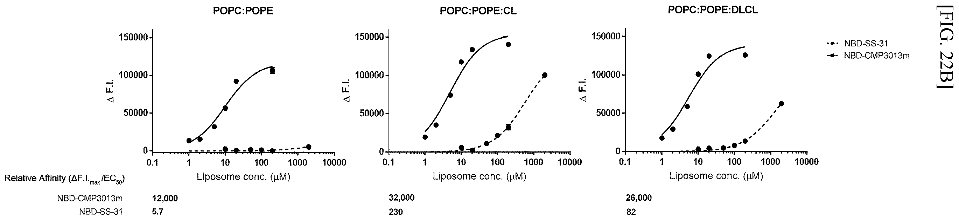

FIGS. 22A and 22B illustrate results showing comparison of the ability of NBD-CMP3013m and NBD-SS-31 to interact with cardiolipin (CL)-containing membranes produced by liposomes: FIG. 22A illustrates results obtained by subjecting each endothelial membrane model (POPC:POPE = 2:1; or POPC:POPE = 2:1, 10% cardiolipin), which has been produced on a sensor chip, to treatment with 1 µM of CMP3013 or SS-31, and then measuring reaction unit (RU) using a surface plasmon resonance technique; FIG. 22B a graph showing changes in fluorescence intensity (ΔF.I.) depending on liposome concentrations in a case where three types of liposome (POPC:POPE = 2:1; POPC:POPE = 2:1, 10% cardiolipin; or POPC:POPE = 2: 1, 10% DLCL) are added to 1 µM of NBD-CMP3013m orNBD-SS-31. At the bottom of each graph, relative affinity of the peptides is expressed as the ΔF.I.ₘₐₓ /EC₅₀ value.

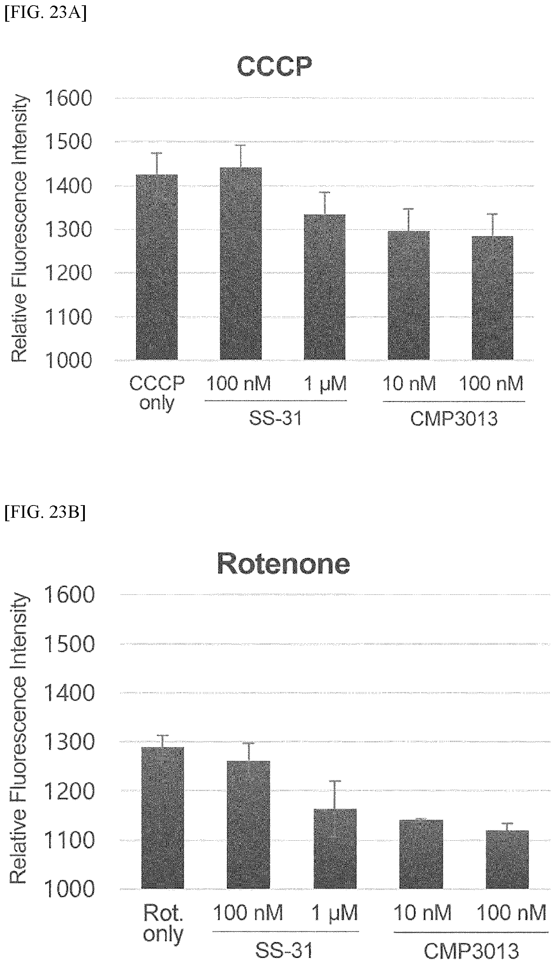

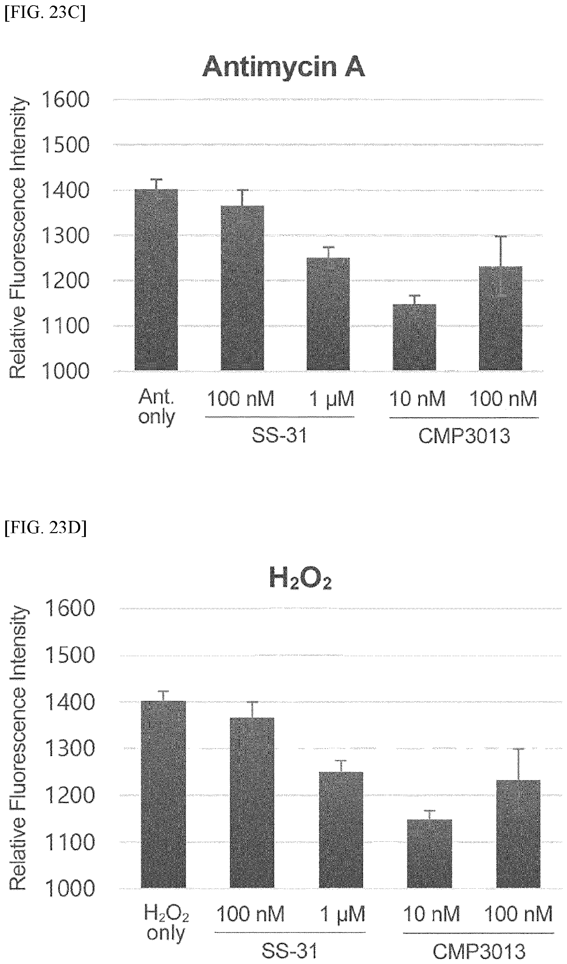

FIGS. 23A to 23D illustrate results showing comparison of the ROS production inhibiting ability of SS-31 and CMP3013: FIG. 23A graphically illustrates the relative fluorescence intensity of cells that have been pretreated with SS-31 (100 nM or 1 µM) or CMP3013 (10 nM or 100 nM), followed by treatment with CCCP to induce mitochondrial damage; FIG. 23B graphically illustrates the relative fluorescence intensity of cells that have been pretreated with SS-31 (100 nM or 1 µM) or CMP3013 (10 nM or 100 nM), followed by treatment with Rotenone to induce mitochondrial damage; FIG. 23C graphically illustrates the relative fluorescence intensity of cells that have been pretreated with SS-31 (100 nM or 1 µM) or CMP3013 (10 nM or 100 nM), followed by treatment with Antimycin A to induce mitochondrial damage; and FIG. 23D graphically illustrates the relative fluorescence intensity of cells that have been pretreated with SS-31 (100 nM or 1 µM) or CMP3013 (10 nM or 100 nM), followed by treatment with hydrogen peroxide (H₂O₂) to induce mitochondrial damage.

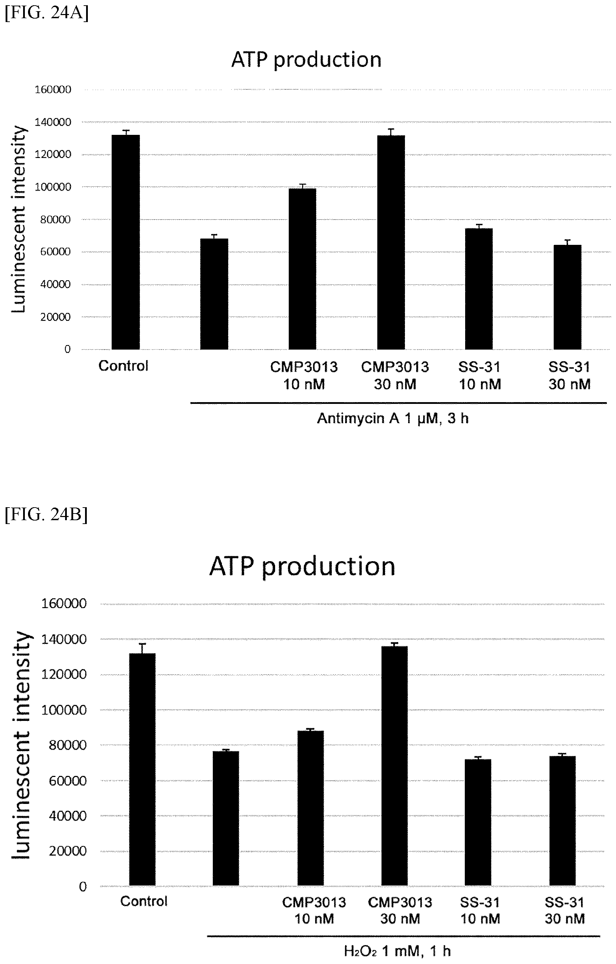

FIGS. 24A to 24B illustrate results showing comparison of the ATP production promoting ability of SS-31 and CMP3013: FIG. 24A illustrates results obtained by treating HeLa cells, which have been pretreated with CMP3013 (10 or 30 nM) or SS-31 (10 or 30 nM), with 1 µM of antimycin A for 3 hours, and then measuring levels of ATP; and FIG. 24B illustrates results obtained by treating HeLa cells, which have been pretreated with CMP3013 (10 or 30 nM) or SS-31 (10 or 30 nM), with 1 mM of H₂O₂ for 1 hour, and then measuring levels of ATP.

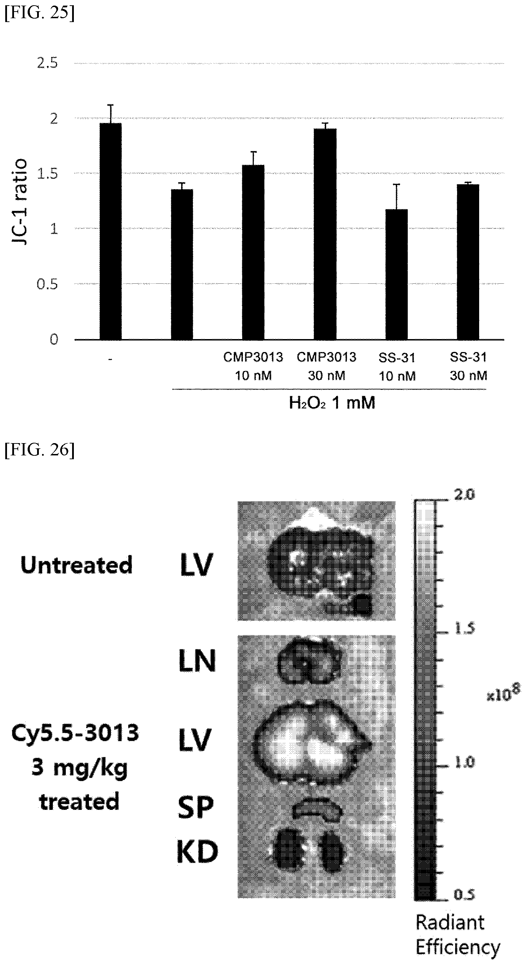

FIG. 25 illustrates results obtained by treating cells, in which a decrease in membrane potential has been induced by H₂O₂, with CMP3013 (10 or 30 nM) or SS-31 (10 or 30 nM), and then measuring the relative change in mitochondrial membrane potential as a JC-1 fluorescence ratio.

FIG. 26 illustrates IVIS images of organs removed from male C57BL/6 mice 24 hours after intravenous injection of CMP3013 peptide labeled with cyanine 5.5 (Cy5.5-3013) at a dose of 3 mg/kg, showing the *in vivo* distribution of CMP3013 (LV, liver; KD, kidney; SP, spleen; LN, lung).

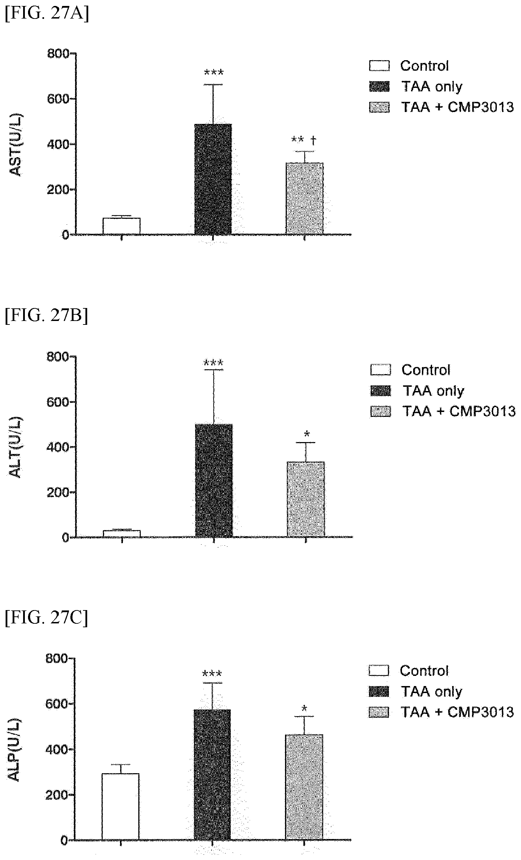

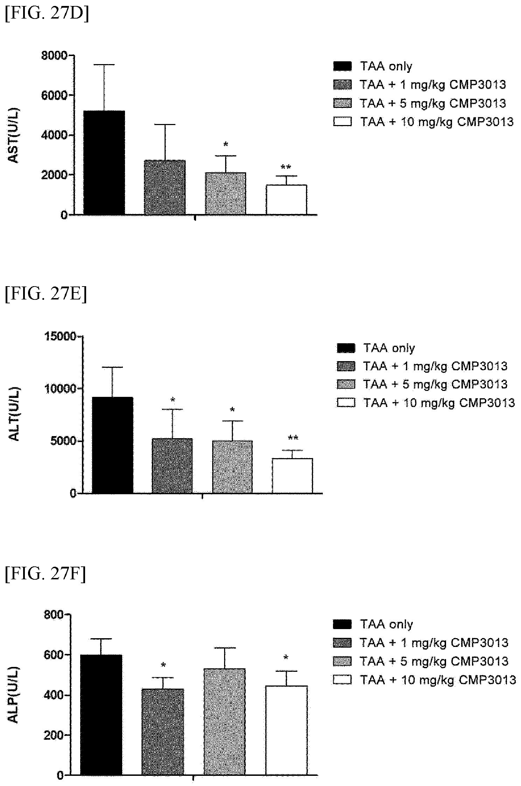

FIGS. 27A to 27F illustrate results that identify the therapeutic effect of CMP3013 for acute liver failure: FIGS. 27A to 27C graphically illustrate results obtained by measuring blood AST (FIG. 27A), ALT (FIG. 27B), and ALP (FIG. 27C), respectively, in mice, in which acute liver failure has been induced by intraperitoneal administration of 100 mg/kg of TAA on the fifth day (Day 5) after intravenous administration of 1 mg/kg of CMP3013 three times every other day (Days 0, 2, and 4); and FIGS. 27D to 27F graphically illustrate results obtained by measuring blood AST (FIG. 27D), ALT (FIG. 27E), and ALP (FIG. 27F), respectively, in mice, in which acute liver failure has been induced by intraperitoneal administration of 100 mg/kg of TAA on the fifth day (Day 5) after intraperitoneal administration of 1, 5, or 10 mg/kg of CMP3013 three times every other day (Days 0, 2, and 4).

### Best Mode for Carrying out Invention

The detailed description to be described later of the present disclosure will be described with reference to specific drawings with respect to specific embodiments in which the present disclosure may be practiced; however, the present disclosure is not limited thereto and, if properly described, is limited only by the appended claims, along with the full scope of equivalents to which such claims are entitled. It should be understood that various embodiments/examples of the present disclosure, although different, are not necessarily mutually exclusive. For example, a particular feature, structure, or characteristic described herein may be changed from one embodiment/example to another embodiment/example or implemented in combinations of embodiments/examples without departing from the technical spirit and scope of the present disclosure. Unless defined otherwise, technical and scientific terms used herein have the same meaning as generally used in the art to which the present disclosure belongs. For purposes of interpreting this specification, the following definitions will apply and whenever appropriate, terms used in the singular will also include the plural and vice versa.

### Definition

As used herein, the term "monomer" or " peptide monomer" refers to a minimum unit that constitutes a monomeric peptide or a dimeric peptide, the monomer consisting of 10 amino acids. The monomer constitutes part or all of a monomeric peptide.

The term "peptide" refers to an amino acid polymer and may comprise, as constituent elements, natural amino acids as well as non-natural amino acids (for example, isomers such as β-amino acids and D-amino acids). For example, when one of the hydrophobic amino acids constituting a peptide is defined as leucine (L), the term "leucine (L)" is used to include not only L-leucine in its natural α-amino acid form, but also its positional isomer, β-leucine, and its enantiomer, D-leucine. Other amino acids described herein (for example, isoleucine (I), phenylalanine (F), tyrosine (Y), valine (V), tryptophan (W), arginine (R), lysine (K), histidine (H), and the like) are also used to include their isomers.

The term "hydrophilic amino acid" may refer to a polar amino acid, and accordingly, it is used to encompass amino acids that have affinity for the polar solvent water, that is, can be mixed with water.

The term "hydrophobic amino acid" may refer to an amino acid that has no or significantly low polarity, and accordingly, it is used to encompass amino acids that have no affinity for the polar solvent water, that is, are not mixed with water.

The term "amphipathic peptide" may be used interchangeably with "double-sided peptide" and refers to a peptide that is structurally double-sided, that is, it has both hydrophobic and hydrophilic properties. One side or moiety of the amphipathic peptide may be hydrophilic, and the other side or moiety may be hydrophobic.

The term "treatment" generally refers to obtaining a desired pharmacological and/or physiological effect. Such an effect has a therapeutic effect in that it partially or completely cures a disease and/or harmful effects caused by the disease. Desirable therapeutic effects include, but are not limited to, prevention of occurrence or recurrence of a disease, improvement of symptoms, reduction of any direct or indirect pathological consequences of a disease, prevention of metastasis, reduction of disease progression rate, improvement or alleviation of disease state, and remission or improved prognosis. Preferably, "treatment" may mean medical intervention for a disease or disorder that has already occurred.

The term "prevention" means obtaining a desired prophylactic pharmacological and/or physiological effect in terms of partially or completely preventing a disease or its symptoms.

The term "administration" refers to providing a substance (for example, a peptide comprising a monomer represented by Formula 1, or dimer thereof, of the present disclosure) to a subject to achieve a prophylactic or therapeutic purpose (for example, prevention or treatment of a disease accompanied by mitochondrial dysfunction).

The term "effective amount" or "therapeutically effective amount" refers to an amount of a compound, composition, or drug that is sufficient to treat a stated condition, pathology, or disease, such as to improve a stated function, for example, to enhance or increase a desired and/or beneficial function, to reduce or decrease an undesired function, or to ameliorate, temporarily relieve, attenuate, and/or delay one or more of the symptoms. For example, with respect to a disease accompanied by mitochondrial dysfunction, the effective amount includes an amount sufficient to delay or restore the mitochondrial dysfunction.

The term "subject" is used interchangeably with "patient" and may be a mammal in need of prevention or treatment of a disease accompanied by mitochondrial dysfunction or other diseases, such as primate (for example, human), companion animal (for example, dog and cat), domestic animal (for example, cow, pig, horse, sheep, and goat), or laboratory animal (for example, rat, mouse, and guinea pig). In an embodiment of the present disclosure, the subject is a human.

As used herein, the term "about" refers to a typical error range for each value known to those skilled in the art. In addition, unless otherwise specified, all numbers, values and/or expressions used herein to express ingredients, conditions, compositions, amounts, and the like are to be understood to be qualified by the term "approximately" as such numbers are inherently approximate reflecting, among other things, the various uncertainties of measurement encountered in obtaining such values.

### Mitochondria-specific peptide monomer

The present disclosure is based, in part, on the surprising discovery that in a case where hydrophobic and hydrophilic amino acids are arranged at specific positions in a peptide, the peptide has excellent cell-penetrating ability and mitochondria-targeting ability, thereby enabling damaged mitochondria to be restored. A monomer of the peptide may consist of a total of 10 amino acids and may comprise hydrophobic and hydrophilic amino acids in a suitable ratio, for example, 1:1, specifically four (4) each, two monomers being linked to each other via a bond at one or two positions to form a dimeric peptide. The peptide monomer may form an α-helical, amphipathic (double-sided) dimeric bundle peptide. The peptide monomer may have an increased α-helical content, which may result in increased intracellular penetrability, and toxicity of the peptide may be reduced due to removal of the α-helix within a cell. Because cell-penetrating ability of the dimer is at least 100 times greater than that of the single-stranded α-helical peptide, once a dimeric bundle is formed, it can be easily delivered into a cell, even at nanomolar concentrations. The disulfide bond of the dimeric bundle peptide, which has been delivered into a cell at a nanomolar concentration, is easily reduced in the intracellular environment so that monomers are formed and monomeric peptides can target mitochondria. It has been experimentally identified that the thus delivered peptide specifically binds to abnormal mitochondria, thereby preventing them from further damage or restoring their function.

According to an aspect of the present disclosure, there is provided a peptide comprising the monomer represented by Formula 1:

<Formula 1> X₁X₂X₃X₄X₅X₆X₇X₈X₉X₁₀

in Formula 1, X₁, X₄, X₅, and X₈ are each independently a hydrophobic amino acid; X₃, X₆, X₇, and X₁₀ are each independently a hydrophilic amino acid; X₂ and X₉ are each independently an amino acid that forms a bond so that two monomers can be linked to each other at one or more positions selected from X₂ and X₉ to form a dimeric peptide; and X₁ is N-terminus and X₁₀ is C-terminus.

In an embodiment, each of the amino acids that constitute the peptide is not limited in terms of its type as long as it is capable of maintaining an α-helical structure while exhibiting amphipathy, and hydrophobic amino acids or hydrophilic amino acids, which are known in the technical field to which the present disclosure belongs, may be appropriately selected and used. In a previous study, the present inventors revealed that the peptide having an increased α-helical content was capable of exhibiting increased intracellular penetrability while exhibiting decreased toxicity due to removal of the α-helix in a case of being present in cells (WO 2015/057009).

In another embodiment, the hydrophobic amino acid may be selected from the group consisting of leucine (L), isoleucine (I), phenylalanine (F), tyrosine (Y), valine (V), norvaline (norV), tryptophan (W), pentylglycine (pg), neopentylglycine (Npg), alanine (A), and cyclohexylalanine (Cha). Accordingly, in the formula, X₁, X₄, X₅, and X₈ may be each independently leucine (L), isoleucine (I), phenylalanine (F), tyrosine (Y), valine (V), norvaline (norV), tryptophan (W), pentylglycine (pg), neopentylglycine (Npg), alanine (A), or cyclohexylalanine (Cha). Preferably, X₁, X₄, X₅, and X₈ may be each independently leucine (L), phenylalanine (F), tyrosine (Y), or cyclohexylalanine (Cha), with leucine (L), phenylalanine (F), and cyclohexylalanine (Cha) being more preferred, but are not limited thereto.

According to yet another embodiment, in Formula 1, at least one (for example, one, two, three, or four) of X₁, X₄, X₅, and X₈ may be cyclohexylalanine (Cha). Here, the remainder of X₁, X₄, X₅, and X₈ may be each independently selected from the group consisting of leucine (L), phenylalanine (F), and tyrosine (Y).

In an embodiment, the hydrophilic amino acid may be a hydrophilic amino acid with a positively charged side chain. For example, the hydrophilic amino acid may be selected from the group consisting of arginine (R), lysine (K), homoarginine (hR), norarginine (norR), histidine (H), omithine (O), diaminobutanoic acid (Dab), and diaminopropanoic acid (Dap). Accordingly, in Formula 1, X₃, X₆, X₇, and X₁₀ may be each independently arginine (R), lysine (K), homoarginine (hR), norarginine (norR), histidine (H), omithine (O), diaminobutanoic acid (Dab), or diaminopropanoic acid (Dap). Preferably, X₃, X₆, X₇, and X₁₀ may be each independently arginine (R), lysine (K), homoarginine (hR), norarginine (norR), or histidine (H), with arginine (R), homoarginine (hR), or norarginine (norR) being more preferred, but are not limited thereto.

According to another embodiment, in Formula 1, X₂ and X₉ may be each independently cysteine (C), homocysteine (Hcy), penicillamine (Pen), selenocysteine (U), or leucine (L), provided that X₂ and X₉ are not leucine (L) at the same time. Preferably, X₂ and X₉ may be each independently cysteine (C), homocysteine (Hcy), or penicillamine (Pen), with cysteine (C) or homocysteine (Hcy) being more preferred.

According to an embodiment, in Formula 1, X₁, X₄, X₅, and X₈ are each independently leucine (L), phenylalanine (F), tyrosine (Y), or cyclohexylalanine (Cha); X₃, X₆, X₇, and X₁₀ are each independently a hydrophilic amino acid; X₂ and X₉ are each independently an amino acid that forms a bond so that two monomers may be linked to each other at one or more positions selected from X₂ and X₉ to form a dimeric peptide, and X₁ is N-terminus and X₁₀ is C-terminus.

According to another embodiment, in Formula 1, X₁, X₄, X₅, and X₈ are each independently a hydrophobic amino acid selected from the group consisting of leucine (L), phenylalanine (F), tyrosine (Y), and cyclohexylalanine (Cha); X₃, X₆, X₇, and X₁₀ are each independently a hydrophilic amino acid selected from the group consisting of arginine (R), lysine (K), homoarginine (hR), norarginine (norR), histidine (H), omithine (O), diaminobutanoic acid (Dab), and diaminopropanoic acid (Dap); X₂ and X₉ are each independently an amino acid that forms a bond so that two monomers may be linked to each other at one or more positions selected from X₂ and X₉ to form a dimeric peptide, and X₁ is N-terminus and X₁₀ is C-terminus.

Preferably, at least one of X₁, X₄, X₅, and X₈ is each independently cyclohexylalanine (Cha) and the remainder thereof may be each independently selected from the group consisting of leucine (L), phenylalanine (F), and tyrosine (Y). Here, preferably, X₃, X₆, X₇, and X₁₀ may be each independently arginine (R), lysine (K), homoarginine (hR), norarginine (norR), or histidine (H), with arginine (R), homoarginine (hR), or norarginine (norR) being more preferred. In addition, X₂ and X₉ may be each independently cysteine (C), homocysteine (Hcy), or penicillamine (Pen), with cysteine (C) or homocysteine (Hcy) being more preferred.

In another embodiment, a peptide comprising the monomer represented by Formula 1 may comprise an amino acid sequence of Hfa-Cys-Arg-Hfa-Hfa-Arg-Arg-Hfa-Cys-Arg, wherein Hfa is or comprises a peptide that is a hydrophobic amino acid selected from the group consisting of leucine (L), phenylalanine (F), tyrosine (Y), and cyclohexylalanine (Cha). Here, at least one of Hfa's may be each independently cyclohexylalanine (Cha).

In yet another embodiment, the monomer represented by Formula 1 may consist of an amino acid sequence of any one of SEQ ID NOS: 1 to 19 listed in Table 1, but is not limited to the listed amino acid sequence as long as its amphipathy and α-helical structure can be maintained.

**[Table 1]**

| **SEQ ID NO.** | **Amino Acid Sequence of monomer (X = Cha)** | **Monomeric peptide name** |
|---|---|---|
| 1 | XCRLLRRLCR | CMP3001m |
| 2 | LCRXLRRLCR | CMP3002m |
| 3 | LCRLXRRLCR | CMP3003m |
| 4 | LCRLLRRXCR | CMP3004m |
| 5 | XCRXLRRLCR | CMP3005m |
| 6 | XCRLXRRLCR | CMP3006m |
| 7 | XCRLLRRXCR | CMP3007m |
| 8 | LCRXXRRLCR | CMP3008m |
| 9 | LCRXLRRXCR | CMP3009m |
| 10 | LCRLXRRXCR | CMP3010m |
| 11 | XCRXXRRLCR | CMP3011m |
| 12 | XCRXLRRXCR | CMP3012m |
| 13 | XCRLXRRXCR | CMP3013m |
| 14 | LCRXXRRXCR | CMP3014m |
| 15 | XCRXXRRXCR | CMP3015m |
| 16 | FCRLXRRXCR | CMP3029m |
| 17 | FCRXXRRXCR | CMP3030m |
| 18 | FCRLXRRLCR | CMP3031m |
| 19 | FCRXXRRLCR | CMP3032m |

A peptide comprising the monomer represented by Formula 1 can be delivered into the cell and interact with the mitochondrial membrane, in particular, cardiolipin in the mitochondrial membrane. In addition, in a case where cells, in which oxidative stress has been induced, are treated with a peptide comprising the monomer represented by Formula 1, production of ROS is inhibited (see Example 2.2.2).

### Mitochondria-specific peptide dimer

According to another aspect of the present disclosure, there is provided a dimeric peptide comprising the above-described peptide as a monomer. The dimeric peptide of this embodiment comprises the monomer represented by Formula 1.

<Formula 1> X₁X₂X₃X₄X₅X₆X₇X₈X₉X₁₀

In Formula 1, X₁ to X₁₀ are as defined above.

In another embodiment, the monomeric peptide represented by Formula 1 may comprise an amino acid sequence of Hfa-Cys-Arg-Hfa-Hfa-Arg-Arg-Hfa-Cys-Arg, wherein Hfa may be a hydrophobic amino acid selected from the group consisting of leucine (L), phenylalanine (F), tyrosine (Y), and cyclohexylalanine (Cha). Here, at least one of Hfa's may be each independently cyclohexylalanine (Cha). In yet another embodiment, the dimeric peptide may comprise a monomeric peptide comprising an amino acid sequence selected from the group consisting of SEQ ID NOS: 1 to 19.

The dimeric peptide of the present disclosure may be formed via bonding of monomeric peptides to each other, in which the bonding may be made at any one or both positions of X₂ and X₉. Accordingly, X₂ and X₉ may be each independently selected from any amino acids that form a bond so that two monomeric peptides may be linked to each other at one or more positions selected from X₂ and X₉ to form a dimeric peptide. For example, X₂ and X₉ may be each independently cysteine (C), homocysteine (Hcy), penicillamine (Pen), selenocysteine (U), or leucine (L), provided that X₂ and X₉ may not be leucine (L) at the same time.

Here, the bond formed between the respective monomeric peptides may include any type of bond that links the peptides to exhibit desired properties according to the present disclosure, and may include, for example, a covalent bond. The covalent bond is not particularly limited as long as it is a type of covalent bond capable of increasing an α-helical content without inhibiting functions of the peptide. For example, the covalent bond may be at least one bond selected from the group consisting of a disulfide bond between cysteines, a diselenide bond, an ester bond, a maleimide bond, a thioester bond, a thioether bond, and a bond formed by a click reaction. More specifically, the covalent bond may be at least one selected from the group consisting of a disulfide bond between cysteine (C), homocysteine (Hcy), or penicillamine (Pen) residues, a diselenide bond between selenocysteine (U) residues, an ester bond, a maleimide bond formed by using a thiol functional group capable of participating in a reaction, a thioester bond, a thioether bond, and a bond formed by a click reaction in a case of containing triple bond- or azide group-containing non-natural amino acids which are capable of causing a click reaction.

In a case where a disulfide bond is formed between two monomers of a dimeric peptide, when cysteine (C) is used in the monomer, four atoms (CSSC; C, carbon; S, sulfur) exist between the two monomeric peptide backbones. Here, when homocysteine (Hcy) is used instead of cysteine (C), it is possible to adjust the distance between the two monomeric peptide backbones to a 5-atom bonding distance (CCSSC or CSSCC) or a 6-atom bonding distance (CCSSCC). In an embodiment, a dimer may be used in which one cysteine and one homocysteine are linked to each other through oxidation. In addition, selenocysteine (U) or penicillamine (Pen) may be used instead of cysteine to form a diselenide bond, hybridization with cysteine or penicillamine, or a disulfide bond of ester.

In an embodiment, the dimeric peptide may be such that peptides (monomeric peptides), each of which comprises the monomer, are linked to each other in a parallel direction where both peptides maintain a N-terminal to C-terminal direction, or in an antiparallel direction where one of the two peptides is in a N-terminal to C-terminal direction and the other is in a C-terminal to N-terminal direction. In another embodiment, peptides (monomeric peptides), each of which comprises the monomer, are linked to each other in an antiparallel direction. Here, the respective monomers may have the same or different sequences from each other.

The dimeric peptide may be lipidated with a fatty acid at the N-terminus. Specifically, the dimeric peptide may have a C₆ to C₁₆ fatty acid bound to a position of X₁. Such fatty acid binding allows the peptide to have further increased cell-penetrating ability.

The dimeric peptide has little or no cytotoxicity at concentrations at which it is effective, and has an effect of restoring various mitochondrial dysfunctions by specifically targeting mitochondria (in particular, cardiolipin present in the inner mitochondrial membrane). According to an embodiment of the present disclosure, it was identified that the dimeric peptide is only cytotoxic at micromolar or higher concentrations, and the concentration thereof, at which 50% of cultured cells are killed, reaches 10 micromolar (see Example 6). In another embodiment of the present disclosure, it was identified that the dimeric peptide is present in significantly higher proportions in the mitochondrial fraction in a case of being applied to cells, and it was further identified that it preferentially targets damaged mitochondria (see Examples 7 and 9). According to yet another embodiment of the present disclosure, it was found that the dimeric peptide has an effect of restoring mitochondrial function and increasing cell viability by selectively binding to cardiolipin exposed in the inner membrane of damaged mitochondria, and that it can inhibit ROS production, promote ATP production, and restore membrane potential by protecting or restoring the cristae structure in the inner mitochondrial membrane (see Examples 10 to 12).

These effects of the dimeric peptide described herein are significantly enhanced as compared with the prior art. Specifically, as compared with SS-31 peptide, which is previously known to bind to cardiolipin, the dimeric peptide of the present disclosure was found to be significantly improved in all aspects, including cell penetrating ability, cardiolipin binding ability, ROS production inhibiting ability, ATP production promoting ability, and membrane potential restoring ability (see Comparative Examples 1 to 5).

The dimeric peptide may be a homodimer (including a dimer of two peptides having different sequences) in which two peptides, each of which comprises the monomer of Formula 1 consisting of 10 amino acids, are linked to each other, and may also be a heterodimer in which the monomer of Formula 1 is linked to another monomeric peptide having a different amino acid length and/or different sequence feature from Formula 1, as long as the characteristic α-helical structure of the present disclosure is maintained. For example, the other monomeric peptide having a different amino acid length may be a monomeric peptide whose length is increased by addition of one or more amino acids to either one or both ends of the monomer of Formula 1.

According to an embodiment of the present disclosure, the dimeric peptides may form an oligomer by self-assembly through interactions with each other (see Example 5). In a case where the dimeric peptides are oligomerized to a certain degree, the cell-penetrating ability may be further increased.

### Pharmaceutical composition

According to yet another aspect of the present disclosure, there is provided a pharmaceutical composition comprising, as an active ingredient, a mitochondria-specific monomeric peptide or dimeric peptide as described above and a method for treating a disease using the same.

In an embodiment, the peptide of the present disclosure targets damaged mitochondria and specifically binds to exposed cardiolipin, thereby exhibiting an effect of preserving/restoring cristae structure so that mitochondrial dysfunction is restored, and such restoration of mitochondrial dysfunction has been demonstrated by the ROS production inhibiting effect, ATP production promoting effect, mitochondrial membrane potential restoring effect, and the like of the peptide (see Examples 9 to 11).

According to still yet another aspect of the present disclosure based on these findings, there is provided a pharmaceutical composition for prevention or treatment of a disease accompanied by mitochondrial dysfunction, comprising, as an active ingredient, a peptide comprising the monomer represented by Formula 1, or a dimer thereof:

<Formula 1> X₁X₂X₃X₄X₅X₆X₇X₈X₉X₁₀

In Formula 1, X₁ to X₁₀ are as defined above. In addition, with respect to the peptide comprising the monomer represented by Formula 1, or a dimer thereof, all contents described in the "Mitochondria-specific peptide monomer" and "Mitochondria-specific peptide dimer" sections equally apply.

In an embodiment, the disease accompanied by mitochondrial dysfunction may be selected from the group consisting of, but not limited to, mitochondrial myopathy, MELAS (mitochondrial encephalopathy, lactic acidosis, and stroke-like episodes) syndrome, Charcot Marie Tooth disease (CMT), Leber hereditary optic neuropathy, Pearson syndrome, Leigh syndrome, Friedreich's ataxia, and Barth syndrome.

The term "a disease accompanied by mitochondrial dysfunction" refers to a disease that occurs as a result of mitochondrial dysfunction (disorder) due to mitochondrial damage and the like, which results from genetic or acquired mutations occurring in mitochondrial DNA (mtDNA) or nuclear genes encoding mitochondrial components, drugs, infections, or other environmental causes. It may be used interchangeably with "mitochondrial disease".

In another embodiment, the dimeric peptide of the present disclosure has a therapeutic effect on acute liver failure (Example 14). In particular, it is encouraging to note that the peptide showed an effect of inhibiting liver damage caused by acute liver failure regardless of the route of administration (that is, in both cases where it is intravenously administered and intraperitoneally administered), and it is encouraging that this therapeutic effect is exerted even when the peptide is administered at a low dose (1 mg/kg).

According to still yet another aspect of the present disclosure based on the above findings, there is provided a pharmaceutical composition for prevention or treatment of a liver disease, comprising, as an active ingredient, the peptide comprising the monomer represented by Formula 1, or a dimer thereof:

<Formula 1> X₁X₂X₃X₄X₅X₆X₇X₈X₉X₁₀

In Formula 1, X₁ to X₁₀ are as defined above.

With respect to the peptide included in the pharmaceutical compositions described herein, all contents described in the "Mitochondria-specific peptide monomer" and "Mitochondria-specific peptide dimer" sections equally apply.

In an embodiment, the liver disease may be selected from the group consisting of, but not limited to, acute liver failure, acute liver injury, liver cirrhosis, and hepatitis.

In another embodiment, the liver disease may be characterized by mitochondrial dysfunction.

In yet another embodiment, it has been found that the dimeric peptide of the present disclosure moves into cells and targets mitochondria even at concentrations below 100 nM, indicating that the dimeric peptide exhibits an effect of restoring mitochondrial dysfunction even at nanomolar concentrations, in contrast to conventional mitochondria-specific peptides that exhibit an effect of restoring mitochondrial dysfunction at micromolar concentrations (see Example 4, Comparative Examples 1 to 5). Accordingly, the pharmaceutical composition may comprise the peptide at a nanomolar concentration.

Meanwhile, to prepare the pharmaceutical compositions of the present disclosure, the peptide may be mixed with a pharmaceutically acceptable carrier and/or excipient. The pharmaceutical compositions may be prepared in the form of lyophilized preparations or aqueous solutions. For example, see Remington's Pharmaceutical Sciences and U.S. Pharmacopeia: National Formulary, Mack Publishing Company, Easton, PA (1984).

The acceptable carriers and/or excipients (including stabilizers) are non-toxic to subjects at the dosages and concentrations used and include, but not limited to, buffers (for example, phosphate, citrate or other organic acids); antioxidants (for example, ascorbic acid or methionine); preservatives (for example, octadecyldimethylbenzyl ammonium chloride; hexamethonium chloride; benzalkonium chloride, benzethonium chloride; phenol, butyl, or benzyl alcohol; alkyl parabens such as methyl or propyl paraben; catechol; resorcinol; cyclohexanol; 3-pentanol; and m-cresol); low molecular weight (about 10 or fewer residues) polypeptides; proteins (for example, serum albumin, gelatin, or immunoglobulin); hydrophilic polymers (for example, polyvinylpyrrolidone); amino acids (for example, glycine, glutamine, asparagine, histidine, arginine, or lysine); monosaccharides, disaccharides, and other carbohydrates such as glucose, mannose, or dextrin; chelating agents (for example, EDTA); sugars (for example, sucrose, mannitol, trehalose, or sorbitol); salt forming counter ions (for example, sodium); metal complexes (for example, Zn-protein complexes); and/or nonionic surfactants (for example, TWEEN^{®}, PLURONICS^{®}, or polyethylene glycol (PEG)).

The pharmaceutical composition of the present disclosure may be formulated into a suitable form known in the art depending on the route of administration.

As used herein, the term "prophylactically or therapeutically effective amount" or "effective amount" refers to an amount of an active ingredient in a composition, which is effective in preventing or treating a disease accompanied by mitochondrial dysfunction or other diseases, the amount being sufficient to prevent or treat the disease at a reasonable benefit/risk ratio applicable to any medical treatment without causing any adverse effects. A level of the effective dose may be determined depending on factors including the patient's health status, type and severity of disease, activity of the drug, sensitivity to the drug, administration method, administration time, administration route and excretion rate, duration of treatment, and drugs used in combination or concurrently therewith, and other factors well known in the medical field. Here, taking all of the above factors into consideration, it is important to administer a minimum amount that allows the maximum effect to be obtained with minimal or no adverse effects, and such an amount may be easily determined by those skilled in the art.

Specifically, the effective amount of the active ingredient in the pharmaceutical composition of the present disclosure may vary depending on age, gender, body weight of the subject (patient), type and severity of disease, form of drug, route and period of administration, and the like, and may be selected appropriately by a person skilled in the art. In general, the effective amount may be a daily dose of about 0.001 to 100 mg/kg, specifically 0.01 to 10 mg/kg, and more specifically 0.1 to 10 mg/kg; however, the scope of the present disclosure is not limited thereto. Administration may be done once a day or in divided doses several times a day, and may also be done over a long period of time by implantation of an infusion pump or the like.

The composition according to the present disclosure may be administered to a subject through various routes. Any mode of administration may be contemplated. The administration may be, for example, parenteral administration, which means administration via injection (for example, bolus injection) or infusion. The parenteral administration includes subcutaneous injection, intravenous injection, intramuscular injection, intraarterial injection, intraperitoneal injection, intracerebral injection, intrathecal injection, or intracerebroventricular injection. The composition according to the present disclosure is preferably administered by subcutaneous injection, intravenous injection, or intraperitoneal injection.

### Treatment method

According to still yet another aspect of the present disclosure, there is provided a method for preventing or treating a disease accompanied by mitochondrial dysfunction, comprising a step of administering a therapeutically effective amount of the pharmaceutical composition to a subject in need thereof. There is also provided a method for preventing or treating a liver disease, comprising a step of administering a therapeutically effective amount of the pharmaceutical composition to a subject in need thereof.

According to still yet another aspect of the present disclosure, there is provided a method for restoring mitochondrial function, comprising a step of administering, to a subject in need of restoration of mitochondrial function, a peptide comprising the monomer represented by Formula 1, or a dimer thereof:

<Formula 1> X₁X₂X₃X₄X₅X₆X₇X₈X₉X₁₀

In Formula 1, X₁, X₄, X₅, and X₈ are each independently a hydrophobic amino acid;

X₃, X₆, X₇, and X₁₀ are each independently a hydrophilic amino acid; X₂ and X₉ are each independently an amino acid that forms a bond so that two monomers can be linked to each other at one or more positions selected from X₂ and X₉ to form a dimeric peptide; and X₁ is N-terminus and X₁₀ is C-terminus.

In a case when CL in the mitochondria is oxidized or becomes structurally abnormal due to various factors, curvature of the cristae in CL disappears so that the various proteins responsible for functions of the electron transport system become physically distant from each other and gradually lose their function. The peptide or the dimer thereof described above can normalize mitochondrial function by binding to CL, restoring curvature of the cristae, and allowing a complex of proteins in the electron transport system to resume their normal function.

For specific description of "therapeutically effective amount", "subject", "disease accompanied by mitochondrial dysfunction", "liver disease", "peptide", and "dimeric peptide" of any of the above compositions, reference is made to the entirety of the contents described in the "Mitochondria-specific peptide monomer", "Mitochondria-specific peptide dimer" and "Pharmaceutical composition" sections.

Hereinafter, the present disclosure will be described in more detail by way of the following examples. However, the following examples are only for illustrating the present disclosure, and the scope of the present disclosure is not limited thereto.

### Examples

### I. Design, synthesis, and selection of mitochondria-specific peptides

### Example 1. Design of peptide library targeting mitochondria

The conventional mitochondria-specific SS-peptides have a very short amino acid sequence with a secondary structure in the form of a beta-sheet that provides amphipathic properties. However, since such SS-peptides have a relatively short distance between the hydrophilic and hydrophobic moieties (approximately 7 Å; FIGS. 1 and 2), once a charge interaction occurs between the phosphate group of a phospholipid such as cardiolipin and the hydrophilic moiety of the peptide, creating a hydrophobic interaction with the lipid moiety of the phospholipid is not sufficiently achieved. In addition, SS-31, which is a representative SS-peptide, has only two charged sites that participate in a 1:1 interaction with cardiolipin.

The present inventors have designed α-helical peptides consisting of 10 amino acids to overcome disadvantages of SS-peptides having a short-length beta-sheet structure which are associated with cardiolipin binding. In particular, the amphipathic α-helical structure of the peptide according to the present disclosure has a longer distance (approximately 11.3 Å) between the hydrophilic and hydrophobic amino acid side chains, which allows for much stronger hydrophobic interactions with the fatty acid side chains of cardiolipin (FIG. 4A). In addition, the α-helical peptide comprises at least four positively charged sites in its helical structure, which allows for binding to multiple cardiolipin molecules.

Meanwhile, in order to induce stronger hydrophobic interactions of the peptide with the fatty acid side chain in the target cardiolipin, the reference amino acid leucine (L) in the hydrophobic moiety of the peptide (amino acids at positions 1, 4, 5, and 8 in the peptide schematically shown in FIG. 4B) was substituted with several other types of hydrophobic amino acids to evaluate its mitochondria-targeting ability.

### Example 2. Synthesis of monomeric peptides

### Example 2.1. Synthesis of CMP3001m to CMP3015m

Monomeric peptides having the amino acid sequence of Formula 2 were synthesized by a solid-phase peptide synthesis as described in Experimental Example 1.1.

<Formula 2> X₁-C-R-X₄-X₅-R-R-X₈-C-R

(where C is cysteine, R is arginine, and X₁, X₄, X₅, and X₈ are each independently leucine (L) or cyclohexylalanine (Cha, X)).

The monomeric peptides were prepared in two types, that is, acetyl-capped type and fluorescently-labeled type. The acetyl-capped monomeric peptides were named CMP3001m to CMP3015m, respectively. Their amino acid sequences, calculated masses, and observed masses are shown in Table 2. The monomeric peptides labeled with TAMRA at N-terminus were also synthesized in the same way as the acetyl-capped monomeric peptides and were named FL-CMP3001m to CMP3015m, respectively. Their amino acid sequences and mass spectrometry results are shown in Table 3.

**[Table 2]**

| **Peptide** | **Sequence (X = Cha)** | **Mass (calcd.) [M+H]⁺** | **Mass (obsd.) [M+H]⁺** |
|---|---|---|---|
| CMP3001m | AcNH-XCRLLRRLCR-C(O)NH₂ | 1382.83 | 1386.18 |
| CMP3002m | AcNH-LCRXLRRLCR-C(O)NH₂ | 1382.83 | 1382.83 |
| CMP3003m | AcNH-LCRLXRRLCR-C(O)NH₂ | 1382.83 | 1382.39 |
| CMP3004m | AcNH-LCRLLRRXCR-C(O)NH₂ | 1382.83 | 1383.14 |
| CMP3005m | AcNH-XCRXLRRLCR-C(O)NH₂ | 1422.86 | 1422.86 |
| CMP3006m | AcNH-XCRLXRRLCR-C(O)NH₂ | 1422.86 | 1422.97 |
| CMP3007m | AcNH-XCRLLRRXCR-C(O)NH₂ | 1422.86 | 1422.73 |
| CMP3008m | AcNH-LCRXXRRLCR-C(O)NH₂ | 1422.86 | 1428.52 |
| CMP3009m | AcNH-LCRXLRRXCR-C(O)NH₂ | 1422.86 | 1429.13 |
| CMP3010m | AcNH-LCRLXRRXCR-C(O)NH₂ | 1422.86 | 1423.68 |
| CMP3011m | AcNH-XCRXXRRLCR-C(O)NH₂ | 1462.89 | 1470.52 |
| CMP3012m | AcNH-XCRXLRRXCR-C(O)NH₂ | 1462.89 | 1469.95 |
| CMP3013m | AcNH-XCRLXRRXCR-C(O)NH₂ | 1462.89 | 1464.63 |
| CMP3014m | AcNH-LCRXXRRXCR-C(O)NH₂ | 1462.89 | 1463.91 |
| CMP3015m | AcNH-XCRXXRPXCR-C(O)NH₂ | 1502.92 | 1503.14 |

**[Table 3]**

| **Peptide** | **Sequence (X = Cha)** | **Mass (calcd.) [M+H]⁺** | **Mass (obsd.) [M+H]⁺** |
|---|---|---|---|
| FL-CMP3001m | TAMRA-NH-XCRLLRRLCR-C(O)NH₂ | 1752.96 | 1755.91 |
| FL-CMP3002m | TAMRA-NH-LCRXLRRLCR-C(O)NH₂ | 1752.96 | 1755.28 |
| FL-CMP3003m | TAMRA-NH-LCRLXRRLCR-C(O)NH₂ | 1752.96 | 1755.23 |
| FL-CMP3004m | TAMRA-NH-LCRLLRRXCR-C(O)NH₂ | 1752.96 | 1753.60 |
| FL-CMP3005m | TAMRA-NH-XCRXLRRLCR-C(O)NH₂ | 1792.99 | 1793.37 |
| FL-CMP3006m | TAMRA-NH-XCRLXRRLCR-C(O)NH₂ | 1792.99 | 1793.51 |
| FL-CMP3007m | TAMRA-NH-XCRLLRRXCR-C(O)NH₂ | 1792.99 | 1793.55 |
| FL-CMP3008m | TAMRA-NH-LCRXXRRLCR-C(O)NH₂ | 1792.99 | 1800.13 |
| FL-CMP3009m | TAMRA-NH-LCRXLRRXCR-C(O)NH₂ | 1792.99 | 1803.25 |
| FL-CMP3010m | TAMRA-NH-LCRLXRRXCR-C(O)NH₂ | 1792.99 | 1793.81 |
| FL-CMP3011m | TAMRA-NH-XCRXXRRLCR-C(O)NH₂ | 1833.02 | 1835.48 |
| FL-CMP3012m | TAMPA-NH-XCRXLRRXCR-C(O)NH₂ | 1833.02 | 1840.67 |
| FL-CMP3013m | TAMRA-NH-XCRLXRRXCR-C(O)NH₂ | 1833.02 | 1835.03 |
| FL-CMP3014m | TAMRA-NH-LCRXXRRXCR-C(O)NH₂ | 1833.02 | 1834 93 |
| FL-CMP3015m | TAMRA-NH-XCRXXRRXCR-C(O)NH₂ | 1873.05 | 1873.14 |

### Example 2.2. Synthesis and selection of deconvolution peptide library

### Example 2.2.1. Synthesis of sub-library of 16 monomeric peptides

To evaluate mitochondria-targeting ability in a case where leucine (L), the reference amino acid for the hydrophobic moiety, is substituted with several other hydrophobic amino acids, synthesis of a library of deconvolution peptides (16 sub-libraries) having the combination as shown in Table 4 was performed. In Table 4, Hdf refers to cyclohexylalanine (Cha), phenylalanine (F), leucine (L), and/or tyrosine (Y), which resulted in increased diversity of hydrophobic amino acids.

**[Table 4]**

| Sub-library | N - | Position 1 | Position 4 | Position 5 | Position 8 | -C |
|---|---|---|---|---|---|---|
| L1X | Ac- | **Cha** | **Hdf** | **Hdf** | **Hdf** | -amide |
| L1F | Ac- | **F** | **Hdf** | **Hdf** | **Hdf** | -amide |
| L1L | Ac- | **L** | **Hdf** | **Hdf** | **Hdf** | -amide |
| L1Y | Ac- | **Y** | **Hdf** | **Hdf** | **Hdf** | -amide |
| L4X | Ac- | **Hdf** | **Cha** | **Hdf** | **Hdf** | -amide |
| L4F | Ac- | **Hdf** | **F** | **Hdf** | **Hdf** | -amide |
| L4L | Ac- | **Hdf** | **L** | **Hdf** | **Hdf** | -amide |
| L4Y | Ac- | **Hdf** | **Y** | **Hdf** | **Hdf** | -amide |
| L5X | Ac- | **Hdf** | **Hdf** | **Cha** | **Hdf** | -amide |
| L5F | Ac- | **Hdf** | **Hdf** | **F** | **Hdf** | -amide |
| L5L | Ac- | **Hdf** | **Hdf** | **L** | **Hdf** | -amide |
| L5Y | Ac- | **Hdf** | **Hdf** | **Y** | **Hdf** | -amide |
| L8X | Ac- | **Hdf** | **Hdf** | **Hdf** | **Cha** | -amide |
| L8F | Ac- | **Hdf** | **Hdf** | **Hdf** | **F** | -amide |
| L8L | Ac- | **Hdf** | **Hdf** | **Hdf** | **L** | -amide |
| L8Y | Ac- | **Hdf** | **Hdf** | **Hdf** | **Y** | -amide |

The library of deconvolution peptides (16 sub-libraries) was synthesized using the Fmoc solid-phase peptide synthesis method as described in Experimental Example 1.1. Specifically, 0.51 mmole/g of Rink amide MBHA resin (Sigma, USA) was used as a solid phase resin, and 20% piperidine (Sigma) was used for deprotection. After deprotection, washing was performed three times with DMF, five times with DCM, and three times again with DMF, and coupling was performed using 6 equivalents each of Fmoc-protected amino acids, DIPEA, and PyBOP. The total volume of the reaction solution was set to 5 ml, and the initial reaction conditions were explored using 1.5 equivalents each of the four amino acids (Y, L, F, and Cha). After the reaction, washing was performed three times with DMF, three times with DCM, and again with DMF. The reaction was then allowed to occur for 2 hours using a cleavage cocktail (95% TFA, 2.5% TIS, and 2.5% DW), and precipitation was performed with cold ether. The solvent was blown off using nitrogen gas and then dissolved in DMSO to perform HPLC. The Phenomenex C18 column (3 µm, 4.6 × 150 mm) was used as a stationary phase on an HPLC instrument (Infinity 1260, Agilent, USA), with 0.1% (v/v) TFA water as buffer A and 0.1% (v/v) TFA ACN as buffer B in a mobile phase. The gradient conditions were changed in a linear gradient of 0% Buffer B at 0 minutes and then 100% Buffer B until 60 minutes. Wavelengths used of the UV detector were set to 209 and 220 nm.

To ensure that coupling of the four amino acids occurs in equal proportions, the equivalent of each amino acid was determined in such a way of decreasing an equivalent ratio of the amino acids that underwent more coupling and increasing an equivalent ratio of the amino acids that underwent less coupling while maintaining a total of 6 equivalents. Specifically, four amino acids were injected into HPLC in equal molar amounts to derive a molar extinction coefficient ratio, and then a molar ratio of the four amino acids contained in the reaction product was calculated using the area under the HPLC peak. The finally found molar ratio of Y, L, F, and Cha was 1.26, 1.33, 1.48, and 1.93 equivalents, respectively, and the coupling ratio under such a condition was observed to be Y:L:F:Cha = 1.06:0.97:0.99:0.99 (FIG. 5).

The purity of the 16 sub-libraries was checked using an HPLC instrument (Infinity 1260, Agilent). Specifically, the Phenomenex C18 column (3 µm, 4.6 × 150 mm) was used as a stationary phase of HPLC, with 0.1% (v/v) TFA water as buffer A and 0.1% (v/v) TFAACN as buffer B in a mobile phase. The gradient conditions were such that 5% buffer B was maintained for 0-5 minutes, 5-70% buffer B was changed in a linear gradient for 5-30 minutes, and then 70-100% buffer B was applied in a linear gradient for 30-40 minutes. Wavelengths used of the UV detector were set to 220 and 280 nm. The synthesized 16 sub-libraries were purified under HPLC conditions, and chromatograms for the respective sub-libraries were obtained.

Among the obtained chromatograms, a representative chromatogram for the L1X sub-library is shown in FIG. 6, and the HPLC chromatogram results for all 16 sub-libraries are presented in Table 5 as a range of continuous retention times (RTs) from the first peak to the last peak.

The amino acid sequences and mass spectrometry results of the monomeric peptides contained in the 16 sub-libraries are shown in Table 5.

**[Table 5]**

| **Sub-library** | **Sequence (X = Cha, Hdf = Cha, F, L or Y)** | **Range of retention time (min)** | **Mass (calcd.) [M+H]⁺** | **Mass (obsd.) [M+H]⁺** |
|---|---|---|---|---|
| L1X | AcNH-XCR(Hdf)(Hdf)RR(Hdf)CR-C(O)NH₂ | 17.2 - 27.8 | 1382.83 - 1502.92 | 1423.39 - 1490.15 |
| L1F | AcNH-FCR(Hdf)(Hdf)RR(Hdf)CR-C(O)NH₂ | 17.6 - 26.1 | 1376.78 - 1496.87 | 1416.48-1538.76 |
| L1L | AcNH-LCR(Hdf)(Hdf)RR(Hdf)CR-C(O)NH₂ | 16.1 - 25.9 | 1341.80 - 1462.89 | 1382.02 - 1450.26 |
| L1Y | AcNH-YCR(Hdf)(Hdf)RR(Hdf)CR-C(O)NH₂ | 15.0 - 22.1 | 1392.78 - 1512.87 | 1436.92 - 1508.94 |
| L4X | AcNH-(Hdf)CRX(Hdf)RR(Hdf)CR-C(O)NH₂ | 17.1 - 24.3 | 1382.83 - 1502.92 | 1424.25 - 1497.60 |
| L4F | AcNH-(Hdf)CRF(Hdf)RR(Hdf)CR-C(O)NH₂ | 17.2 - 23.9 | 1376.78 - 1496.87 | 1417.47 - 1491.04 |
| L4L | AcNH-(Hdf)CRL(Hdf)RR(Hdf)CR-C(O)NH₂ | 16.2 - 23.7 | 1341.80-1462.89 | 1383.53 - 1457.07 |
| L4Y | AcNH-(Hdf)CRY(Hdf)RR(Hdf)CR-C(O)NH₂ | 15.9 - 21.9 | 1392.78 - 1512.87 | 1433.44 - 1553.49 |
| L5X | AcNH-(Hdf)CR(Hdf)XRR(Hdf)CR-C(O)NH₂ | 17.2 - 26.5 | 1382.83 - 1502.92 | 1457.18 - 1546.49 |
| L5F | AcNH-(Hdf)CR(Hdf)FRR(Hdf)CR-C(O)NH₂ | 16.2 - 26.0 | 1376.78 - 1496.87 | 1417.35 - 1490.84 |
| L5L | AcNH-(Hdf)CR(Hdf)LRR(Hdf)CR-C(O)NH₂ | 15.2 - 26.0 | 1341.80-1462.89 | 1382.08 - 1456.47 |
| L5Y | AcNH-(Hdf)CR(Hdf)YRR(Hdf)CR-C(O)NH₂ | 14.9 - 22.6 | 1392.78 - 1512.87 | 1433.22 - 1507.51 |
| L8X | AcNH-(Hdf)CR(Hdf)(Hdf)RRXCR-C(O)NH₂ | 16.3 - 27.0 | 1382.83 - 1502.92 | 1384.44 - 1506.44 |
| L8F | AcNH-(Hdf)CR(Hdf)(Hdf)RRFCR-C(O)NH₂ | 15.8 - 25.9 | 1376.78 - 1496.87 | 1411.27 - 1500.47 |
| L8L | AcNH-(Hdf)CR(Hdf)(Hdf)RRLCR-C(O)NH₂ | 15.9 - 25.9 | 1341.80 - 1462.89 | 1343.15 - 1472.48 |
| L8Y | AcNH-(Hdf)CR(Hdf)(Hdf)RRYCR-C(O)NH₂ | 15.1 - 23.2 | 1392.78 - 1512.87 | 1393.60 - 1500.97 |

### Example 2.2.2. Analysis of ROS production inhibiting ability of monomeric peptide sub-libraries

Using the 16 peptide sub-libraries synthesized in Example 2.2.1., it was evaluated which types of hydrophobic amino acids located at positions 1, 4, 5, and 8 of the hydrophobic moiety had the best ROS (reactive oxygen species) production inhibiting ability.

Analysis of ROS levels in cells was performed according to the method described in Experimental Example 6. Specifically, HeLa cells (96-well plates, 2 × 10⁴ cells/well) were cultured for 1 day and then treated with 100 nM of the monomeric peptide sub-library for 3 hours. Peptide-treated cells were washed with PBS and stained with 25 µM DCFDA for 30 minutes. The cells were then treated with 0.5 mM H₂O₂ to induce oxidative stress. Production of ROS was read by fluorescence intensity using a microplate reader (TECAN) at 37°C for 30 minutes.

Each experiment was repeated 10 times and the averages of the measured values are illustrated in FIGS. 7A to 7D. The most preferred amino acid at each position of the hydrophobic moiety was determined by the ROS production inhibiting effect, and the results are shown in Table 6.

**[Table 6]**

| **Position 1** | **Position 4** | **Position 5** | **Position 8** |
|---|---|---|---|
| F | Cha or L | Cha | Cha or L |

In a case of the amino acid at position 4, either cyclohexylalanine (Cha) or leucine (L) was selected for the preferred amino acid, as the CMP3013 peptide has leucine (L) at this position.

### Example 2.3. Synthesis of CMP3029m to CMP3032m

Four monomeric peptides were synthesized by combining the hydrophobic amino acids selected in Example 2.2, and named CMP3029m to CMP3032m, respectively. The amino acid sequences and mass spectrometry results of these monomeric peptides are shown in Table 7. TAMRA-labeled monomeric peptides were also synthesized by the same method, and named FL-CMP3029m to FL-CMP3032m, respectively. The amino acid sequences and mass spectrometry results of these monomeric peptides are shown in Table 8.

**[Table 7]**

| **Peptide** | **Sequence (X = Cha)** | **Mass (calcd.) [M+H]⁺** | **Mass (obsd.) [M+H]⁺** |
|---|---|---|---|
| CMP3029m | AcNH-FCRLXRRXCR-C(O)NH₂ | 1456.84 | 1455.83 |
| CMP3030m | AcNH-FCRXXRRXCR-C(O)NH₂ | 1496.87 | 1496.75 |
| CMP3031m | AcNH-FCRLXRRLCR-C(O)NH₂ | 1416.81 | 1417.17 |
| CMP3032m | AcNH-FCRXXRRLCR-C(O)NH₂ | 1456.84 | 1457.45 |

**[Table 8]**

| **Peptide** | **Sequence (X = Cha)** | **Mass (calcd.) [M+H]⁺** | **Mass (obsd.) [M+H]⁺** |
|---|---|---|---|
| FL-CMP3029m | TAMRA-NH-FCRLXRRXCR-C(O)NH₂ | 1826.97 | 1827.62 |
| FL-CMP3030m | TAMRA-NH-FCRXXRRXCR-C(O)NH₂ | 1868.01 | 1867.39 |
| FL-CMP3031m | TAMRA-NH-FCRLXRRLCR-C(O)NH₂ | 1786.94 | 1788.10 |
| FL-CMP3032m | TAMRA-NH-FCRXXRRLCR-C(O)NH₂ | 1826.97 | 1828.63 |

### Example 3. Synthesis of dimeric bundle peptides

### Example 3.1. Synthesis of CMP3001 to CMP3015

To make the monomeric peptide synthesized in Example 2.1 into dimeric bundles, air oxidation was used to form a disulfide bond between the two monomeric peptides. First, the fluorescently-unlabeled monomeric peptides were dissolved in 0.1 M ammonium bicarbonate buffer at a concentration of 5-10 mg/ml, and dimerization was performed according to the method described in Experimental Example 1.2. The dimeric peptides purified by HPLC were named CMP3001 to CMP3015, respectively, and their masses were analyzed using MALDI-TOF. The amino acid sequence, purity analysis, and mass spectrometry results for these dimeric peptides are shown in Table 9 (the line between the two cysteines indicates a disulfide bond in Table 9, and the same applies to all of Tables 10 to 12). Synthesis of the fluorescently-labeled dimeric bundle peptide was achieved by mixing the fluorescently-unlabeled monomeric peptides with the TAMRA-labeled monomeric peptides in a 1:1 ratio and performing dimerization according to the method described in Experimental Example 1.2. The reaction products were purified by HPLC and their masses were checked by MALDI-TOF. The respective peptides were named FL-CMP3001 to FL-CMP3015. The amino acid sequences, purity analysis by HPLC, and mass spectrometry results for the fluorescently-labeled dimeric bundle peptides FL-CMP3001 to FL-CMP3015 are shown in Table 10. The purified FL-CMP3001 to FL-CMP3015 all exhibited >95% purity (Table 10).

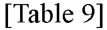

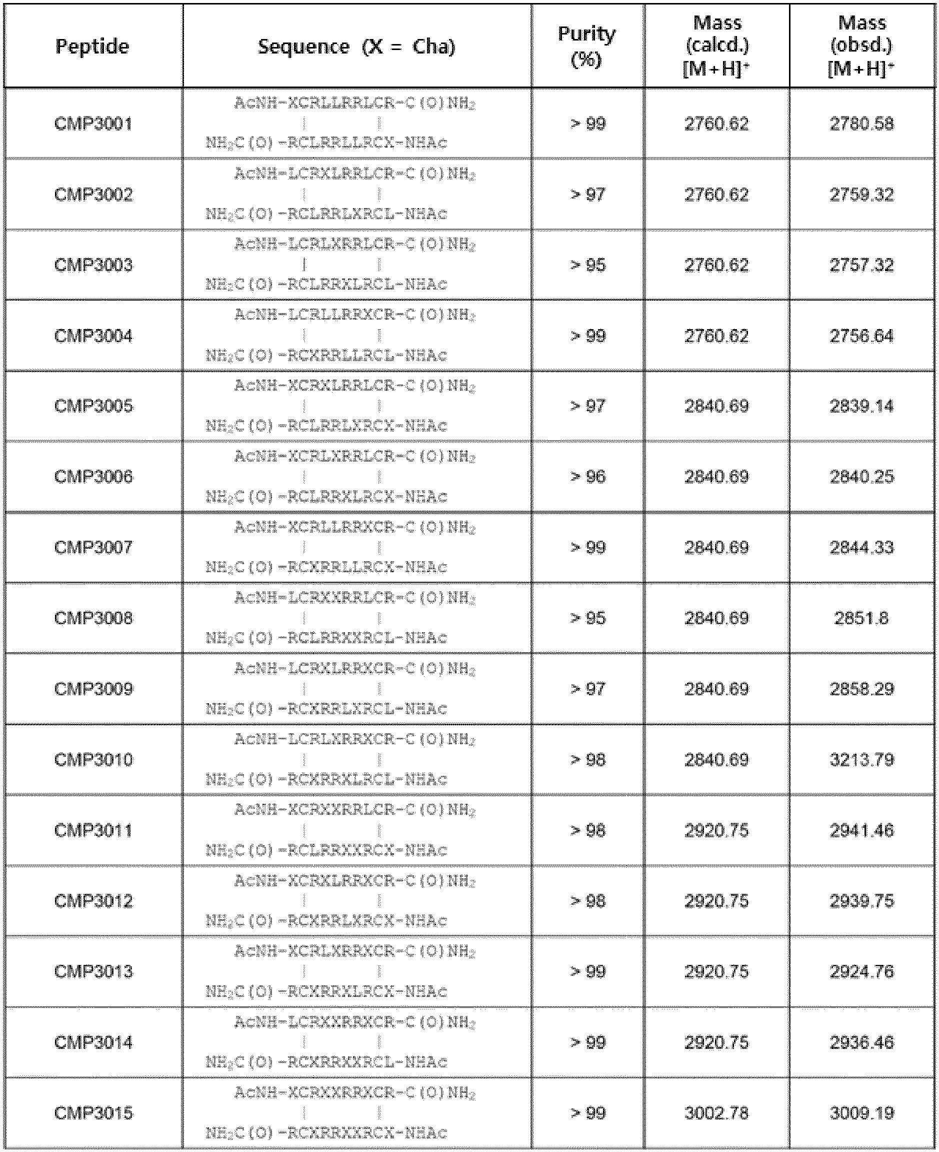

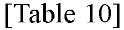

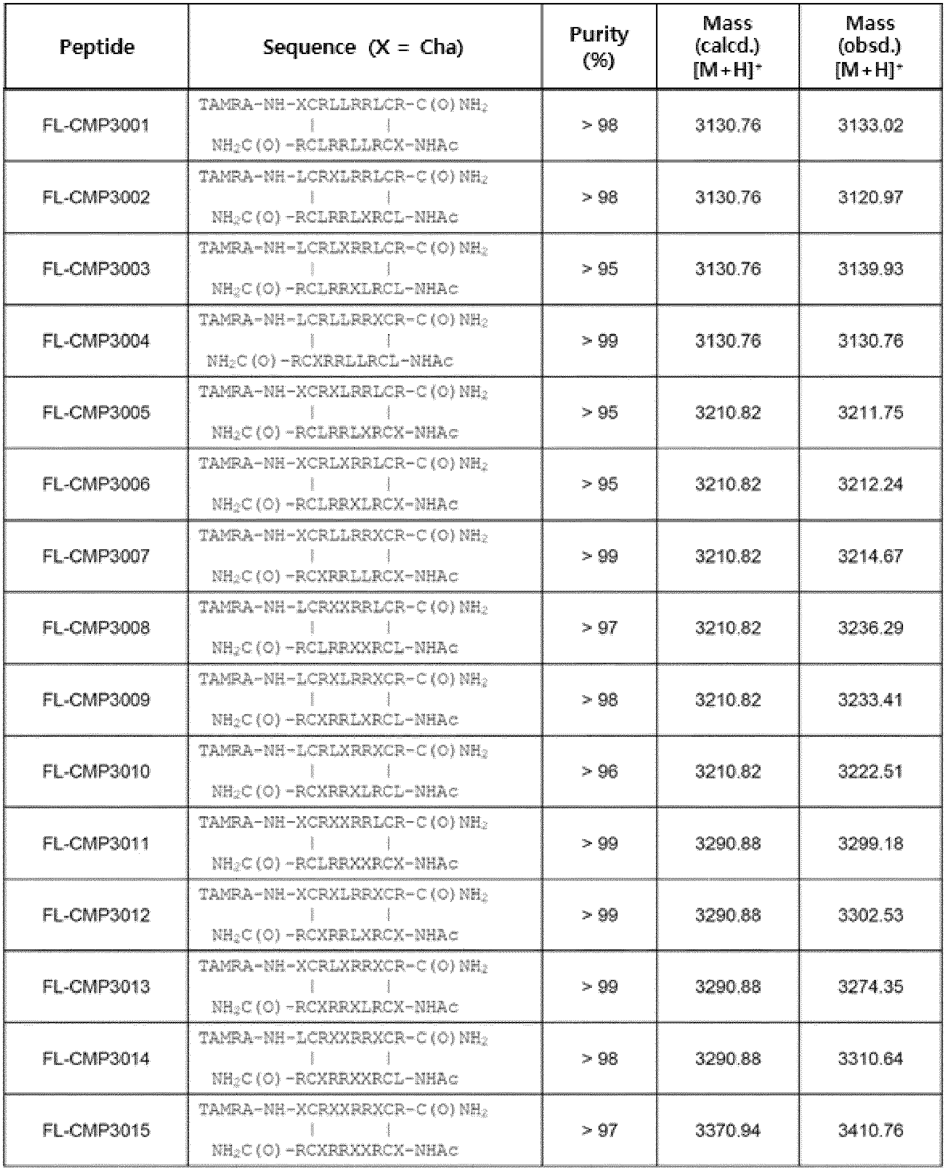

The yield of dimeric peptides varied depending on the position and number of cyclohexylalanine (Cha), and no dimeric peptide was particularly difficult to synthesize. As expected, the peptide comprising four cyclohexylalanine (Cha) residues was relatively poorly soluble in water. However, the peptides comprising three or fewer cyclohexylalanine (Cha) residues showed unexpectedly high solubility in water.

### Example 3.2. Synthesis of CMP3029 to CMP3032

The monomeric peptides CMP3029m to CMP3032m synthesized in Example 2.3 were used to synthesize dimeric bundle peptides. Dimerization was performed according to the method described in Experimental Example 1.2. using the same synthesis method as in Example 3.1. Then, two types of dimers, that is, fluorescently-unlabeled dimers and fluorescently-labeled dimers were synthesized, and were named CMP3029 to CMP3032 and FL-CMP3029 to FL-CMP3032, respectively. The amino acid sequences, purity analysis by HPLC, and mass spectrometry results for these dimeric peptides are shown in Tables 11 and 12, respectively.

**[Table 11]**

| **Peptide** | **Sequence (X** = **Cha)** | **Purity (%)** | **Mass (calcd.) [M+H]⁺** | **Mass (obsd.) [M+H]⁺** |
|---|---|---|---|---|
| CMP3029 |     AcNH-FCRLXRRXCR-C(O)NH2 ;      ; NH2C(O)-RCXRRXLRCF-NHAc | 99.9 | 2908.65 | 2907.93 |
| CMP3030 |     AcNH-FCRXXRRXCR-C(O)NH2 ;      ; NH2C(O)-RCXRRXXRCF-NHAc | 99.5 | 2988.72 | 2985.88 |
| CMP3031 |     AcNH-FCRLXRRLCR-C(O)NH2 ;      ; NH2C(O)-RCLRRXLRCF-NHAc | 99.9 | 2827.59 | 2830.78 |
| CMP3032 |      AcNH-FCRXXRRLCR-C(O)NH2 ;      ; NH2C(O)-RCLRRXXRCF-NHAc | 99.9 | 2907.65 | 2905.42 |

**[Table 12]**

| **Peptide** | **Sequence (X = Cha)** | **Purity (%)** | **Mass (calcd.) [M+H]⁺** | **Mass (obsd.) [M+H]⁺** |
|---|---|---|---|---|
| FL-CMP3029 | TAMRA-NH-FCRLXRRXCR-C(O)NH2 ;          ; NH2C(O)-RCXRRXLRCF-NHAc | 97.5 | 3280.80 | 3276.08 |
| FL-CMP3030 | TAMRA-NH-FCRXXRRXCR-C(O)NH2 ;       ; NH2C(O)-RCXRRXXRCF-NHAc | 99.4 | 3359.86 | 3358.64 |
| FL-CMP3031 | TAMRA-NH-FCRLXRRLCR-C(O)NH2 ;          ; NH2C(O)-RCLRRXLRCF-NHAc | 92.2 | 3197.72 | 3194.94 |
| FL-CMP3032 | TAMRA-NH-FCRXXRRLCR-C(O)NH2 ;          ; NH2C(O)-RCLRRXXRCF-NHAc | 93.1 | 3277.79 | 3277.18 |

### Example 3.3. Identification of sequence direction of each monomer in dimeric peptide

To determine whether two monomers are linked to each other in a parallel or antiparallel direction in the dimeric peptide formed according to the method of dimerizing monomeric peptides by air oxidation described in Experimental Example 1.2, experiments were performed in which different protecting groups, that is, Trt (triphenyl methyl) and Acm (acetamidomethyl) are used for the two cysteine (C) residues contained in each monomeric peptide. Because the Trt protecting group dissociates from thiol under acidic conditions, the cysteine residue remains in a form of thiol in a case of being treated with a cleavage cocktail containing TFA. On the other hand, because the Acm protecting group dissociates from thiol only under strong acidic conditions, the protecting group remains attached to the thiol even in a case of being treated with a cleavage cocktail containing TFA.

First, two monomeric peptides containing one each cysteine-Trt and cysteine-Acm at two different cysteine positions (X₂, X₉) of CMP3013m were prepared by solid-phase peptide synthesis. Then, a disulfide bond was formed between the two different monomeric peptides under air oxidation conditions, and then the dimeric peptide with only one disulfide bond was purified by HPLC. The dimeric peptide with only one disulfide bond was then dissolved in tertiary distilled water containing 10% acetic acid, and reaction was allowed to occur for 50 minutes under strong oxidizing conditions created by dropwise addition of a methanol solution containing iodine so that the Acm protecting group was removed and a new disulfide bond was to be formed. In addition, air oxidation reaction was used to synthesize the CMP3013 dimeric peptide, and the retention time for this peptide was measured on HPLC together with the retention time for the CMP3013 antiparallel dimeric peptide synthesized using the protecting group (FIG. 8A). Purification of the peptides was performed by HPLC with the Zorbax C18 column (3.5 µm, 4.6 × 150 mm) as a stationary phase. Buffer A [tertiary distilled water containing 0.1% (v/v) TFA] and buffer B [ACN containing 0.1% (v/v) TFA] were used in a binary solvent system as a mobile phase. The gradient method was as follows: a linear gradient of 5% buffer B for 5 minutes followed by 5-70% buffer B for 5 to 30 minutes; 70-100% buffer B, 30-40 minutes. The flow rate of the mobile phase was set to 1.0 ml/min.

The HPLC chromatograms of the dimeric peptide formed by air oxidation (CMP3013 airoxidation) and the antiparallel dimeric peptide (CMP3013 antiparallel) are shown in FIG. 8A, and the chromatograms for the individual reaction products (antiparallel dimers) are shown in FIGS. 8B and 8C, respectively. From the results shown, it was identified that the CMP3013 dimeric bundle peptide formed by air oxidation has an anti-parallel structure.

### II. Characterization and efficacy analysis of mitochondria-specific peptides

### Example 4. Evaluation of cell-penetrating ability

Cell-penetrating ability was evaluated using the fluorescently-labeled versions of the dimeric bundle peptides synthesized in Example 3, and measured as EC₅₀ values according to the method described in Experimental Example 3. Specifically, HeLa cells were treated with various concentrations of the TAMRA-labeled dimeric bundle peptide for 3 hours, and EC₅₀ values were measured. EC₅₀ is a concentration of the corresponding peptide at which half of the cells exhibit fluorescence by peptide penetration.

As a result of analysis of fluorescence intensity, it was identified that all dimeric bundle peptides moved into the cells at concentrations below 100 nM (see Table 13 and FIGS. 9A and 9B). A lower EC₅₀ concentration may be considered to have higher cell penetrating ability. As can be seen in Table 13, even the peptide, which was evaluated as having the poorest cell-penetrating ability, had an EC₅₀ concentration of only 100 nanomolar.

In particular, FL-CMP3013 contains three cyclohexylalanine (Cha) residues instead of leucine (L) residues at positions 1, 5, and 8, and exhibited relatively higher cell-penetrating ability than the other peptides due to its antiparallel structure (EC₅₀ = 35 nM; Table 13 and FIG. 9A). FL-CMP3029 and FL-CMP3030 also exhibited excellent cell-penetrating ability, comparable to FL-CMP3013 (EC₅₀ = 34-35 nM; Table 13 and FIG. 9B).

**[Table 13]**

| **Peptide** | **EC₅₀ (nM)** | **Peptide** | **EC₅₀ (nM)** |
|---|---|---|---|
| FL-CMP3001 | 48.8 | FL-CMP3011 | 52.8 |
| FL-CMP3002 | 55.6 | FL-CMP3012 | 44.6 |
| FL-CMP3003 | 46.4 | FL-CMP3013 | 35.3 |
| FL-CMP3004 | 55..1 | FL-CMP3014 | 81.2 |
| FL-CMP3005 | 47.1 | FL-CMP3015 | 44.1 |
| FL-CMP3006 | 90.7 | FL-CMP3029 | 35 |
| FL-CMP3007 | 102.3 | FL-CMP3030 | 34 |
| FL-CMP3008 | 69.4 | FL-CMP3031 | 62 |
| FL-CMP3009 | 65.5 | FL-CMP3032 | 56 |
| FL-CMP3010 | 64.3 | - | |

### Example 5. Evaluation of oligomerization tendency

To determine whether the dimeric bundle peptides according to the present disclosure exhibit self-association properties due to interactions, the retention time (RT) on the HPLC column for each peptide was observed depending on changes in temperature and its tendency of self-association was determined through changes in the above values. Specifically, 10 µl of each peptide solution at 0.2 mM was injected into HPLC, and the time at which the peptide was detected (RT) was measured at 40°C and 5°C, respectively. Here, the stationary phase and mobile phase conditions of the HPLC were set using well-known methods. In general, the better the oligomer is formed, the detection time becomes longer at 40°C, which is a relatively higher temperature, thereby resulting in larger ΔΔRT that is a difference from the value measured at 5°C. The ΔΔRT values measured for CMP3001 to CMP3015 are shown in Table 14.

**[Table 14]**

| **Peptide** | **Position of Cha in each peptide** | **Oligomerization tendency (RT at 40-5°C) ΔΔRT(min)** |
|---|---|---|
| CMP3001 | 1 | 1.849 |
| CMP3002 | 4 | 1.864 |
| CMP3003 | 5 | 1.596 |
| CMP3004 | 8 | 1.771 |
| CMP3005 | 1, 4 | 2.362 |
| CMP3006 | 1, 5 | 2.140 |
| CMP3007 | 1, 8 | 2582 |
| CMP3008 | 4, 5 | 2.178 |
| CMP3009 | 4, 8 | 1.938 |
| CMP3010 | 5, 8 | 2.098 |
| CMP3011 | 1, 4, 5 | 3.402 |
| CMP3012 | 1, 4, 8 | 4.365 |
| CMP3013 | 1, 5, 8 | 2.683 |
| CMP3014 | 4, 5, 8 | 2.651 |
| CMP3015 | 1, 4, 5, 8 | 3.325 |

As expected, the results showed that oligomerization between the peptides tended to increase as the number of cyclohexylalanine (Cha) in the peptide increased. However, this tendency did not appear position-specific for cyclohexylalanine (Cha) in the peptides that contain one or two cyclohexylalanine (Cha) residues based on the monomer. That is, the peptides containing two or fewer cyclohexylalanine (Cha) residues exhibited similar levels of oligomerization tendency regardless of whether the amino acid residue was present at position 1, 4, 5, or 8. In contrast, the peptides containing three or more cyclohexylalanine (Cha) residues showed differences in oligomerization tendency depending on whether the amino acid residue was present at position 1, 4, 5, or 8. Meanwhile, it was identified that the peptide containing four cyclohexylalanine (Cha) residues was relatively well oligomerized.

As a result of identifying the relationship between oligomerization tendency and cell-penetrating ability, for dimeric bundle peptides that do not contain cyclohexylalanine (Cha), oligomerization was positively correlated with cell-penetrating ability (data not shown); however, for dimeric bundle peptides that contain cyclohexylalanine (Cha), there was no significant correlation between the degree of oligomerization between the dimeric bundle peptides and their cell-penetrating ability (FIG. 10). The solubility of a peptide in water may vary depending on the proportion of cyclohexylalanine (Cha) in the peptide, and excessive oligomerization of the peptide may lead to a decrease in solubility. This means that oligomerization generally increases cell-penetrating ability, whereas oligomerization beyond a certain threshold may decrease cell-penetrating ability.

### Example 6. Comparative analysis of cytotoxicity

The relative cytotoxicity of the dimeric bundle peptides according to the present disclosure was compared and analyzed. For this purpose, 1 × 10⁵ HeLa cells were treated with 100 nM of each peptide for 3 hours, washed three times with PBS, and then resuspended in PBS. After 30 minutes, five random photographs of each sample were taken under a light microscope to measure the number of cells normally attached to the cell culture dish.

The measurement results are shown in FIG. 11. In general, the peptides containing two cyclohexylalanine (Cha), which is a non-natural amino acid, residues on a monomer basis exhibited higher cytotoxicity than the peptides containing only one cyclohexylalanine (Cha) residue. On the other hand, for the peptides containing three cyclohexylalanine (Cha) residues (CMP3011 to CMP3014), there was a position-specific difference in toxicity for this amino acid. That is, CMP3011 had lower cell-penetrating ability as compared with CMP3012 and CMP3013, and showed greater cytotoxicity than both peptides. Although the bundle peptides of CMP3011 to CMP3014 were isomers that differ only in the position of cyclohexylalanine (Cha), both the one with the relatively highest cytotoxicity (CMP3011) and the one with the relatively lowest cytotoxicity (CMP3013) were present among them. Since these differences in cytotoxicity did not correlate with cell-penetrating ability, the relatively less toxic peptides were selected and used preferentially for other tests.

Meanwhile, the results obtained by observing cytotoxicity of CMP3013 at different concentrations are shown in FIG. 12. The cytotoxicity was measured by WST-1 assay. Specifically, 1 × 10⁴ HeLa cells were seeded into 96-well plates. After 24 hours, the cells were treated with different concentrations of CMP3013 peptide indicated in FIG. 12 and incubated for one day. At the end of the incubation, the culture medium was replaced with new culture medium, treatment with EZ-Cytox (DoGEN, South Korea) at 10 µL was performed per well, and incubation was performed for 30 minutes. Then, the absorbance was measured at 450 nm. As a result, it was identified that the cytotoxicity of CMP3013 was only observed at micromolar or higher levels, and the concentration, at which 50% of the cultured cells were killed, reached 10 micromolar (FIG. 12).

### Example 7. Evaluation of mitochondria-targeting ability

To determine at what rate the dimeric bundle peptides according to the present disclosure target mitochondria, mitochondrial fractionation was performed according to the method described in Experimental Example 4. Specifically, HeLa cells were treated with each of FL-CMP3001 to FL-CMP3015 at its EC₅₀ concentration (see Table 13) for 3 hours. Then, the cells were harvested and fractionation was performed.

The results showed that 53-80% of the dimeric bundle peptides target mitochondria (FIG. 13). Among them, CMP3013 was found to be present at the highest percentage in the mitochondrial fraction, followed by CMP3010 and CMP3004. Furthermore, almost all types of peptides were fractionated significantly more in mitochondria than in cytosol.

### Example 8. Identification of the ROS production inhibiting ability in cells

Typically, treatment with hydrogen peroxide (H₂O₂) increases production of ROS, leading to reduced mitochondrial function. Therefore, in order to compare the ability of FL-CMP3001 to FL-CMP3015 peptides to restore function of damaged mitochondria, the ability of each peptide to inhibit reactive oxygen species (ROS) production was evaluated. ROS levels were analyzed at the cellular level according to the method described in Experimental Example 6. Specifically, HeLa cells were treated with each peptide at its EC₅₀ concentration for 3 hours, and then the cells were stained with DCFDA. Subsequently, after treatment with 0.5 mM of H₂O₂, the fluorescence was detected and imaged by confocal microscopy. The fluorescence intensity of the cells was calculated with an image analysis program (ZEN blue).

As a result, although there were differences in ROS production inhibiting ability depending on the type of peptide treated, an ROS production inhibiting effect was generally observed for all types of peptides (FIG. 14A). In particular, the greatest inhibition of ROS production was observed in the cells treated with FL-CMP3013 peptide at its EC₅₀ concentration (35 nM). It was identified that the green fluorescence increased by H₂O₂ was significantly reduced by FL-CMP3013 (FIGS. 14A and 14B).

On the other hand, even referring to the analysis results of ROS production inhibiting ability for 16 monomeric peptide sub-libraries (each sub-library containing 64 types of peptides) containing a total of 256 types of peptides synthesized in Example 2.2.1, an ROS production inhibiting effect was also observed in all sub-libraries. In particular, the peptides, in which the amino acid at position 1 is phenylalanine (F), the amino acids at positions 4 and 8 are cyclohexylalanine (Cha) or leucine (L), and the amino acid at position 5 is cyclohexylalanine (Cha), showed the best ROS production inhibiting ability (see Example 2.2.2 and FIGS. 7A to 7D).

### III. Effects of mitochondria-specific peptides on restoration of mitochondrial dysfunction

### Example 9. Identification of effect of preferential binding to damaged mitochondria

Among the various types of peptides according to the present disclosure, CMP3013, CMP3029, CMP3030, CMP3031, and CMP3032 were selected as therapeutic candidates by comprehensively considering the cell-penetrating ability, cytotoxicity, ROS production inhibiting ability, and the like of each peptide evaluated through the above examples, and additional analysis was performed on these peptides.

First, HeLa cells, which were treated with 100 nM of FL-CMP3013 for 3 hours, were stained with MitoTracker^{™} and fluorescence analysis was performed, according to the method described in Experimental Example 5. To damage mitochondria, treatment with carbonyl cyanide m-chlorophenyl hydrazone (CCCP) at a concentration of 20 µM was performed for 30 minutes. As a result of the analysis, it was identified that FL-CMP3013 penetrated into mitochondria at a high proportion; however, the correlation factor between FL-CMP3013 and mitotracker was unexpectedly low in the extended form of mitochondria (FIG. 15A).

Next, in order to study the above phenomenon more specifically, treatment with FL-CMP3013 and CCCP was performed according to the method described in Experimental Example 5 and stained with nonyl acridine orange (NAO) to label cardiolipin for generation of ultra-high resolution images. The results surprisingly showed that FL-CMP3013 co-localized more with donut-shaped or bulge-shaped mitochondria whose damage was induced by CCCP treatment than with the expanded form of mitochondria without CCCP treatment (FIG. 15B). From the viewpoint that round-shaped mitochondria are known to be a kinetic phenomenon that occurs when they are dysfunctional and unable to function normally, the observed properties of FL-CMP3013 suggest that the peptide can specifically bind to dysfunctional or abnormal mitochondria and furthermore that FL-CMP3013 interacts with cardiolipin present in the inner mitochondrial membrane.

In order to cross-validate these interesting results, the fluorescently-labeled fission initiation protein Drp1 (Dynamin-related protein 1) was expressed, and it was identified whether this protein co-localized with CMP3013. Expression of Drp1-GFP was performed according to the method described in Experimental Example 10. As a result, FL-CMP3013 was found to co-localize perfectly with Drp1, consistent with the above observed results (FIG. 15C). Experiments were also performed on CMP3029, CMP3030, CMP3031, and CMP3032 using the same method as CMP3013, and these peptides were also found to overlap with Drp1 (FIG. 15D). Notably, among the above peptides, CMP3029 overlapped the most with Drp1 (FIG. 15D). Drp1 is known to be the first protein recruited from the cytosol for fission when mitochondria are damaged. Thus, the above experimental results show that the CMP3013 peptide preferentially moves (or penetrates) into damaged mitochondria that require fission.

In addition, to observe whether CMP3013 moves into damaged mitochondria, HeLa cells were treated with FL-CMP3013 (100 nM, 3 hours) and mitotracker, and mitochondrial damage was induced by CCCP while imaging the cells with confocal microscopy. The resulting time-lapse images showed that the correlation (indicated by the r value) between CMP3013 and mitotracker increased over time in the HeLa cells whose mitochondria were damaged by CCCP, which identifies that CMP3013 moves into damaged mitochondria (FIG. 15E).

The above experimental results show that the CMP3013, CMP3029, CMP3030, CMP3031, and CMP3032 peptides preferentially target damaged mitochondria and, in particular, bind to cardiolipin in the inner mitochondrial membrane.

### Example 10. Identification of effect of preventing cell death induced by CCCP

It was identified in Example 9 that the peptides according to the present disclosure preferentially target damaged mitochondria. Thus, HeLa cells, in which damage was induced by CCCP, were treated with FL-CMP3013 and cell survival was observed for 4 hours to determine whether the peptides disclosed herein directly affect viability of the cells. As a result, the cells treated with CCCP and FL-CMP3013 survived even after 4 hours, whereas the cells treated with CCCP alone did not survive (FIG. 16). In other words, the cells treated with FL-CMP3013 exhibited significantly higher cell survival than the cells treated with CCCP.

Taking the results of this experiment together with the results of FIGS. 15A to 15E of Example 9, FL-CMP3013 specifically binds to cardiolipin in the inner mitochondrial membrane. In this regard, since it is known that in normal mitochondria, cardiolipin is protected by many proteins of the cristae organizing system or functional proteins of the inner membrane, FL-CMP3013 is thought to preferentially bind to cardiolipin exposed by damage to mitochondria such as inner mitochondrial membrane. In other words, CMP3013 would not bind to cardiolipin if the surface of the inner mitochondrial membrane was completely protected by the proteins. Therefore, it can be seen that the peptide according to the present disclosure exhibits an effect of restoring mitochondrial function and further increasing cell survival by selectively binding to cardiolipin exposed in the damaged inner mitochondrial membrane.

### Example 11. Mitochondrial function restoring ability and cristae protecting ability

### Example 11.1. Identification of ROS production inhibiting ability, ATP production promoting ability, and membrane potential restoring ability

To determine mitochondrial function restoring ability, an effect of the α-helical peptide of the present disclosure on mitochondrial ROS, ATP level, and membrane potential was tested using cell-based assays.

First, in the cells pretreated with FL-CMP3013 for 3 hours at different concentrations, different damage conditions were created using H₂O₂, antimycin A, or CCCP as a mitochondrial damaging agent, and levels of ROS and ATP were analyzed according to the methods described in Examples 7 and 8, respectively. The results showed that the α-helical peptide FL-CMP3013 restored the function of damaged mitochondria in a dose-dependent manner, regardless of the damage conditions for the mitochondria. Specifically, it was identified that for the increase in ROS production and the decrease in ATP production induced by H₂O₂, antimycin A, or CCCP, FL-CMP3013 inhibited ROS production and resumed ATP production, respectively (FIGS. 17A to 17C, related to inhibited ROS production; FIGS. 17D to 17F, related to increased ATP production).

Next, the cells were treated with FL-CMP3013 at two concentrations (10 or 30 nM) for 3 hours, and then treated with 1 nM H₂O₂ for 1 hour. The membrane potential of mitochondria was measured according to the method described in Experimental Example 7. As a result, it was identified that the mitochondrial membrane potential decreased by H₂O₂ was increased by FL-CMP3013 treatment in a concentrationdependent manner (FIG. 17G).

In addition, the mitochondrial membrane potential restoring ability was tested by treatment with Cyclosporin A (CsA), which is a positive control, FL-CMP3012, FL-CMP3013, and FL-CMP3015 in different types of cells and damage conditions, respectively. Specifically, 3 × 10⁶ SH-SY5Y cells were stained with JC-1 dye and seeded in 96-well plates at 1 × 10⁵ per well. Treatment with 20 µM of antimycin A was performed to induce a decrease in mitochondrial membrane potential. Simultaneously, each sample was treated with CsA or the peptide (1 µM) according to the disclosure to restore the mitochondrial membrane potential. The fluorescence ratio (red/green) of JC-1 was measured in the presence of CsA or the peptide. The changes in mitochondrial membrane potential were relative changes in mitochondrial membrane potential measured by comparing the membrane potential change at 15 minutes after antimycin A treatment with the membrane potential of normal cells which was set to 1. As a result, FL-CMP3013 as well as FL-CMP3012 and FL-CMP3015 showed similar restoration effects on the mitochondrial membrane potential, and these effects were comparable to cyclosporine A (FIG. 17H).

### Example 11.2. Identification of cristae protecting ability

In order to determine the mode of action of the peptide according to the disclosure, attenuation of cristae number in mitochondria was examined in relation to impaired mitochondrial function caused by H₂O₂ treatment. In other words, it was investigated whether promotion of normal mitochondrial function caused by CMP3013 was related to preservation of cristae structure. To evaluate this protective effect, transmission electron microscopy (TEM) images were used to observe changes in the shape and number of cristae in damaged HeLa cells in the presence or absence of CMP3013.

As a result, it was found that in the presence of the peptide of the present disclosure, long and extended forms of cristae are present in the number consistent with the no-treatment group, and long forms of cristae are almost absent in the cells damaged by H₂O₂ (FIG. 17I, (b)). In other words, H₂O₂ treatment destroys most of the cristae structures in mitochondria, and even under this condition where the cristae structure is destroyed, treatment with CMP3013 resulted in the same number of normal cristae structures as in the control group with no treatment (FIG. 17I, (c) and (d)).

The results of this series of experiments are consistent with the results of Example 11.1 which showed that almost all mitochondrial function was restored even in a case of being treated with the peptides of the present disclosure, including CMP3013, at low concentrations of 10 to 30 nM. From these results, it can be eventually seen that restoration of mitochondrial function was associated with structural preservation of cristae.

On the other hand, considering the results of the cytotoxicity test in Example 6, the concentration of the peptide disclosed herein, which can efficiently restore damage in mitochondrial function, is about 10 to 30 nM, and the therapeutic index in this concentration range is calculated to be around 300 to 1,000. These indicators show that the peptides disclosed herein, including CMP3013, have very high safety for the human body.

### Example 12. Identification of mechanism of action of mitochondria-specific peptide

### Example 12.1. Cell-penetration mechanism of mitochondria-specific peptide

A study was conducted to obtain mechanism information on cell-penetration and final fate of the mitochondria-specific peptide disclosed herein.

First, to study the mechanism of endocytosis, HeLa cells were subjected to pretreatment under various inhibitory conditions as follows: Cooling to 4°C (inhibition of ATP-dependent pathway); EIPA [5-(*N*-ethyl-*N*-isopropyl)amiloride] treatment (inhibition of macropinocytosis); MβCD (methyl-β-cyclodextrin) treatment (cholesterol depletion); sodium chlorate (NaClO₃, inhibition of proteoglycan-dependent pathway); chlorpromazine (CPZ, inhibition of clathrin-mediated endocytosis). The cells were then incubated with 100 nM of FL-CMP3013 for 3 hours and then FACS analysis was performed according to the method described in Experimental Example 3. According to the results shown in FIG. 18A, CMP3013 was most likely to penetrate into the cells using various endocytosis mechanisms, in particular, cholesterol-mediated pathway.

In addition, microscopic images obtained by treating HeLa cells, which express an endosomal marker (CellLight^{™}), with FL-CMP3013 and lysotracker according to the method described in Experimental Example 5 revealed that the endosomal marker co-localized to less than 20% (FIG. 18B). These observations suggest that most of the peptides in the cytosol are generated by endosomal escape. In addition, part of CMP3013 is located in lysosomes, suggesting that not all peptides escape into the cytosol (FIG. 18B).

Next, to investigate whether mitochondrial damage affects the amount of peptide present in lysosomes, cardiolipin-specific targeting was investigated using NAO in the presence or absence of CCCP. As a result, it was identified that the proportion of peptide present in lysosomes remained the same in the presence or absence of CCCP (0.29 and 0.30, respectively; FIGS. 18C and 18D). These observations suggest that CMP3013 does not by itself promote mitophagy, another important pathway for removal of dysfunctional mitochondria.

In contrast, it was identified that the proportion of CMP3013 present in the cytosol was significantly increased in a case of being treated with CCCP (0.43; FIGS. 18C and 18D) as compared with a case of being not treated with CCCP (0.08; FIGS. 18C and 18D).

These results support that CMP3013 targets cardiolipin, which is exposed by mitochondrial damage, in the inner membrane. Furthermore, it has been demonstrated that the peptide according to the present disclosure penetrates into a cell by endocytosis, exits the endosome, and preferentially moves into damaged mitochondria.

### Example 12.2. Identification of cardiolipin-specific binding

To further validate specificity of CMP3013 for cardiolipin-rich membranes identified in Example 9, two model systems mimicking the inner mitochondrial membrane were constructed using POPC (1-palmitoyl-2-oleoyl-sn-glycero-3-phosphocholine) and POPE (1-palmitoyl-2-oleoyl-sn-glycero-3-phosphoethanolamine), and cardiolipin binding assays were performed using an NBD-labeled CMP3013 monomer (NBD-CMP3013m), according to the method described in Experimental Example 12.

For this purpose, the NBD-labeled CMP3013 monomeric peptide was first synthesized. The sequence and mass spectrometry data of the peptide are shown in Table 15, and the HPLC chromatogram of the peptide is shown in FIG. 19A.

**[Table 15]**

| **Peptide** | **Sequence (X = Cha)** | **Mass (calcd.) [M+H]⁺** | **Mass (obsd.) [M+H]⁺** |
|---|---|---|---|
| NBD-CMP3013m | NBD-NH-XCRLXRRXCR-C(O)NH₂ | 1695.97 | 1698.69 |

The surface plasmon resonance (SPR) technique was used to determine binding ability of the peptide to liposome. Specifically, an L1 sensor chip was inserted into a Biacore^{™} T200 SPR system (Cytiva, USA) and then liposome was injected so that the liposome binds to the sensor chip. Subsequently, 1 µM of NBD-CMP3013m peptide was injected to check the signal intensity increased by the binding of liposome and CMP3013.

In the inner membrane model system generated on a sensor chip, the peptide of the present disclosure elicited approximately 50% greater response unit (RU) for the POPC/POPE membrane containing 10% cardiolipin as compared with the POPC/POPE membrane alone (see top graphs in FIG. 22A). Furthermore, dissociation of the peptide from the cardiolipin-containing membrane could not be observed because the membrane itself was disrupted under all attempted dissociation conditions. These findings suggest that dissociation of the peptide according to the present disclosure from cardiolipin is very slow and, as a result, CMP3013 has a very strong interaction with a cardiolipin-containing membrane (data not shown).

In addition, experiments using another inner membrane model prepared by the liposome technique showed that the fluorescently-labeled CMP3013m bound almost 10-fold faster to the 10% cardiolipin (CL)-containing POPC/POPE liposome and exhibited 2-fold greater final fluorescence intensity, as compared with the POPC/POPE liposome alone (FIG. 19B). It was observed that dissociation of the peptide from the cardiolipin (CL)-containing membrane surface, which is measured using a competition assay with an unlabeled peptide, was approximately 10-fold slower than dissociation from the cardiolipin-free membrane. That is, in a case where cardiolipin is present in the inner membrane, the dissociation rate of CMP3013m decreased by 10-fold (FIGS. 19B and 19C). The interactions occurring between the positively charged groups of CMP3013 and the negatively charged phosphate moieties of cardiolipin may affect their fast binding, while the hydrophobic interactions between the hydrophilic and lipid moieties of the two substances may contribute significantly to enhanced interaction therebetween (FIGS. 19B and 19C).

On the other hand, pathogenic cardiolipin remodeling means that a decrease in cardiolipin content or an increase in oxidized mono-lyso cardiolipin (MLCL) and oxidized di-lyso cardiolipin (DLCL) occurs in the inner mitochondrial membrane. To observe whether CMP3013 can bind to a pathogenically altered inner mitochondrial membrane, two additional artificial membranes were prepared. One consists of POPC:POPE (2: 1) liposomes with cardiolipin in varying proportions (0 to 20%) (FIG. 19D), and the other comprises the same liposomes with 10% DLCL (FIG. 19B). As a result of the experiment, it was identified that the monomeric form of CMP3013 bound to two liposomes with a cardiolipin content of 5 to 10% and the same liposome containing 10% DLCL as compared with a membrane containing 20% cardiolipin (FIGS. 19B and 19D). These results suggest that CMP3013m can bind to a cardiolipin-damaged membrane and overcome these pathogenic changes in the inner mitochondrial membrane.

Chemically important events (or reactions) in mitochondria occur in the inner membrane, where cardiolipin (CL) causes the curvature necessary for functional proteins, such as those that make up the electron transport system, to function properly. However, pathogenic cardiolipin remodeling destroys this curvature of the inner membrane, leading to mitochondrial dysfunction (FIG. 20). The amphipathic α-helical peptide penetrates into the cell and binds to multiple cardiolipins to regenerate the curvature of the inner mitochondrial membrane. In short, the peptide according to the present disclosure binds specifically and strongly to cardiolipin (CL) or a pathogenically remodeled cardiolipin-containing membrane and recruits functional proteins of the mitochondria, thereby correcting mitochondrial dysfunction (FIG. 20). This function of CMP3013 is consistent with the results obtained by confocal microscopic analysis of colocalization of CMP3013 with the CL-specific molecule NAO in Example 9 (FIG. 15B), which provides strong evidence that CMP3013 (or monomers thereof) binds strongly to the CL-rich inner membrane of damaged mitochondria and stays there to protect the membrane or the structure of cristae.

Through Examples 9 to 12, the present inventors have shown that the peptide CMP3013 (or reduced monomers thereof) preferentially binds to damaged mitochondria and can promote restoration and maintenance of cristae structure destroyed by hydrogen peroxide (H₂O₂) or other damaging agents. In particular, since CMP3013 has at least four positive charges and four hydrophobic moieties, it preferentially and more tightly binds to multiple cardiolipin (CL) molecules than other components of the inner mitochondrial membrane (IMM). As a result, it was identified that CMP3013 can promote normal mitochondrial function by protecting the cristae structure so that an increase in ROS inhibition ability and ATP production ability, and an increase in mitochondrial membrane potential are induced.

### IV. Comparative analysis with prior art

SS (Szeto-Schiller) peptide is a representative peptide known to exhibit therapeutic effects by targeting mitochondria and shows a β-sheet conformation in which amino acids containing a relatively hydrophobic benzene ring and positively charged amino acids are alternately bonded (FIG. 2). Two types of SS peptide are well known, of which the peptide named SS-31 has been reported to enhance mitochondrial function by binding to cardiolipin. In addition, SS-31 is undergoing animal and clinical trials for various diseases related to mitochondrial dysfunction due to its excellent therapeutic effects achieved by improvement of mitochondrial function. However, the phase 3 clinical trial of SS-31 for mitochondrial myopathy, which was conducted for three years (2017-2020), failed. SS-31 is a peptide in β-sheet conformation and has low cell-penetrating ability. Thus, to achieve a therapeutic effect, a high concentration of SS-31 peptide needs to be administered; however, in this case, there is a problem of increased cytotoxicity. Therefore, in order to use a peptide at low concentrations, it is necessary to improve cell-penetrating ability of the peptide. Furthermore, in order to achieve a therapeutic effect using a peptide at low concentrations, there is a need to increase the number of cardiolipins with which a single peptide molecule interacts.

The present disclosure was designed to solve the technical problems faced by existing mitochondria-targeting peptides as described above. The peptides of the present disclosure have significantly improved cell-penetrating ability and can be used at low concentrations. These peptides have a maximized therapeutic effect because a single peptide molecule interacts more with cardiolipin. The following Comparative Examples 1 to 5 demonstrate that the peptides of the present disclosure have a significant effect over the prior art through comparative analysis of the cell-penetrating ability, cardiolipin binding ability, mitochondrial function-related ROS production inhibiting ability, ATP production promoting ability, membrane potential restoring ability, and the like between the conventional mitochondria-targeting peptide SS-31 and CMP3013 peptide according to an embodiment of the present disclosure.

SS-31 used in the following comparative examples was synthesized and purified by the same method as the solid phase peptide synthesis described in Experimental Example 1.1 (see FIG. 1 for the chemical structure of SS-31).

### Comparative Example 1. Comparison of cell-penetrating ability and mitochondria-targeting ability between SS-31 and CMP3013

Cell-penetrating ability was performed according to the method described in Experimental Example 3, and mitochondria-targeting ability was assessed by mitochondrial fractionation according to the method described in Experimental Example 4. In addition to SS-31 as a conventional peptide, a mitochondrialpenetrating peptide of Kelly's group (MPP1a; see Horton, Kristin L et al. "Mitochondria-penetrating peptides." Chemistry & biology vol. 15,4 (2008): 375-82) was used as an additional comparison group. The amino acid sequence of MPPla is as follows: X(dR)XKX(dR)XK (where X is cyclohexylalanine, dR is D-arginine, and K is lysine).

As a peptide according to an embodiment of the present disclosure, in addition to CMP3013, LR10, which is included in the L1L, L4L, L5L, and L8L sub-libraries, was used as an additional comparison group. The amino acid sequence of LR10 is as follows: AcNH-RCRLLRRLRLCR-C(O)NH₂.

First, as a result of comparing the cell-penetrating ability, CMP3013 and LR10 showed approximately 100-fold improvement in cell penetration rate as compared with the conventional SS-31 (FIG. 21A). CMP3013 was evaluated to have the best cell-penetrating ability as it showed the lowest EC₅₀ value, and LR10 was also determined to have a nanomolar level of EC₅₀ value similar to CMP3013. In contrast, MPPla and SS-31 were determined to have a micromolar level of EC₅₀ value, and it was identified that they had significantly lower cell-penetrating ability than CMP3013 (FIG. 21A).

Next, to determine to what extent the four peptides target mitochondria, cells were treated with each peptide at the EC₅₀ concentration determined above, and the cells were fractionated. As a result, as compared with the other peptides, CMP3013 showed the highest mitochondrial/cytoplasmic abundance ratio (M/C ratio, 4.1) (FIG. 21B).

Based on the results of the above comparative experiments, the peptides according to the present disclosure, such as CMP3013 and LR10, have significantly improved cell-penetrating ability and mitochondria-targeting ability as compared with the conventional SS-31 and MPPla peptides, and thus can be delivered in therapeutically effective amounts even at low concentrations.

### Comparative Example 2. Comparison of cardiolipin binding ability between SS-31 and CMP3013

Two types of model systems mimicking the inner mitochondrial membrane were constructed using POPC and POPE according to the method described in Experimental Example 12, and NBD-labeled CMP3013 monomer (NBD-CMP3013m) and NBD-labeled SS-31 (NBD-SS-31) were used to compare and analyze their cardiolipin binding ability.

As described in Example 12.2, CMP3013m elicited approximately 50% greater response unit (RU) for the POPC/POPE membrane containing 10% cardiolipin (POPC:POPE:CL) as compared with the POPC/POPE membrane alone (POPC:POPE) in the inner membrane model system created on a sensor chip using the surface plasmon resonance (SPR; Biacore) technique (top graphs of FIG. 22A). In contrast, SS-31 did not show any difference in binding to the two types of membrane (bottom graphs of FIG. 22A). Furthermore, CMP3013m did not dissociate from the cardiolipin-containing membrane due to its very strong binding capacity.

Furthermore, even in experiments with other inner membrane models prepared by the liposomal technique, CMP3013m also showed better binding capacity for all types of membranes (liposomes with cardiolipin or di-lysocardiolipin) than SS-31 (FIG. 22B). Although SS-31 exhibited selective binding capacity for a cardiolipin-containing membrane, its binding capacity was significantly weaker than CMP3013m.

As shown in Example 12 and FIG. 20, cardiolipin causes curvature of the inner membrane that allows mitochondria to function normally. Both CMP3013 and SS-31 have a common mechanism of action that each specifically binds to cardiolipin in the inner mitochondrial membrane to provide curvature to the inner membrane, thereby allowing the cristae structure to be maintained. Since CMP3013 disclosed herein has approximately 100-fold stronger binding capacity for cardiolipin than SS-31, it is expected for CMP3013 to overcome the problem that existing mitochondria-specific peptides such as SS-31 have little therapeutic effect at low concentrations.

### Comparative Example 3. Comparison of ROS production inhibiting ability between SS-31 and CMP3013

To compare effects of the two peptides on restoration of mitochondrial function, SS-31 and CMP3013 were tested for their ROS production inhibiting ability. The relative ROS levels in cells were compared by fluorescence intensity according to the method described in Experimental Example 6. SH-SY5Y cells were treated with four different mitochondrial damaging agents (200 µM CCCP, 50 µM rotenone, 10 µM antimycin A, or 50 µM H₂O₂) to induce production of ROS by mitochondrial damage.

As a result, as shown in FIGS. 23A to 23D, SS-31 at nanomolar levels did not exhibit a significant ROS production inhibiting effect under all damage conditions. In contrast, CMP3013 exhibited equivalent or better ROS production inhibiting ability than SS-31 even at a low concentration of less than 1/10 times the SS-31 concentration, regardless of damage conditions.

### Comparative Example 4. Comparison of ATP production promoting ability between SS-31 and CMP3013

For comparison of efficacy of the two peptides on restoration of mitochondrial function, SS-31 and CMP3013 were tested for their ATP production promoting ability. The relative ATP levels of cells were compared by fluorescence intensity according to the method described in Experimental Example 8, and mitochondrial damage was induced by treatment with two different mitochondrial damaging agents (1 µM of antimycin A or 1 mM of H₂O₂).

As a result, as shown in FIGS. 24A and 24B, SS-31 failed to promote ATP production under all damage conditions and at all concentrations. In contrast, CMP3013 of the present disclosure exhibited an effect of promoting resumption of ATP production even in a case of being applied at a concentration as low as 10 nM.

### Comparative Example 5. Comparison of membrane potential restoring ability between SS-31 and CMP3013

For comparison of efficacy of the two peptides on restoration of mitochondrial function, SS-31 and CMP3013 were tested for their effect on membrane potential. The change in mitochondrial membrane potential was measured as a fluorescence ratio of JC-1 according to the method described in Experimental Example 8, and 1 mM of H₂O₂ was used as a mitochondrial damaging agent.

As a result, mitochondria are damaged by H₂O₂ treatment, resulting in lower mitochondrial membrane potential (ΔΨm). CMP3013 increased mitochondrial membrane potential (ΔΨm) at a concentration below 10 nM, whereas SS-31 failed to increase mitochondrial membrane potential even at a concentration at 30 nM or higher (FIG. 25).

The experimental results of Comparative Examples 3 to 5 demonstrate that the α-helical peptides of the present disclosure exhibit a significantly higher effect of restoring mitochondrial function at much lower concentrations, as compared with conventional mitochondria-targeting peptides including SS-31.

### V. Therapeutic effects of mitochondria-specific peptides on diseases

### Example 13. Distribution of peptides in organs

To determine the *in vivo* distribution of the α-helical peptide according to the present disclosure when administered systemically by intravenous injection, the cyanine 5.5-labeled peptide CMP3013 was intravenously administered to normal male C57BL/6 mice at a dose of 3 mg/kg, and organs were harvested 24 hours later for IVIS imaging analysis. As a result, it was identified that CMP3013 was mainly distributed in the liver, lung, spleen, and kidney (FIG. 26).

### Example 14. Therapeutic effects on acute liver failure

### Example 14.1. Identification of inhibitory effect of intravenously administered CMP3013 on liver injury

### Example 14.1.1. Preparation of animal model of acute liver failure and blood analysis

Eight-week-old female C57BL/6 mice received intravenous administration of CMP3013 a total of three times at a dose of 1 mg/kg at 2-day intervals (Days 0, 2, and 4) followed by intraperitoneal administration of thioacetamide (TAA; Sartorius, Germany) at a dose of 100 mg/kg on the fifth day (Day 5) to induce acute liver failure. Serum samples were obtained from blood collected from the heart on Day 6, and the samples were analyzed using a hematology analyzer (Hitachi Chemical Industries, Ltd., Japan). Blood AST (aspartate aminotransferase), ALT (alanine aminotransferase), and ALP (alkaline phosphatase) were measured and evaluated as liver injury markers.

### Example 14.1.2. Identification of decrease in liver injury-related markers

It was identified whether TAA-induced liver injury was inhibited in the mice pre-injected with CMP3013 according to the method described in Example 14.1.1.

First, no adverse events occurred in any of the experimental animals following administration of CMP3013, and no animals died. In addition, no weight loss was observed due to administration of TAA and/or CMP3013.

Blood AST, ALT and ALP, which are liver injury markers, were all found to be significantly reduced in the CMP3013-administered group (FIGS. 27A to 27C). AST, which was at a level of 73.40 ± 12.05 U/L in the normal control group, soared to 488.20 ± 174.17 U/L by administration of TAA, and this level was significantly reduced to 316.40 ± 52.65 U/L by administration of CMP3013, representing a 35% reduction as compared with the group having received TAA alone (FIG. 27A). ALT, which was 29.40 ± 7.02 U/L in the normal control group, was rapidly elevated to 499.40 ± 242.69 U/L by administration of TAA, and this level was reduced to 332.60 ± 86.31 U/L in the CMP3013-administered group (FIG. 27B). In addition, ALP was measured at 292.40 ± 42.08 U/L in the normal control group, 573.00 ± 117.87 U/L in the TAA-administered group, and 462.00 ± 81.10 U/L in the CMP3013-administered group, which represents a 19% decrease as compared with the TAA-administered group (FIG. 27C).

These results suggest that a low dose (1.0 mg/kg) of CMP3013 can provide a sufficient level of efficacy to rescue TAA-induced damage in the liver.

### Example 14.2. Identification of dose-dependent inhibitory effect of intraperitoneally administered CMP3013 on liver injury

### Example 14.2.1. Preparation of animal model of acute liver failure and blood analysis

Seven-week-old female C57BL/6 mice received intraperitoneal administration of CMP3013 a total of three times at a dose of 1 mg/kg at 2-day intervals (Days 0, 2, and 4) followed by intraperitoneal administration of thioacetamide at a dose of 100 mg/kg on the fifth day (Day 5) to induce acute liver failure. Serum samples were obtained on the seventh day (Day 7) and analyzed using a hematology analyzer. Blood AST (aspartate aminotransferase), ALT (alanine aminotransferase), and ALP (alkaline phosphatase) were measured and evaluated as liver injury markers.

### Example 14.2.2. Identification of decrease in liver injury-related markers

It was identified whether TAA-induced liver injury was inhibited in the mice pre-injected with CMP3013 at different concentrations according to the method described in Example 14.2.1.

As a result, it was identified that blood AST, ALT, and ALP, which are liver injury markers, were all significantly reduced in the CMP3013-administered group (FIGS. 27D to 27F). In particular, AST and ALT tended to decrease in response to administered CMP3013 in a dose-dependent manner (FIGS. 27D and 27E). These results suggest that a low dose (1.0 mg/kg) of CMP3013 inhibits liver injury, and furthermore, a high dose (10 mg/kg) of CMP3013 can provide a further enhanced liver injury inhibitory effect.

### VI. Conclusion and Discussion

The mitochondria-specific peptide (in the form of a monomer or a dimer thereof) of the present disclosure, which has amphipathic properties and an α-helical structure by being constructed so that the amino acids at positions 1, 4, 5, and 8 are hydrophobic and the amino acids at positions 3, 6, 7, and 10 are hydrophilic, has several important advantages that suggest high potential for use as a therapeutic agent. First, the peptides of the present disclosure, including CMP3013 and CMP3029, have very high cell-penetrating ability, and thus are easily located in cells even at concentrations as low as nanomolar levels (EC₅₀ = 35 nM). Second, the peptides disclosed herein preferentially enter mitochondria (about 80%), which means that there is not a large amount present in the cytosol to cause side effects. Third, the peptides disclosed herein preferentially bind to cardiolipin (CL), which is a specific phospholipid in the inner mitochondrial membrane, and protects cristae structure, thereby providing a robust membrane that allows functional proteins to function properly. Fourth, and most importantly, the peptides disclosed herein have strong binding capacity for multiple (or a plurality of) cardiolipin molecules, resulting in a much stronger interaction than expected in a typical 1:1 complex. As a result, dissociation of the peptide of the present disclosure from a cardiolipin-containing layer is very slow, making its effect even more potent.

In addition, the finding that the peptides of the present disclosure have very low correlation with the extended form of mitochondria while having higher correlation with the donut-shaped or bulge-shaped mitochondria may also be equally important for their potential use as a therapeutic agent (FIG. 15A). In other words, as mentioned above, these findings suggest that the peptides disclosed herein are selectively located in damaged mitochondria where protective cristae organizing system is not present. For this reason, it is expected that only a small amount of the peptide according to the present disclosure will be needed to effectively block formation of damaged cardiolipin (or cristae) and maintain functional cristae without affecting functional mitochondria. That is, for the peptides disclosed herein, the nanomolar therapeutic concentrations required to minimize cytotoxicity should be sufficient to protect mitochondrial function, including ATP production and increase of mitochondrial membrane potential.

The embodiments described above showed that the α-helical amphipathic peptides of the present disclosure, CMP3013, CMP3029, and the like preferentially bind to abnormal and damaged mitochondria and promote restoration and/or maintenance of cristae structure destroyed by treatment with H₂O₂ or other damaging agents. It is very encouraging that in both *in vitro* cell-based experiments and *in vivo* animal model studies, therapeutic effects were promoted by very low concentrations of the peptide disclosed herein. Mitochondrial homeostasis, including biosynthesis and mitophagy, allows cells to rapidly replace dysfunctional mitochondria with intact organelles. Therefore, therapeutic agents do not need to repair all defective mitochondria, and it is expected that some of the damaged mitochondria repaired by the peptides disclosed herein are sufficient to restore normal tissue function.

This expectation is supported by the fact that CMP3013, α-helical amphipathic peptide disclosed herein, was effective in inhibiting liver cell damage or restoring damaged liver cells even when administered at low doses in an animal model of acute liver failure induced by TAA.

### VII. Materials and methods

### Experimental Example 1. Preparation of peptides

### Experimental Example 1.1. Synthesis, isolation, and purification of peptides

Peptides were synthesized using a standard 9-fluorenylmethyloxycarbonyl (Fmoc) solid-phase peptide synthesis (SPPS) method on an SPS microwave peptide synthesizer (Discover, CEM, USA).

Specifically, rink amide MBHAresin was deprotected with 20% (v/v) piperidine in *N*,*N*-dimethylformamide (DMF), and coupling of Fmoc-protected amino acids was performed in the presence of *N,N*-diisopropylethylamine (DIPEA) and (benzotriazol-1-yloxy)tripyrrolidinophosphonium hexafluorophosphate (PyBOP). Deprotection and coupling were repeated until a peptide having a desired sequence was synthesized. After addition of the last amino acid, the N-terminus of the peptide was acetylated. Acetylation was performed by adding acetic anhydride and 1-hydroxybenzotriazole hydrate (HOBt).

For fluorescent labeling, 5-carboxytetramethylrhodamine (5-TAMRA) was conjugated to the peptide in the presence of O-(1H-6-chlorobenzotriazole-1-yl)-1,1,3,3-tetramethyluronium hexafluorophosphate (HCTU), HOBt, and DIPEA. 6-(7-nitrobenzofurazan-4-ylamino) hexanoic acid (C6-NBD) was labeled with PyBOP and DIPEA.

The synthesized peptides on the resins were cleaved using a cleavage cocktail [containing trifluoroacetic acid (TFA)/1,2-ethanedithiol (EDT)/distilled water/triisopropylsilane (TIS) = 94:2.5/2.5/1, v/v]. For precipitation, a 1:1 (v/v) mixture of n-hexane and diethyl ether was added to the cleaved peptides. The precipitated peptides were centrifuged at 5000 rpm for 15 minutes and the peptide pellets were dissolved in DMSO.

Purification of the peptides was performed using high-performance liquid chromatography (HPLC). The mobile phase consisted of water with 0.1% (v/v) TFA and acetonitrile (ACN) with 0.1% (v/v) TFA, and a Zorbax C18 column (3.5 µm, 4.6 × 150 mm) was used as the stationary phase. The purified peptides were lyophilized and dissolved in an appropriate solvent. The molecular weights of the peptides were determined using a MALDI-TOF mass spectrometer (Bruker, USA).

### Experimental Example 1.2. Peptide dimerization

For preparation of dimeric peptides, each of the lyophilized peptides was dissolved in 0.1 M ammonium bicarbonate. In some cases, ACN was added up to 40% (v/v) to increase solubility of the peptide. The prepared peptide solution was stirred and oxidized by using a shaker under atmosphere. After oxidation, the dimerized peptide was purified and lyophilized.

### Experimental Example 2. Cell culture

HeLa cells were cultured in Dulbecco's modified Eagle's medium (DMEM; Cytiva, SH30243.01, USA) supplemented with 10% fetal bovine serum (FBS; Gibco, 16000-044, USA) and antibiotics (Gibco, 15240-062, USA). The cells were incubated in a 5% CO₂, humidified incubator at 37°C.

### Experimental Example 3. Flow cytometry

1 × 10⁵ HeLa cells were seeded into a 24-well plate. After 24 hours, the peptide was added to the cells and further incubation was performed for 3 hours. The incubated cells were harvested, washed with PBS, suspended in PBS, and then analyzed by fluorescence-activated cell sorting (FACS; BD Accuri, USA). The cells whose fluorescence intensity exceeds the maximum intensity of untreated cells were classified as fluorescence-positive cells. The EC₅₀ (half maximal effective concentration) value was defined as a concentration of a dimeric peptide at which the corresponding fluorescently-labeled peptide dimer enters half of the incubated cells.

### Experimental Example 4. Fractionation of mitochondria

Intracellular distribution of peptides was detected using the mitochondria isolation method. TAMRA-labeled peptides were added to 6 × 10⁶ HeLa cells at their EC₅₀ concentration, and the cells were incubated for 3 hours. The cells were harvested, washed with cold PBS, and suspended in cold IB (30 mM Tris-HCl, pH 7.4; 225 mM mannitol; 75 mM sucrose; and 0.1 mM EGTA). Homogenization was performed using a precooled Dounce homogenizer until 80-90% of the cells were disrupted. Lysed cells were monitored under a light microscope. The homogenate was transferred to an e-tube and centrifuged at 4°C, 600 g for 5 minutes. The supernatant was carefully collected and centrifuged once again. The supernatant was then centrifuged at 4°C, 7,000g for 10 minutes. The obtained supernatant (cytosolic fraction) was transferred to a new e-tube, and the pellet (mitochondrial fraction) was resuspended in an equal volume of cold IB. The fluorescence intensity of each fraction was measured by using a microplate reader [TECAN, Switzerland; excitation (Ex)/emission (Em) = 510/570 nm].

### Experimental Example 5. Observation using confocal microscopy and ultra-high resolution microscopy

For microscopic observation, HeLa cells were cultured in glass-bottom dishes (SPL, 200350, Korea). The cells were then treated with peptides for 3 hours and stained with MitoTracker^{™} (Thermo Fisher Scientific, A1372, USA; 100 nM, 1 hr), nonyl acridine orange (NAO; Thermo Fisher Scientific, A1372; 100 nM, 1 hr), Hoechst 33342 (1 µg/mL, 20 min), CellLight^{™} early endosomes-GFP (Thermo Fisher Scientific, C10586; 2 µL/10,000 cells, 16 hr), and/or LysoTracker^{™} (Thermo Fisher Scientific, L7525; 50 nM, 30 min). The cells were then rinsed with PBS and HBSS was added thereto. Images of the stained cells were obtained using a microscope (Zeiss LSM 880 and ELYRA PS. 1). To generate ultra-high resolution images, a structured illumination process was performed with ZEN Black software. Pearson r values were calculated by using ImageJ software.

### Experimental Example 6. Analysis of cellular ROS levels

### Intracellular ROS levels were measured using two methods.

The first method was performed as follows. First, cells cultured on glass bottom dishes were treated with TAMRA-labeled peptides at their EC₅₀ concentration for 3 hours. Then, the cells were stained with 2',7'-dichlorofluorescin diacetate (H₂DCFDA; Sigma, 287810, USA; 25 µM, 30 min) and washed twice with PBS. The cells were treated with 500 µM H₂O₂ in HBSS and incubated for an additional 10 minutes. DCF signal of the cells was detected by confocal microscope and an image thereof was taken. Green signal intensity of each image was calculated using ZEN blue software.

The second method was performed as follows. First, 2 × 10⁴ HeLa cells were seeded into a 96-well plate. After 24 hours, the cells were incubated with each peptide for 3 hours, and then staining with H₂DCFDA (25 µM, 30 min) was performed. After performing washing twice with PBS, the cells in HBSS were used for analysis of ROS levels. The DCF signal of each well was obtained using a microplate reader (TECAN) at 37 °C (Ex/Em = 485/535 nm).

### Experimental Example 7. Measurement of mitochondrial ROS and membrane potential

2 × 10⁴ HeLa cells were seeded in a 96-well plate 24 hours before the experiment. The cells were then stained with MitoSOX^{™} (Thermo Fisher Scientific, M36008; 5 µM, 10 min) or tetramethylrhodamine ethyl ester (TMRE; Sigma, 87917; 600 nM, 15 min). The cells were washed with PBS, and HBSS was added thereto. Fluorescence signal of the cells was measured with a microplate reader (TECAN) at 37°C (MitoSox, Ex/Em = 510/580 nm; TMRE, Ex/Em = 525/575 nm).

### Experimental Example 8. Measurement of ATP levels

Cellular ATP levels were assessed using an ATP detection kit (Abcam, ab 113849, USA) according to the manufacturer's instructions. Fluorescence luminescence was measured with a microplate reader (Promega, Glomax 96 microplate luminometer, USA).

### Experimental Example 9. Transmission electron microscopy

1.8 × 10⁶ HeLa cells were fixed with modified Karnovsky fixative [2% paraformaldehyde and 2% glutaraldehyde dissolved in 0.05 M sodium cacodylate buffer] at 4°C for 3 hours. A sample was washed three times with 0.05 M sodium cacodylate buffer (pH 7.2) at 4°C for 10 minutes, and then post-fixed at 4°C for 2 hours with 1% osmium tetroxide dissolved in 0.05 M sodium cacodylate buffer (pH 7.2). The sample was then washed twice briefly with distilled water. Subsequently, the sample was then en bloc stained with 0.5% uranyl acetate at 4°C overnight. The stained sample was dehydrated with increasing concentrations of ethanol (once each in 30%, 50%, 70%, 80%, and 90% ethanol, and three times in 100% ethanol, each for 10 minutes), and then subjected to a transition step in which treatment with 100% propylene oxide is performed twice for 15 minutes. The sample was infiltrated with a series of propylene oxide and Spurr's resin (1:1 ratio for 1.5 hours, 1:2 ratio for 1.5 hours, treatment with Spurr's resin alone overnight, and then once more the same treatment for 4 hours), embedded in Spurr's resin, and polymerized at 70 °C overnight. The completely polymerized sample was sectioned by using an ultramicrotome (MT-X; RMC, USA). The obtained sections were stained with 2% uranyl acetate and Reynolds' lead citrate for 7 minutes each. Observation and imaging of the sample was performed using JEM-ARM200F TEM.

### Experimental Example 10. Plasmid construction and transfection

Wild-type Drp1 cDNA (kindly provided by Dr. Sunyong Chung, Ajou University School of Medicine) was amplified by polymerase chain reaction and inserted into the N-terminus of the eGFP gene in the pcDNA3.1-plasmid. Transfection of the plasmid was performed using Lipofectamine^{™} 3000 (Thermo Fisher Scientific, L3000001) according to the manufacturer's instructions.

### Experimental Example 11. Western blotting

For Western blotting, cells were harvested and suspended in buffer [50 mM Tris-HCl, pH 7.5; 150 mM NaCl; 1 mM EDTA; 1% IGEPAL CA-630; 0.25% sodium deoxycholate; 0.1% SDS; and protease inhibitor (Gendepot, P3100-001, USA)]. The cells were disrupted by sonication and 20 µg of protein was transferred to a nitrocellulose membrane by SDS polyacrylamide gel electrophoresis. Then, blocking with 5% non-fat milk was performed, and then treatment with primary and secondary antibodies was sequentially performed. The primary antibodies used were anti-COX4 (Abclonal, A6564), anti-α-tubulin (Abclonal, AC012), anti-glyceraldehyde-3-phosphate dehydrogenase (GAPDH; Genetex, GTX100118), and anti-Drp1 (BD biosciences, 611112), respectively.

### Experimental Example 12. Cardiolipin binding assay

To detect interaction of NBD-labeled peptides or C6-NBD [6-(7-nitrobenzofurazan-4-ylamino)hexanoic acid] with liposome, changes in fluorescence intensity of NBD were measured.

For the association experiments, 1 µM of NBD-labeled peptide dissolved in PBS was prepared in a black 96-well plate. Liposome (POPC:POPE = 2:1 or POPC:POPE = 2:1, containing 10% cardiolipin) was added to the peptide until the desired concentration was achieved. The mixture of NBD-peptide and liposome was mixed well through pipetting, and then fluorescence emission of the sample was monitored (λₑₓ = 467 nm, λₑₘ = 536 nm).

For the association experiments depending on cardiolipin ratio, 1 µM of NBD-labeled peptide or C6-NBD dissolved in PBS was prepared in a black 96-well plate. Liposome (POPC:POPE = 2:1, containing 0 to 20% cardiolipin) was added to the peptide until the desired concentration (5 µM) was achieved. This mixture was mixed well through pipetting and fluorescence emission of the sample was monitored (λₑₓ = 467 nm, λₑₘ = 536 nm).

For dissociation experiments, 1 µM of NBD-labeled peptide dissolved in PBS was prepared in a black 96-well plate. Liposome (POPC:POPE = 2:1 or POPC:POPE = 2:1, containing 10% cardiolipin) was added to the peptide until the desired concentration (100 µM) was achieved. The mixture of NBD-peptide and liposome was mixed well through pipetting and fluorescence intensity of the mixture was measured (λₑₓ = 467 nm, λₑₘ = 536 nm). The unlabeled peptide (acetylated peptide) was then added to the mixture and mixed well through pipetting. Incubation in the dark was performed for 10 minutes, and then changes in fluorescence intensity were monitored. Peptide addition and fluorescence measurement were repeated until the concentration of acetylated peptide reached 40 µM.

### Experimental Example 13. Animal experiment

All animal experiments were conducted in accordance with the Guidelines for the Care and Use of Laboratory Animals of the National Research Council (U.S.) under the prior review and approval of the OrientBio's Animal Experiment Ethics Committee (Animal Care and Use Number: ORIENT-IACUC-21158).

### Experimental Example 14. Statistical Analysis

All unpaired Student's t-tests were performed using GraphPad Prism software. Data represent mean values, and error bars represent standard deviations. Statistical significance was assessed with a p value less than 0.05 (ns, p ≥ 0.05; *, p < 0.05; **, p < 0.01; ***, p < 0.001; ****, p < 0.0001).

The statistical analysis in Example 14 was performed using a statistical analysis program (SPSS Statistics 22 for Analysis, IBM, USA). The evaluation items were expressed as mean and standard deviation, and the mean values of the respective evaluation items were compared by one-way ANOVA and Dunnett's multiple comparison tests, and a p value of less than 0.05 (*, p < 0.05; **, p < 0.01; ***, p < 0.001) was determined to be statistically significant.

## Claims

1. A pharmaceutical composition for prevention or treatment of a disease accompanied by mitochondrial dysfunction, comprising, as an active ingredient, a peptide comprising the monomer represented by Formula 1, or a dimer thereof:
<Formula 1> X₁X₂X₃X₄X₅X₆X₇X₈X₉X₁₀
in Formula 1,
X₁, X₄, X₅, and X₈ are each independently a hydrophobic amino acid,
X₃, X₆, X₇, and X₁₀ are each independently a hydrophilic amino acid,
X₂ and X₉ are each independently an amino acid that forms a bond so that two monomers can be linked to each other at one or more positions selected from X₂ and X₉ to form a dimeric peptide, and
X₁ is N-terminus and X₁₀ is C-terminus.

2. The pharmaceutical composition of claim 1, wherein the disease accompanied by mitochondrial dysfunction is selected from the group consisting of mitochondrial myopathy, MELAS (mitochondrial encephalopathy, lactic acidosis, and stroke-like episodes) syndrome, Charcot Marie Tooth disease (CMT), Leber hereditary optic neuropathy, Pearson syndrome, Leigh syndrome, Friedreich's ataxia, and Barth syndrome.

3. A pharmaceutical composition for prevention or treatment of a liver disease, comprising, as an active ingredient, a peptide comprising the monomer represented by Formula 1, or a dimer thereof:
<Formula 1> X₁X₂X₃X₄X₅X₆X₇X₈X₉X₁₀
in Formula 1,
X₁, X₄, X₅, and X₈ are each independently a hydrophobic amino acid,
X₃, X₆, X₇, and X₁₀ are each independently a hydrophilic amino acid,
X₂ and X₉ are each independently an amino acid that forms a bond so that two monomers can be linked to each other at one or more positions selected from X₂ and X₉ to form a dimeric peptide, and
X₁ is N-terminus and X₁₀ is C-terminus.

4. The pharmaceutical composition of claim 3, wherein the liver disease is selected from the group consisting of acute liver failure, acute liver injury, liver cirrhosis, and hepatitis.

5. The pharmaceutical composition of any one of claims 1 to 4, wherein X₁, X₄, X₅, and X₈ are each independently leucine (L), isoleucine (I), phenylalanine (F), tyrosine (Y), valine (V), norvaline (norV), tryptophan (W), pentylglycine (pg), neopentylglycine (Npg), alanine (A), or cyclohexylalanine (Cha).

6. The pharmaceutical composition of claim 5, wherein X₁, X₄, X₅, and X₈ are each independently leucine (L), phenylalanine (F), tyrosine (Y), or cyclohexylalanine (Cha).

7. The pharmaceutical composition of claim 6, wherein one or more of X₁, X₄, X₅, and X₈ are each independently cyclohexylalanine (Cha).

8. The pharmaceutical composition of any one of claims 1 to 4, wherein X₃, X₆, X₇, and X₁₀ are each independently arginine (R), lysine (K), homoarginine (hR), norarginine (norR), histidine (H), ornithine (O), diaminobutanoic acid (Dab), or diaminopropanoic acid (Dap).

9. The pharmaceutical composition of claim 8, wherein X₃, X₆, X₇, and X₁₀ are each independently arginine (R), lysine (K), homoarginine (hR), norarginine (norR), or histidine (H).

10. The pharmaceutical composition of any one of claims 1 to 4, wherein X₂ and X₉ are each independently cysteine (C), homocysteine (Hcy), penicillamine (Pen), selenocysteine (U), or leucine (L), provided that X₂ and X₉ are not leucine (L) at the same time.

11. The pharmaceutical composition of claim 10, wherein X₂ and X₉ are each independently cysteine (C), homocysteine (Hcy), or penicillamine (Pen).

12. The pharmaceutical composition of any one of claims 1 to 4, wherein the dimer is such that the two peptides, each of which comprises the monomer represented by Formula 1, are linked to each other in an antiparallel direction.

13. The pharmaceutical composition of claim 12, wherein X₂ and X₉ are each independently cysteine (C), homocysteine (Hcy), or penicillamine (Pen), and the linkage is by a disulfide bond.

14. The pharmaceutical composition of any one of claims 1 to 4, wherein the monomer represented by Formula 1 is a peptide consisting of an amino acid sequence of any one of SEQ ID NOS: 1 to 19.

15. The pharmaceutical composition of any one of claims 1 to 4, wherein the composition comprises the monomer or the dimer thereof at a nanomolar concentration.

16. A peptide or a dimer thereof, the peptide comprising the monomer represented by Formula 1:
<Formula 1> X₁X₂X₃X₄X₅X₆X₇X₈X₉X₁₀
in Formula 1,
X₁, X₄, X₅, and X₈ are each independently leucine (L), phenylalanine (F), tyrosine (Y), or cyclohexylalanine (Cha),
X₃, X₆, X₇, and X₁₀ are each independently a hydrophilic amino acid,
X₂ and X₉ are each independently an amino acid that forms a bond so that two monomers can be linked to each other at one or more positions selected from X₂ and X₉ to form a dimeric peptide, and
X₁ is N-terminus and X₁₀ is C-terminus.

17. The peptide or the dimer thereof of claim 16, wherein X₁, X₄, X₅, and X₈ are each independently leucine (L), phenylalanine (F), or cyclohexylalanine (Cha).

18. The peptide or the dimer thereof of claim 17, wherein one or more of X₁, X₄, X₅, and X₈ are each independently cyclohexylalanine (Cha).

19. The peptide or the dimer thereof of claim 16, wherein X₃, X₆, X₇, and X₁₀ are each independently arginine (R), lysine (K), homoarginine (hR), norarginine (norR), histidine (H), ornithine (O), diaminobutanoic acid (Dab), or diaminopropanoic acid (Dap).

20. The peptide or the dimer thereof of claim 19, wherein X₃, X₆, X₇, and X₁₀ are each independently arginine (R), lysine (K), homoarginine (hR), norarginine (norR), or histidine (H).

21. The peptide or the dimer thereof of claim 16, wherein X₂ and X₉ are each independently cysteine (C), homocysteine (Hcy), penicillamine (Pen), selenocysteine (U), or leucine (L), provided that X₂ and X₉ are not leucine (L) at the same time.

22. The peptide or the dimer thereof of claim 16, wherein the dimer is such that the two peptides, each of which comprises the monomer represented by Formula 1, are linked to each other in an antiparallel direction.

23. The peptide or the dimer thereof of claim 16, wherein the peptide or dimer interacts with cardiolipin in a mitochondrial membrane.

24. A peptide or a dimer thereof, the peptide comprising a monomer consisting of an amino acid sequence of any one of SEQ ID NOS: 1 to 19.

25. A method for restoring mitochondrial function, comprising administering, to a subject in need of restoration of mitochondrial function, a peptide comprising the monomer represented by Formula 1, or a dimer thereof:
<Formula 1> X₁X₂X₃X₄X₅X₆X₇X₈X₉X₁₀
in Formula 1,
X₁, X₄, X₅, and X₈ are each independently a hydrophobic amino acid,
X₃, X₆, X₇, and X₁₀ are each independently a hydrophilic amino acid,
X₂ and X₉ are each independently an amino acid that forms a bond so that two monomers can be linked to each other at one or more positions selected from X₂ and X₉ to form a dimeric peptide, and
X₁ is N-terminus and X₁₀ is C-terminus.
